# FASCICULE DE BREVET EUROPEEN

(11) **EP 0 891 344 B1**
(45) Date de publication et mention de la délivrance du brevet: **18.12.2002**
(21) Numéro de dépôt: 97919488.3
(22) Date de dépôt: 04.04.1997
(51) Int. Cl.: C07D 295/12

(54) **ANTAGONISTES DU RECEPTEUR ALPHA-1 ADRENERGIQUE**
ALPHA-1 ADRENERGISCHER REZEPTOR ANTAGONISTEN
ALPHA-1 ADRENERGIC RECEPTOR ANTAGONISTS

(30) Priorité: 05.04.1996 US 628276
(43) Date de publication de la demande: 20.01.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif-sur-Yvette (FR); CHARBIER de LASSAUNIERE, Pierre-Etienne, F-75016 Paris (FR); AUVIN, Serge, F-91240 Saint-Michel-sur-Orge (FR); AUGUET, Michel, F-91120 Palaiseau (FR)
(74) Mandataire: Bourgouin, André
(86) Numéro de dépôt international: FR9700615
(87) Numéro de publication internationale: WO97037983

(56) Documents cités:
- EP-A- 0 048 045
- EP-A- 0 343 961
- EP-A- 0 395 313
- EP-A- 0 434 561
- WO-A-93/21179
- WO-A-95/00131
- WO-A-95/04049
- WO-A-96/08480
- GB-A- 2 263 110
- US-A- 2 722 529
- US-A- 2 833 770

## Description

Les neurorécepteurs sont considérés comme des entités dynamiques qui constituent un moyen de manipulation clinique accessible. Le neurotransmetteur sympathique norépinéphrine et l'hormone surrénale épinéphrine régulent de nombreuses activités physiologiques au travers d'interactions avec les récepteurs α- et β-adrénergiques. Les récepteurs α₁-adrénergiques sont les médiateurs de nombre des effets physiologiques des catécholamines, notamment la glycogénolyse, la contraction du muscle lisse vasculaire et génito-urinaire, l'hyperpolarisation du muscle lisse intestinal, la force contractile et l'arythmie cardiaques.

Des composés structurellement comparables aux composés selon l'invention, ont été décrits dans les documents suivants :WO 95/00131, WO 95/04049, EP 343961, US 2722529 et US 2833770. Mais l'application revendiquée dans la présente demande (traitement de l'hypertension portale et de la cirrhose) n'est ni décrite ni suggérée dans ces documents. Dans l'un de ses aspects, l'invention concerne un composé répondant
soit à la formule I: 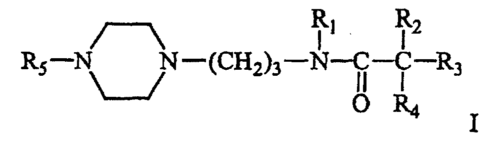 dans laquelle :
R₁ représente un hydrogène ou un alkyle inférieur ;
R₂ représente un hydrogène, un allyle inférieur, OR₆, S(O)ₘR₆ dans lequel m vaut 0, 1 ou 2, NHS(O)ₙR₆ dans lequel n vaut 1 ou 2, OC(O)R₆ , C(O)NR₆R₇, NR₆R₇ ou N(R₇)C(O)R₆ ;
chacun des R₃ et R₄, de façon indépendante l'un de l'autre, représente l'un des radicaux suivants substitués une à quatre fois ou non-substitués : alkyle inférieur, cyclohexyle, phényle, napthyle, benzyle dans lequel ledit substituant est un alkyle inférieur, un halogène, OR₆, SR₆, NR₆R₇, CN, NO₂, CF₃ ou phényle, ou R₃ et R₄, ensemble avec l'atome de carbone auquel ils sont conjointement liés, forment le radical 9-xanthényle ou 9-fluorényle ;
R₅ représente un phényle substitué une à quatre fois dans lequel ledit substituant est un alkyle inférieur, CN, un halogène, un hydroxy-alkyle inférieur, cyclopropylhydroxyméthyle, un alkoxy inférieur-alkyle inférieur, ou alkoxy inférieur ; et
chacun des R₆ et R₇, de façon indépendante l'un de l'autre, représente un hydrogène ou l'un des radicaux suivants substitués une à quatre fois ou non-substitués : un allyle inférieur, phényle, benzyle dans lequel ledit substituant est un alkyle inférieur, un halogène, ou un alcoxy inférieur ; ou un de ses sels pharmaceutiquement acceptables,
soit à l'une des formules suivantes:
   α-hydroxy-N-[3-{4-(2-méthoxyphényl)-1-pipérazinyl}-propyl]-benzèneacétamide ;
   α-amino-N-[3-{4-(2,5-diméthylphényl)-1-pipérazinyl}-propyl]-benzèneacétamide ;
   N-[3-{4-(2,5-diméthylphényl)-1-pipérazinyl}-propyl]-α-(méthylsulfonylamino)-benzèneacétamide ;
   N-[3- {4-(2,5-diméthylphényl)-1-pipérazinyl}-propyl]-α-benzylaminocarbonylbenzèneacétamide ;
   N-[3-{4-(2,5-diméthylphényl)-1-pipérazinyl}-propyl]-α-(4-méthylphényl)-sulfonyl amino-benzèneacétamide ;
   N-[3-{4-(2,5-diméthylphényl)-1-pipérazinyl}-propyl]-α-benzylcarbonylaminobenzèneacétamide ;
   étant entendu que le terme "inférieur", lorsqu'il se réfère au radical alkyl ou alkoxy, signifie au plus 6 atomes de carbone .

Dans l'un des modes de réalisation, R₁ représente l'hydrogène ou un alkyle inférieur, R₂ représente un hydrogène, un alkyle inférieur, OR₆ (dans lequel R₆ représente un hydrogène ou un benzyle, SR₆ (dans lequel R₆ est un alkyle inférieur), NH₂, NHSO₂R₆ (dans lequel R₆ est un alkyle inférieur ou un benzyle), OC(O)R₆ (dans lequel R₆ est un benzyle ou CONR₆R₇ ou N(R₇)C(O)R₆ (dans lequel R₇ représente un hydrogène et R₆ représente un benzyle); R₅ représente un phényle substitué, dans lequel ledit substituant est un alkyle inférieur, CN, un halogène, un hydroxy-alkyle inférieur, cyclopropylhydroxyméthyle, un alcoxy inférieur-alkyle inférieur, ou OR₆ (dans lequel R₆ est un alkyle inférieur) ; et R₃ et R₄, de façon indépendante l'un de l'autre, représentent l'un des radicaux suivants substitués ou non-substitués : un alkyle inférieur, cyclohexyle, phényle, naphtyle, dans lequel ledit substituant est un halogène, OR₆ (dans lequel R₆ est un alkyle inférieur), ou un phényle.

Dans un autre mode de réalisation, R₁ représente un hydrogène et R₅ représente le 2,5-diméthyl-phényle ; R₂ représente OR₆ (dans lequel R₆ représente un hydrogène, un alkyle inférieur, ou un benzyle); et R₃ représente un alkyle inférieur et R₄ représente un phényle.

Dans un autre mode de réalisation, R₁ représente un hydrogène et R₅ représente le 2-méthoxy-phényle ou le 2,5-diméthyl-phényle ; R₂ représente OR₆ (dans lequel R₆ représente un hydrogène, un alkyle inférieur, ou un benzyle); et R₃ et R₄, ensemble avec l'atome de carbone auquel ils sont conjointement liés, constituent le 9-xanthényle ou le 9-fluorényle.

Les exemples suivants sont des exemples de composés selon la présente invention :

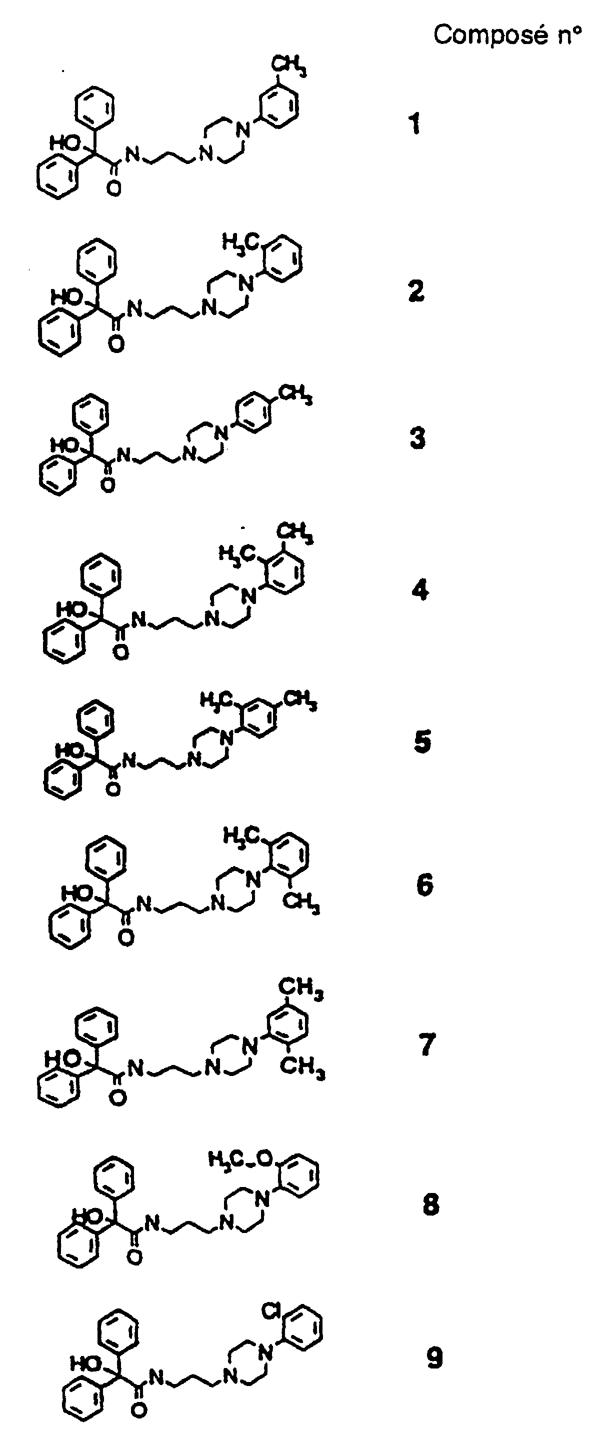

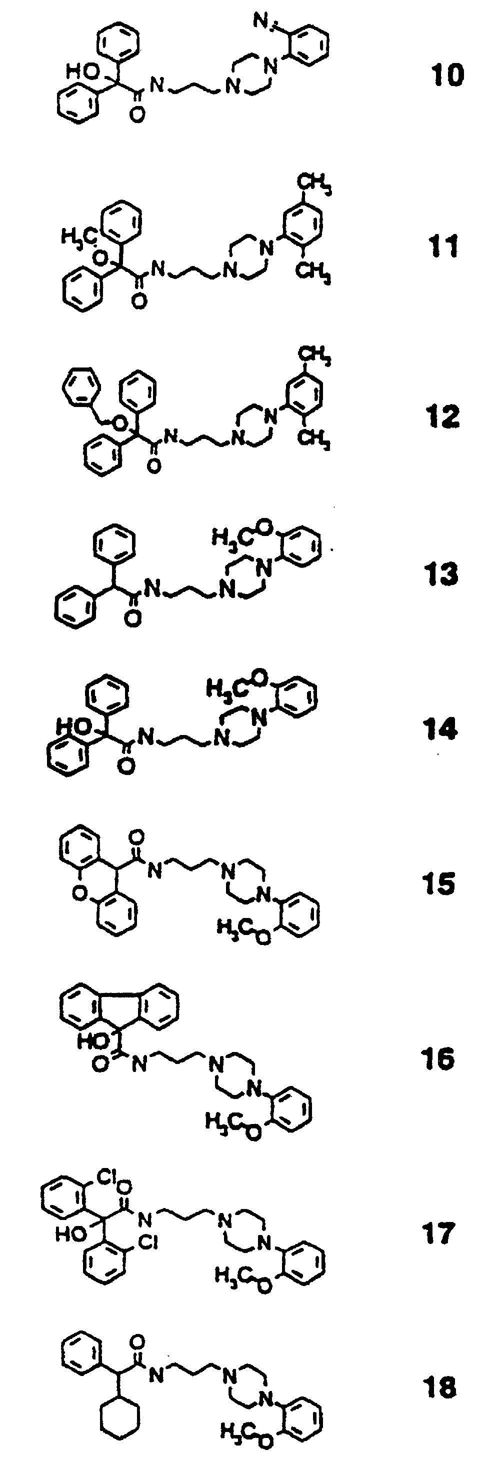

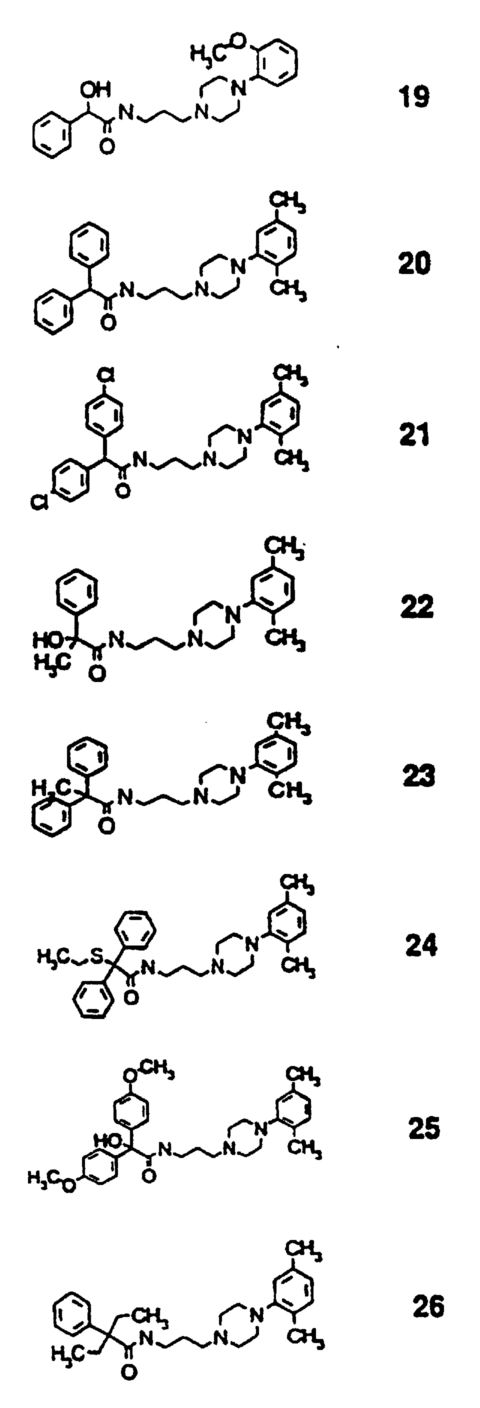

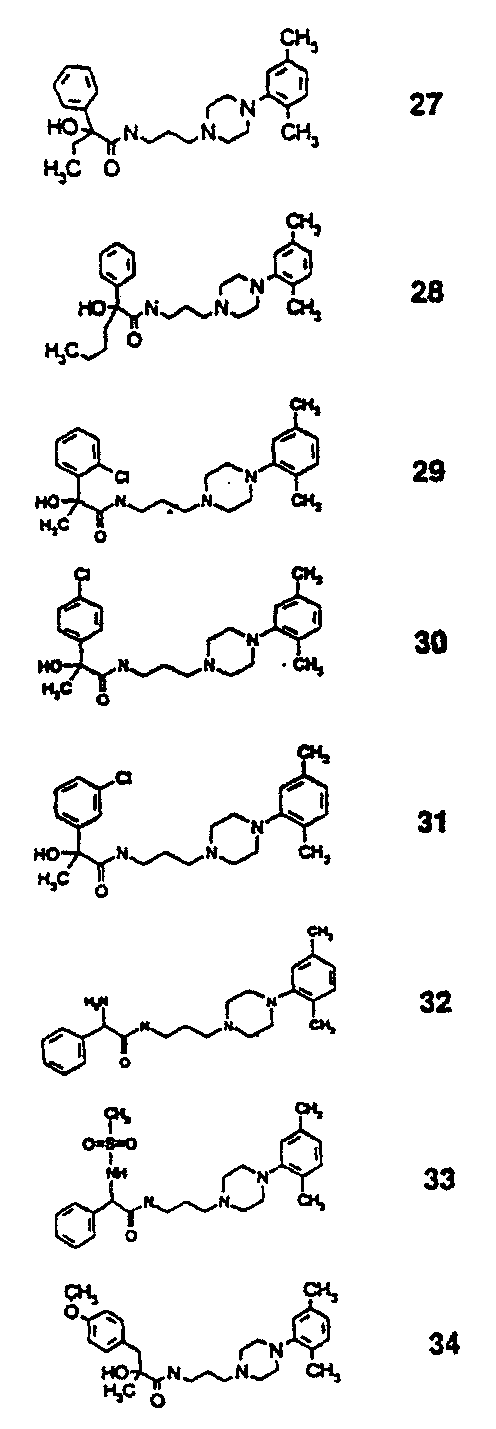

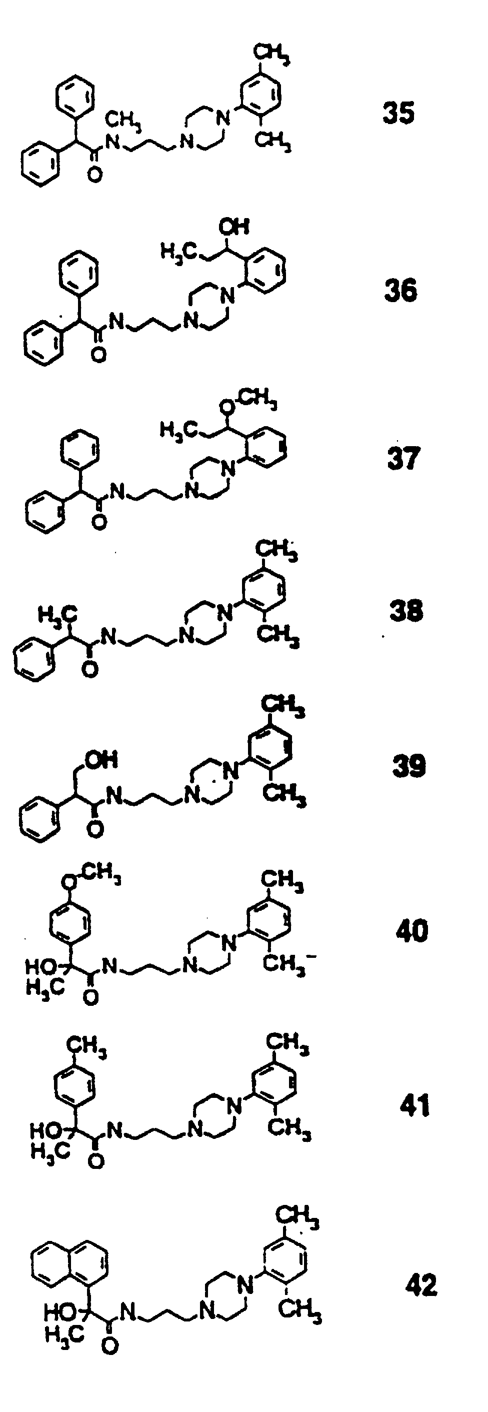

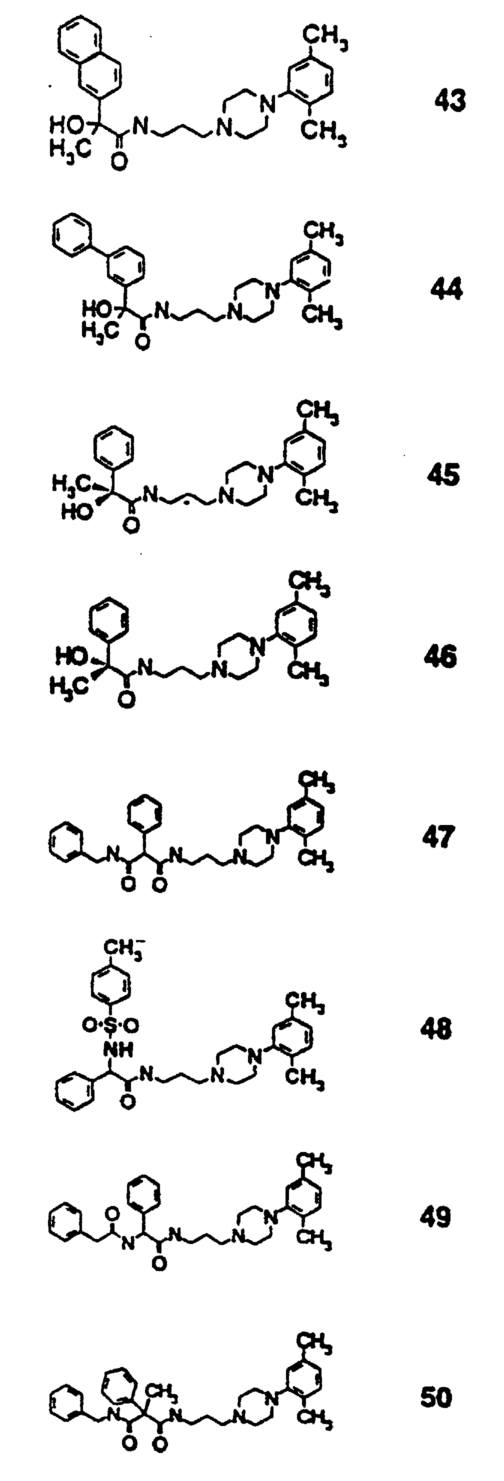

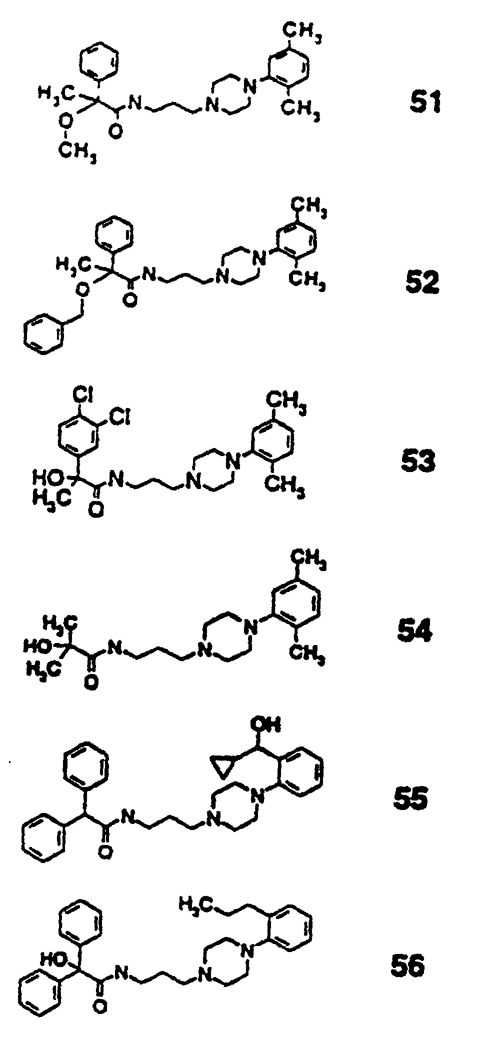

Selon un autre aspect, la présente invention concerne l'application à titre de médicaments des produits selon l'invention, ainsi qu'une composition pharmaceutique comprenant un composé selon l'invention.

Selon un mode de réalisation, R₁ représente un hydrogène, R₅ représente le 2,5-diméthyl-phényle ; R₂ représente OR₆ (dans lequel R₆ représente un hydrogène, un alkyle inférieur, ou un benzyle) ; R₃ représente un alkyle inférieur ; et R₄ représente un phényle.

Tel qu'utilisé ici, un "alkyle inférieur" inclut les groupes hydrocarbonés aliphatiques saturés à la fois linéaires, ramifiés, ou à chaîne droite, ayant de 1 à 6 atomes de carbone. Des exemples de groupes alkyles inférieurs incluent le méthyle, l'éthyle, le propyle, l'isopropyle, le butyle, le t-butyle, et similaires.

Tel qu'utilisé ici, un "alcoxy inférieur" inclut les groupes hydrocarbonés aliphatiques saturés à la fois linéaires, ramifiés, et à chaîne droite, ayant de 1 à 6 atomes de carbone et un atome d'oxygène. Des exemples d'alcoxy inférieurs incluent le méthoxy, l'éthoxy, le propoxy, l'isopropoxy, le butoxy, et similaires.

Tel qu'utilisé ici, un halogène représente un chloryle, fluoryle, brominyle, ou iodo.

Les composés de la présente invention peuvent comporter un centre asymétrique et se présenter comme des racémates, des mélanges racémiques, ou des diastéréomères individuels, avec tous les isomères possibles, y compris les isomères optiques, l'ensemble étant inclus dans la présente invention. Pour simplifier, lorsqu'aucune configuration spécifique n'est décrite dans. les formules structurales, on comprend que toutes les formes énantiomèriques et leurs mélanges sont représentées. Dans un mode de réalisation, l'atome de carbone lié à R₂, R_{3,} et R₄ est dans la configuration "S".

Les composés de cette invention peuvent être fournis sous forme de sels acceptables pharmaceutiquement. Des sels acceptables incluent, mais ne sont pas limités à, les sels acides d'addition d'acides inorganiques tels que le chlorhydrate, le sulfate, le phosphate, le diphosphate, l'hydrobromide, et le nitrate ou d'acides organiques tels que l'acétate, le matéate, le fumarate, le tartrate, le succinate, le citrate, le lactate, le méthanesulfonate, le p-toluènesulfonate, le pamoate, le salicylate, l'oxalate, et le stéarate. Sont également compris dans la présente invention, lorsque cela est possible, les sels formés à partir de bases comme l'hydroxyde de sodium ou de potassium. Pour d'autres exemples de sels acceptables pharmaceutiquement, voir "Pharmaceutical Salts", J. Pharm. Sci. 66:1 (1977).

La présente invention concerne également une méthode de traitement de troubles dont le récepteur α₁-adrénergique constitue un médiateur. Des exemples de tels troubles incluent l'hypertension, les troubles de l'appareil urinaire inférieur (par exemple l'hyperplasie bénigne de la prostate), l'arythmie, l'ischémie myocardique, les concentrations lipidiques sériques élevées, le glaucome, les troubles de la motilité de l'appareil gastro-intestinal, la synthèse du cholestérol, le dysfonctionnement érectile, la maladie de Raynaud, les pathologies congestives cardiaques, l'hypertension portale, et la résistance pluri-médicamenteuse, la cirrhose.

L'invention concerne également l'utilisation des produits de l'invention, pour la préparation d'un médicament destiné à traiter l'hyperplasie bénigne de la prostate, l'hypertension portale et la cirrhose.

Une quantité thérapeutique efficace d'un composé de ladite invention et une substance support pharmaceutiquement acceptable (par exemple, le carbonate de magnésium, le lactose, ou un phospholipide avec lequel le composé thérapeutique peut former une micelle) forment ensemble une composition pharmaceutique (par exemple, une pilule, un comprimé, une capsule, ou un liquide) pour l'administration (par exemple, par voie orale, intraveineuse, transdermique, ou sous-cutanée) à un sujet ayant besoin du composé. La pilule, le comprimé, ou la capsule peut être recouvert d'une substance capable de protéger la composition des acides gastriques ou des enzymes intestinales dans l'estomac du sujet pendant une période de temps suffisante pour permettre à la composition de passer à l'état non digéré dans l'intestin grêle du sujet

La dose d'un composé de la présente invention pour le traitement des maladies ou troubles mentionnés ci-dessus varie en fonction du mode d'administration, de l'âge et du poids corporel du sujet, et de la condition du sujet à traiter, et en définitive elle sera décidée par le médecin ou vétérinaire traitant. Une telle quantité du composé telle que déterminée par le médecin ou le vétérinaire traitant sera appelée ici une "quantité thérapeutique efficace".

Est également considérée comme faisant partie de l'invention une méthode de préparation du composé de formule (I) et les intermédiaires chimiques nouveaux utilisés dans ces synthèses telles que décrits ici.

D'autres caractéristiques et avantages de la présente invention seront apparents à partir de la description détaillée de l'invention et à partir des revendications.

On considère qu'un homme de l'art peut, en se basant sur la description donnée ici, utiliser la présente invention dans toute son étendue. Les modes de réalisation spécifiques suivants ne sont ainsi mentionnés qu'à titre simplement illustratif, et ne limitent pas la portée de la divulgation de quelque façon que ce soit.

A moins qu'ils ne soient définis autrement, tous les termes techniques et scientifiques utilisés ici ont la même signification que celle couramment comprise par l'homme de l'art ordinaire auquel cette invention s'applique.

### Synthèse

Comme cela est représenté sur le Schéma I, les composés de l'invention peuvent généralement être préparés par condensation de dérivés du 3-amino-1-propanol de formule générale (II) (Kim, J.K. et al., J. Org. Chem. 54:1714 (1989) et Koepke, S.R. et al., J. Org. Chem. 44:2718 (1979)) avec les dérivés acides appropriés de formule générale (III).

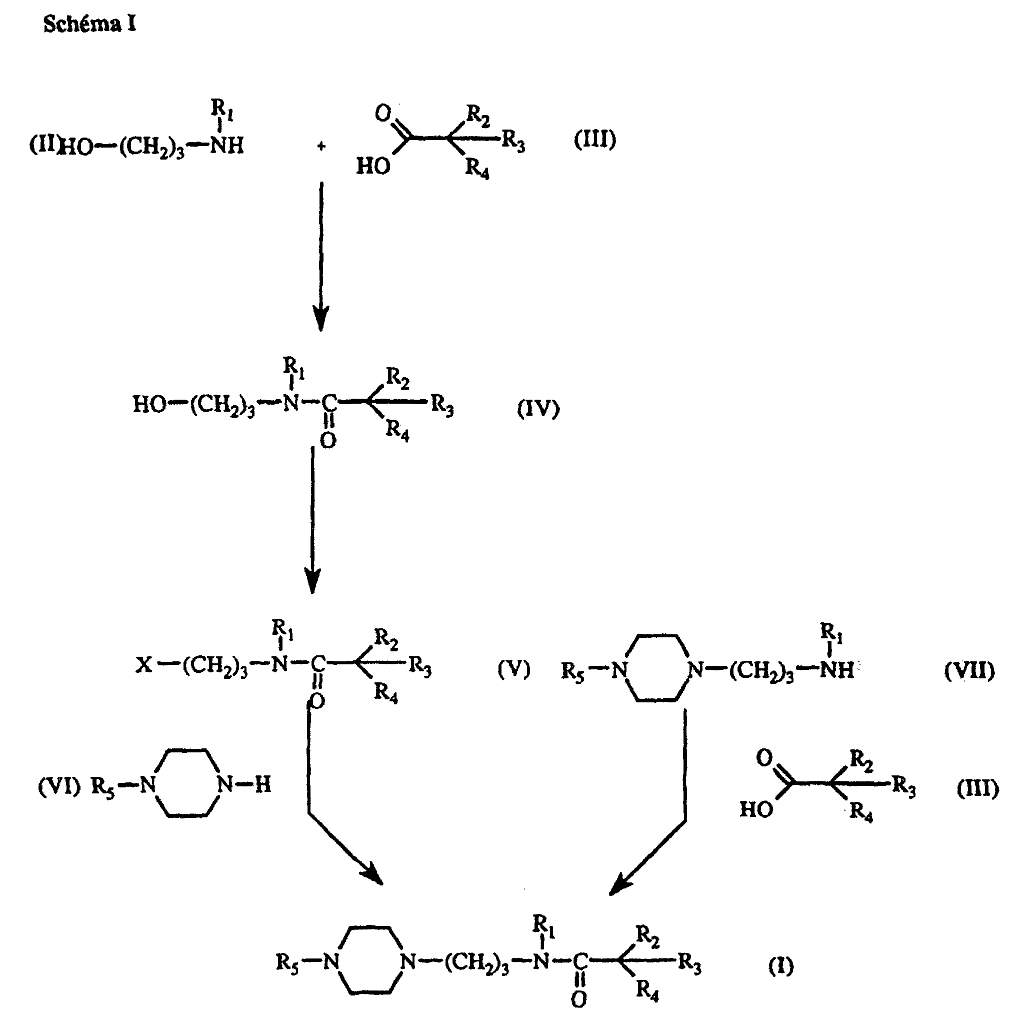

Dans le Schéma I, R₁-R₅ sont définis ci-dessus et X représente un groupement éliminable; par exemple, le méthane-sulfonyloxy, le p-toluène-sulfonyloxy, le chlore, le brome ou l'iode. Des dérivés de l'acide benzylique (III), dans lesquels R₃ et R₄ représentent des aromatiques et R₂ représente OH, sont obtenus soit en chauffant à 80° C des dérivés benzyles et un hydroxyde de métal alcalin (par exemple, KOH) à l'état solide (Toda, F. et al., Chem. Letts. pp. 373-376, The Chemistry Society of Japan (1990)), tel qu'illustré par l'exemple 25, soit en chauffant à 95° C dans des solvants organiques aqueux (Rzeszotarski, W.J. et al., J. Med. *Chem.* 27:156 (1984)).

Des dérivés acides (III), dans lesquels R₃ représente une chaîne alkyle, R₄ représente un aromatique, et R₂ représente OH, peuvent être obtenus à partir d'autres méthodes. Une méthode implique la réaction de dérivés de l'acétophénone avec le triméthylsilyl-cyanure en présence d'une quantité catalytique de ZnI₂ à température ambiante pour obtenir des cyanohydrines après hydrolyse acide (Gassman, P.G. et al., Tetrahedron Lett. 40:3773 (1978)). Ces cyanohydrines sont ensuite transformées en acides α-hydroxy en les faisant refluer dans de l'HCl concentré (Org. Synth. Coll. Vol. 1:289 (1941)). Une autre méthode implique l'alkylation de trianions d'acides α-hydroxycarboxyliques à -78° C avec des chlorures d'alkyle (Newcomb M., et al., J. Org. Chem. 43:3963 (1978)) ce qui conduit également aux acides alkylés souhaités tel qu'illustré par l'exemple 28. Encore une autre méthode est la synthèse d'esters α-aryl-alcanoïques à partir d'α-cétoesters, par exemple l'éthyl-pyruvate, et de nucléophiles aryles tels que les bromures d'aryle magnésium (Salomon, R.G. et al., J. Org. Chem. 47:4692 (1982)). Les dérivés d'aryle magnésium sont de façon pratique préparés en faisant refluer du bromure d'aryle et de la poudre de magnésium dans du THF ou du diéthyl-éther sec ou anhydre ("dry"). L'étape de condensation a lieu en faisant passer par l'intermédiaire de tubulures ("cannulating") le dérivé froid d'aryle magnésium sur l'α-cétoester préalablement refroidi (-78° C) tel qu'illustré par l'exemple 40. La saponification est facilement effectuée en agitant ces esters en présence de KOH dans du méthanol pendant plusieurs heures. Des esters α-aryl-α-hydroxy alcanoïques chiraux sont accessibles principalement par l'intermédiaire de l'addition de réactifs organométalliques sur des α-cétoesters chiraux (Basavaiah, D. and Bharathi, T.K., Tetrahedron Lett. 32:3417 (1991)) en utilisant, par exemple, le trans-2-phénylcyclohexanol comme auxiliaire chiral.

Les dérivés acides (III) dans lesquels R₃ représente un hydrogène ou une chaîne alkyle, R₄ représente un aromatique, et R₂ représente un aminocarbonyle sont préparés de façon pratique à partir de diesters phénylmaloniques. L'étape optionnelle d'alkylation a lieu, dans des conditions anhydres, en présence d'une base forte telle que NaH et d'un agent d'alkylation R₆-X, tel que défini ci-dessus, dans un solvant polaire sec, par exemple le DMF ou le THF. La saponification avec un équivalent de KOH éthanolique à température ambiante permet l'accès à des dérivés mono-acides qui sont facilement transformés en dérivés carboxamides par réaction avec une amine NR₆R₇, telle que définie ci-dessus, dans des conditions peptidiques classiques. Cette étape de condensation a lieu en présence de dicyclohexylcarbodiimide et d'hydroxybenzotriazole comme réactifs de couplage dans un solvant inerte de préférence le dichlorométhane ou le DMF (Bodanszky, M. et Bodanszky, A., The Practice of Peptide Synthesis, 145 (Springer-Verlag, 1984)). De façon alternative, ces dérivés carboxamides sont accessibles par la condensation directe d'une amine NR₆R₇, telle que décrite ci-dessus, avec des diesters phénylmaloniques en les chauffant dans un mélange de solvants tel que THF/Toluène en présence de sodiumdiéthyldihydroaluminate (Sim, T.B. et Yoon, N.M., Synlett 827 (1994)). La dégrotection de la seconde fonction acide est effectuée exactement tel que décrit précédemment.

La réaction de couplage peptidique de composés de formules générales (II) et (III) est bien connue dans l'art antérieur (Bodanszky, M. et Bodanszky, A., The Practice of Peptide Synthesis, 145 (Springer-Verlag, 1984)). Le groupe hydroxyle primaire de composés de formule générale (IV) peut être facilement converti en un groupe éliminable, par exemple un groupe sulfonyloxy, par réaction avec un chlorure de méthanesulfonyle en présence de triéthylamine dans du dichlorométhane ou du chlorure de p-toluènesulfonyle dans de la pyridine. Lorsque R₂ représente OH, l'activation sélective de l'alcool primaire sera effectuée seulement grâce à des réactions de type halogénation, par exemple avec des iodures de formule générale (V), dans laquelle X représente I, qui sont préparés de façon pratique par réaction avec de l'iode, triphénylphosphine, et imidazole (Garegg, P.J. et al., J. Chem. Soc. Perkin 1, 681 (1982)) tel qu'illustré par l'intermédiaire synthétisé dans l'exemple 1.2. La réaction de condensation d'un composé de formule générale (V) avec un composé de formule générale (VI) (voir schéma I) peut être réalisée en chauffant le mélange à 80° C dans un solvant polaire; par exemple l'acétonitrile, pendant plusieurs heures.

De façon alternative, des composés de formule générale (I) peuvent être préparés par une condensation peptidique classique d'un dérivé d'amine primaire de formule générale (VII) (pour une préparation pratique voir Wu, Y.H. et al., J. Med. Chem. 12:876 (1969)) avec un dérivé d'acide de formule générale (III).

Comme cela est représenté dans le Schéma II, des composés de formule générale (VI), par exemple les formules (VIa), (VIb) et (VIc), avec un alkyle inférieur, un hydroxyalkyle, ou un alcoxyalkyle comme substituants, peuvent généralement être préparés.

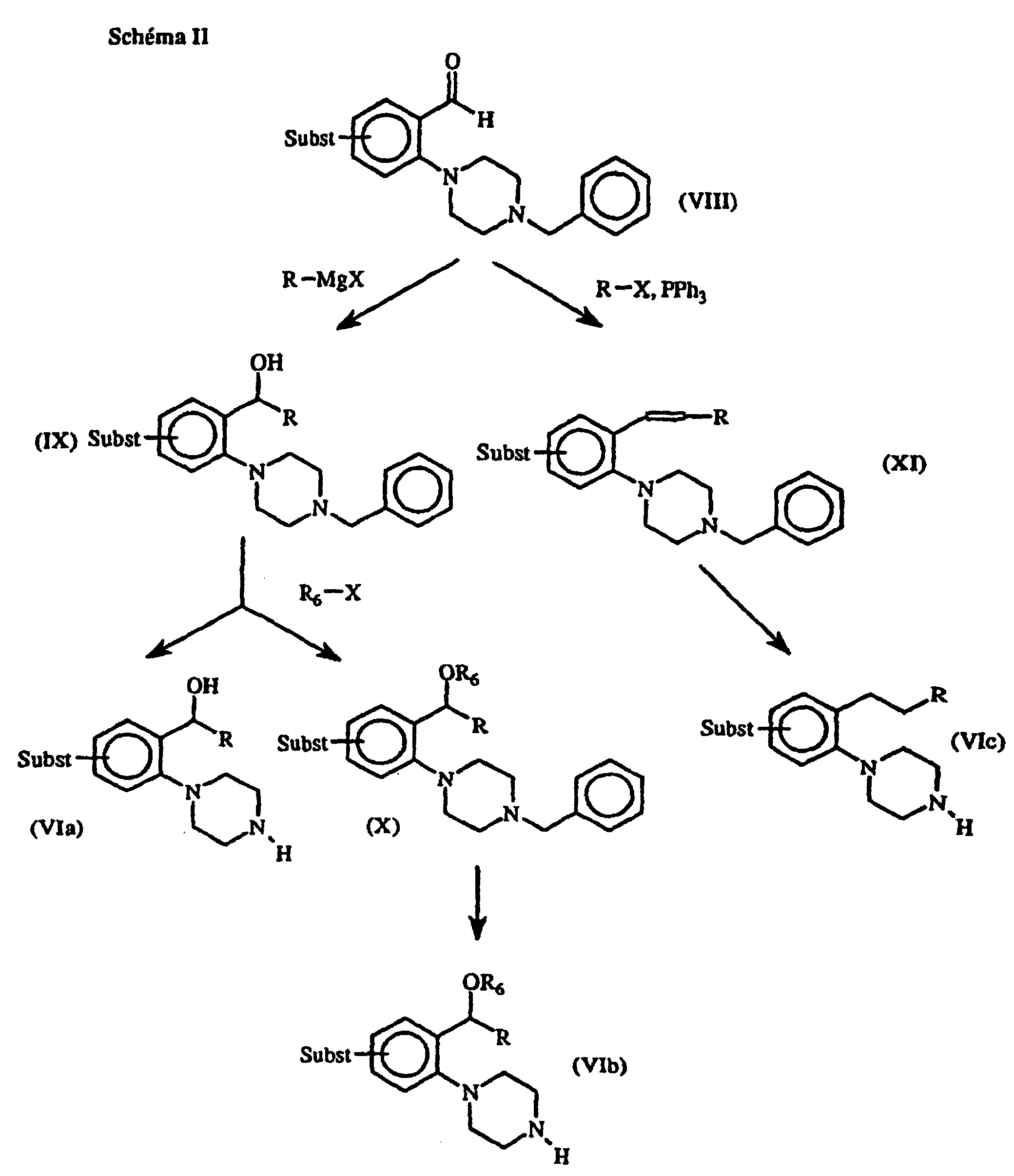

Dans le Schéma II, R₆ tel que défini ci-dessus, représente un groupe alkyle, Subst représente un substituant tel que défini dans R₅, et X représente un groupe éliminable, par exemple le méthanesulfonyloxy, le p-toluènesulfonyloxy, un chlore, un brome ou un iode. La condensation entre des dérivés benzaldéhydes de formule générale (VIII) (Walter, H.N. et al., Synthesis, pp. 641-645 (1987)) et des réactifs de Grignard alkyles dans du diéthyléther ou THF permet d'obtenir l'alcool secondaire correspondant de formule générale (IX), tel qu'illustré par l'intermédiaire 36.2. Les composés de formule générale (VIa) sont obtenus par déprotection de composés de formule générale (IX) selon des méthodes générales connues de débenzylation, par exemple l'hydrogénation catalytique ou la réaction avec un chlorofonnate, tel que le vinylchloroformate ou l'α-chloroéthylchloroformate, suivie d'une hydrolyse ou méthanolyse. D'autres méthodes de débenxylation telles que celles trouvées dans Green, T.W. et Wuts, P.G.M., Protective Groups in Organic Synthesis, 2d ed., 364, (John Wiley & Sons Inc., 1991) peuvent également être utilisées à condition qu'elles soient compatibles avec les substituants du cycle aryle du composé de formule générale (1X). Les composés de formule générale (IX) peuvent être O-alkylés dans des conditions anhydres à terapérature ambiante en présence d'une base forte, par exemple NaH, et d'un agent d'alkylation R₆-X, tel que défini ci-dessus, dans un solvant polaire sec, par exemple DMF ou THF. La débenzylation des composés de formule générale (X) suivant la procédure déjà décrite permet l'obtention de composés de formule générale (VIb).

De façon alternative, les composés de formule générale (VIII) peuvent être transformés en un composé de formule générale (XI) par l'intermédiaire d'une réaction de type réaction de Wittig impliquant un réactif alkylphosphorane généré à partir d'un sel d'alkylphosphonium et d'une base forte, par exemple nBuLi ou phényllithium, dans du THF ou du diéthyléther sec. Les composés de formule générale (VIc) sont obtenus par l'intermédiaire d'une réduction de la double liaison et d'une débenzylation concommittantes dans des conditions d'hydrogénation catalytique.

Les composés de formule générale (I), dans laquelle R₂ représente OH, peuvent être O-alkylés dans des conditions anhydres en présence d'une base forte telle que NaH et d'un agent d'alkylation R₆-X dans un solvant polaire sec, par exemple le DMF ou le THF. Les composés de formule générale (I), dans laquelle R₂ représente une amine modifiée (par exemple un sulfonyle amide ou un carboxamide), sont synthésisés par réaction d'un chlorosulfonyle ou d'un dérivé acide halogéné sur un amino-acide libre dans des conditions analogues à celles de la procédure Schotten-Baumann (Bodanszky, M. et Bodanszky, A., The Practice of Peptide Synthesis, 9 (Springer-Verlag, 1984)).

Ce qui suit décrit la synthèse chimique spécifique des composés 1 à 56. D'autres composés selon l'invention peuvent être préparés de manière analogue par un homme de l'art ordinaire.

### Exemple 1 : α-hydroxy-N-[3-{4-(3-méthylphényl)-1-pipérazinyl}-propyl]-α-phényl-benzèneacétamide, dichlorhydrate (Composé 1) :

### 1.1) α-hydroxy-N-(3-hydroxypropyl)-α-phényl-benzèneacétamide :

11,34 g (55 mmol) de dicyclohexylcarbodiimide sont ajoutés en une fois à une solution froide (0-5° C) de 3,82 ml (50 mmol) de 3-amino-1-propanol, 7,43 g (55 mmol) d'hydroxybenzotriazole et 11,4 g (50 mmol) d'acide benzylique dans 60 ml de DMF. La solution est laissée à réchauffer jusqu'à température ambiante et est agitée pendant 15 heures. La filtration de la dicyclohexylurée précipitée suivie de l'évaporation de la liqueur mère permet l'obtention d'un résidu huileux qui est dissous dans 150 ml d'acétate d'éthyle. Cette solution est rincée successivement avec de l'eau (2 x 100 ml), NaOH 1N (2 x 100 ml) et une solution saturée de NaCl (100 ml). La phase organique est séchée sur du sulfate de sodium et le solvant est évaporé sous pression réduite pour produire 12,8 g (89 %) du composé titre sous forme d'un solide blanc ; p.f.: 12b-127° C.

RMN-¹H (100 MHz, CDCl₃, δ) : 7,40(m, 10H, 2 x Ph) ; 6,95(m, 1H, CO-NH); 3,98 (s, 1H, OH) ; 3,45 (m, 4H, CH₂-NH, CH₂-OH) ; 2,88 (m, 1H, CH₂-OH) ; 1,63 (m, 2H, CH₂).

### 1.2) α-hydroxy-N-(3-iodopropyl)-α-phényl-benzèneacétamide :

14,26 g (56 mmol) d'iode sont ajoutés en plusieurs fois à une solution froide (0-5° C) de 14,7 g (56 mmol) de triphénylphosphine et 3,82 g (56 mmol) d'imidazole dans un mélange de 90 ml de diéthyléther et 30 ml d'acétonitrile. Un précipité jaune se forme rapidement pendant l'agitation à température ambiante pendant une heure. A cette suspension jaune, on ajoute goutte à goutte une solution de 8 g (28 mmol) de α-hydroxy-N-(3-hydroxypropyl)-a-phényl-benzèneacétamide dans 90 ml de diéthyléther et 30 ml d'acétonitrile, et l'agitation est poursuivie pendant 15 heures. Le mélange résultant est filtré et concentré de façon à obtenir un résidu huileux marron qui est purifié par chromatographie flash (éluent : pétrole léger (bp. 40-65° C) / acétate d'éthyle : 8/2 puis 6/4). L'évaporation des phases appropriées sous pression réduite permet l'obtention de 8,86 g (80 %) du composé titre sous forme d'un solide jaune pâle ; p.f. : 105-106° C.

RMN-¹H (100 MHz, CDCl₃, δ) : 7,35 (m, 10H, 2 x Ph) ; 6,57 (m, 1H, CO-NH) ; 3,81 (s, 1H, OH) ; 3,40 (m, 2H, CH₂-NH) ; 3,09 (t, 2H, CH₂-I) ; 2,00 (m, 2H, CH₂).

### 1.3) α-hydroxy-N-[3-{4-(3-méthylphényl)-1-pipérazinyl}-propyl]-α-phényl-benzèneacétamide, dichlorhydrate :

Un mélange de 0,6 g (3,4 mmol) de 1-(3-méthylphényl)-pipérazine et 1,34 g (3,4 mmol) de α-hydroxy-N-(3-iodopropyl)-α-phényl-henzèneacétamide dans 20 ml d'acétonitrile est chauffé au reflux pendant 3 heures. Après refroidissement jusqu'à température ambiante, le mélange réactionnel est concentré sous pression réduite. Le résidu huileux est dissous dans 50 ml d'acétate d'éthyle et rincé avec NaOH 1N (2 x 50 ml) et de l'eau (2 x 50 ml). Après le traitement habituel, le produit brut est purifié par chromatographie flash (éluent : acétate d'éthyle). L'évaporation du solvant sous pression réduite donne 0,78 g (52 %) de la base du Composé 1 qui est transformée en son sel de chlorhydrate par une solution éthérée de chlorure d'hydrogène ; p.f. : 195-196° C.

RMN-¹H (400 MHz, DMSO d6, δ) : 11,04 (bs, 1H, ⁺NH) ; 8,42 (t, 1H, NH-CO) ; 7,35 (m, 10H, 2 x Ph) ; 7,14 (m, 1H, Ph-CH₃) ; 6,80 (m, 2H, Ph-CH₃) ; 6,70 (m, 1H, Ph-CH₃) ; 6,34 (bs, 1H, OH); 3,75 (d, 2H, ⁺NH-CH₂) ; 3,45 (d, 2H, CH₂-NH-CO) ; 3,10 (m, 8H, pipérazine) ; 2,27 (s, 3H, CH₃) ; 1,91 (m, 2H, CH₂).

Analyse élémentaire pour C₂₈H₃₃N₃O₂, 2 HCl : % Calc : C, 65,11 ; H, 6,83 ; N, 8,14 ; % obtenus : C, 65,02 ; H, 6,80 ; N, 8,10.

### Exemple 2 : α-hydroxy-N-[3-{4-(2-méthylphényl)-1-pipérazinyl}-propyl]-α-phényl-benzèneacétamide, dichlorhydrate. 0,5 H₂O (Composé 2) :

Le composé 2 est préparé d'une manière analogue à celle de l'exemple 1, sauf que la 1-(2-méthylphényl)-pipérazine est utilisée à la placé de la 1-(3-méthylphényl)-pipérazine.

RMN-¹H (400 MHz, DMSO d6, δ) : 10,97 (bs, 1H, +NH) ; 8,43 (t, 1H, NH-CO) ; 7,34 (m, 10H, 2 x Ph) ; 7,18 (m, 2H, Ph-CH₃) ; 7,00 (m, 2H, Ph-CH₃) ; 4,49 (bs, 1H, OH) ; 3,44 (d, 2H, ⁺NH-CH₂) ; 3,23 (m, 2H, CH₂-NHCO) ; 3,09 (m, 8H, pipérazine) ; 2,26 (s, 3H, CH₃) ; 1,94 (m, 2H, CH₂).

Analyse élémentaire pour C₂₈H₃₃N₃O₂, 2 HCl, 0,5 H₂O : % Calc : C, 64,00 ; H, 6,90 ; N, 8,00; % obtenus : C, 63,89 ; H, 6,87 ; N, 7,92 ; p.f.: 191-192° C.

### Exemple 3 : α-hydroxy-N-[3-{4-(4-méthylphényl)-1-pipérazinyl}-propyl]-α-phényl-benzèneacétamide, dichlorhydrate (Composé 3) :

Le composé 3 est préparé d'une manière analogue à celle de l'exemple 1, sauf que la 1-(4-méthylphényl)-pipérazine est utilisée à la place de la 1-(3-méthylphényl)-pipérazine ; p.f.: 204,5° C.

RMN-¹H (400 MHz, DMSO d6, δ) : 11,00 (bs, 1H, ⁺NH) ; 8,42 (t, 1H, NH-CO) ; 7,33 (m, 10H, 2 x Ph) ; 7,00 (m, 4H, Ph-CH₃) ; 6,78 (bs, 1H, OH) ; 3,68 (d, 2H, ⁺NH-CH₂) ; 3,45 (d, 2H, CH₂-NH-CO) ; 3,15 (m, 8H, pipérazine) ; 2,22 (s, 3H, CH₃) ; 1,93 (m, 2H, CH₂).

Analyse élémentaire pour C₂₈H₃₃N₃O₂, 2 HCl : % Calc : C, 65,11 ; H, 6,83 ; N, 8,14 ; % obtenus : C, 64,91 ; H, 6,89 ; N, 8,11.

### Exemple 4 : N-[3-{4-(2,3-diméthylphényl)-1-pipérazinyl}-propyl]-α-hydroxy-α-phényl-benzèneacétamide, chlorhydrate, 0,25 H₂O (Composé 4) :

Le composé 4 est préparé d'une manière analogue à celle de l'exemple 1, sauf que la 1-(2,3-diméthylphényl)-pipérazine est utilisée à la place de la 1-(3-méthylphényl)-pipérazine ; p.f. : 125-126° C.

RMN-¹H (400 MHz, DMSO d6, δ) : 11,05 (bs, 1H, ⁺NH) ; 8,43 (t, 1H, NH-CO) ; 7,39 (m, 10H, 2 x Ph) ; 7,07 (m, 1H, Ph-CH₃) ; 6,93 (m, 1H, Ph-CH₃) ; 6,89 (m, 1H, Ph-CH₃) ; 4,34 (bs, 1H, OH) ; 3,43 (d, 2H, ⁺NH-CH₂) ; 3,23 (m, 2H, CH₂ -NH-CO) ; 3,06 (m, 8H, pipérazine) ; 2,22 (s, 3H, CH₃) ; 2,17 (s, 3H, CH₃) ; 1,95 (m, 2H, CH₂).

Analyse élémentaire pour C₂₉H₃₅N₃O₂, HCl, 0,25 H₂O : % Calc : C, 69,86 ; H, 7,38 ; N, 8,43 ; O, 7,22 ; % obtenus : C, 69,93 ; H, 7,63 ; N, 8,53 ; O, 7,41.

### Exemple 5 : N-[3-{4-(2,4-diméthylphényl)-1-pipérazinyl}-propyl]-α-hydroxy-α-phényl-benzèneacétamide, fumarate, monohydrate (Composé 5) :

Le composé 5 est préparé d'une manière analogue à celle de l'exemple 1, sauf que la 1-(2,4-diméthylphényl)-pipérazine est utilisée à la place de la 1-(3-méthylphényl)-pipérazine ; p.f.: 177° C.

RMN-¹H (400 MHz, DMSO d6, δ) : 8,35 (t, 1H, NH-CO) ; 7,35 (m, 10H, 2 x Ph); 6,93 (m, 3H, Ph-CH₃) ; 6,75 (bs, OH, COOH) ; 6,62 (s, 2H, CH=CH) ; 3,39 (m, 2H, CH₂-NH-CO) ; 2,87 (bs, 4H, pipérazine) ; 2,71 (bs, 4H, pipérazine) ; 2,56 (m, 2H, N-CH₂) ; 2,20 (s, 6H, 2 x CH₃) ; 1,71 (m, 2H, CH₂).

Analyse élémentaire pour C₂₉H₃₅N₃O₂, fumarate, monohydrate : % Calc : C, 66,99 ; H, 6,98 ; N, 7,10 ; O, 18,93 ; % obtenus : C, 67,21 ; H, 6,72 ; N, 6,87 ; O, 18,80.

### Exemple 6 : N-[3-{4-(2,6-diméthylphényl)-1-pipérazinyl}-propyl]-α-hydroxy-α-phényl-benzèneacétamide, chlorhydrate, 0,5 H₂O (Composé 6) :

Le composé 6 est préparé d'une manière analogue à celle de l'exemple 1, sauf que la 1-(2,6-diméthylphényl)-pipérazine est utilisée à la place de la 1-(3-méthylphényl)-pipérazine ; p.f. : 183-184° C.

RMN-¹H (400 MHz, DMSO d6, δ) : 10,59 (bs, 1H, ⁺NH) ; 8,44(t, 1H, NH-CO); 7,35 (m, 10H, 2 x Ph) ; 7,04 (m, 1H, Ph-CH₃) ; 6,97 (m, 2H, Ph-CH₃) ; 3,65 (t, 2H, ⁺NH-CH₂) ; 3,34 (bs, 1H, OH) ; 3,71-2,93 (m, 8H, pipérazine) ; 3,22 (m; 2H,. CH₂-NHCO) ; 2,29 (s, 3H, CH₃) ; 2,27 (s, 3H, CH₃) ; 1,93 (m, 2H, CH₂).

Analyse élémentaire pour C₂₉H₃₅N₃O₂, HCl, 0,5 H₂O : % Calc : C, 69,24; H, 7,41; N, 8,35 ; O, 7,95 ; % obtenus : C, 69,59 ; H, 7,72 ; N, 8,42 ; O, 7,65.

### Exemple 7 : N-[3-{4-(2,5-diméthylphényl)-1-pipérazinyl}-propyl]-α-hydroxy-α-phényl-benzèneacétamide, 1,5 HCl, 0,25 H₂O (Composé 7) :

Le composé 7 est préparé d'une manière analogue à celle de l'exemple 1, sauf que la 1-(2,5-diméthylghényl)-pipérazine est utilisée à la place de la 1-(3-méthylphényl)-pipérazine; p.f.: 171-172° C.

RMN-¹H (400 MHz, DMSO d6, δ) : 11,01 (bs, 1H, ⁺NH) ; 8,43 (t, 1H, NH-CO) ; 7,34 (m, 10H, 2 x Ph) ; 7,06 (d, 1H, Ph-CH₃, J = 7,5 Hz) ; 6,82 (d, 2H, Ph-CH₃) ; 6,90 (bs, 1H, OH) ; 3,43 (m, 2H, ⁺NH-CH₂) ; 3,22 (m, 2H, CH₂-NHCO) ; 3,10 (m, 8H, pipérazine) ; 2,26 (s, 3H, CH₃) ; 2,20 (s, 3H, CH₃) ; 1,93 (m, 2H, CH₂).

Analyse élémentaire pour C₃₀H₃₇N₃O₂, 1,5 HCl, 0,25 H₂O : % Calc : C, 67,40 ; H, 7,22 ; N, 8,13 ; O, 6,97 ; % obtenus : C, 67,10 ; H, 7,17 ; N, 8,22 ; O, 7,25.

### Exemple 8 : N-[3-{4-(2-éthoxyphényl)-1-pipérazinyl}-propyl]-α-hydroxy-α-phényl-benzèneacétamide, 2 HCl, 0,25 H₂O (Composé 8) :

Le composé 8 est préparé d'une manière analogue à celle de l'exemple 1, sauf que la 1-(2-éthoxyphényl)-pipérazine est utilisée à la place de la 1-(3-méthylphényl)-pipérazine ; p.f. : 189°C.

RMN-¹H (400 MHz, DMSO d6, δ) : 11,01 (bs, 1H, ⁺NH) ; 8,43 (t, 1H, NH-CO) ; 7,34 (m, 10H, 2 x Ph) ; 6,96 (m, 4H, Ph-N) ; 5,36 (bs, 1H, OH) ; 4,04 (q, 2H, OCH₂, J = 6,95 Hz) ; 3,48 (m, 4H, ⁺NH-CH₂ + pipérazine) ; 3,22 (m, 2H, CH₂-NHCO) ; 3,05 (m, 6H, pipérazine) ; 1,93 (m, 2H, CH₂) ; 1,37 (t, 3H, CH₃).

Analyse élémentaire pour C₂₉H₃₅N₃O₃, 2 HCl, 0,25 H₂O : % Calc : C, 63,21 ; H, 6,86 ; N, 7,63 ; O, 9,44 ; % obtenus : C, 63,16 ; H, 6,85 ; N, 7,60 ; O, 9,64.

### Exemple 9 : N-[3-{4-(2-chlorophényl)-1-pipérazinyl}-propyl]-α-hydroxy-α-phényl-benzèneacétamide, fumarate (Composé 9) :

Le composé 9 est préparé d'une manière analogue à celle de l'exemple 1, sauf que la 1-(2-chlorophényl))-pipérazine est utilisée à la place de la 1-(3-méthylphényl)-pipérazine ; p.f.: 190° C.

RMN-¹H (400 MHz, DMSO d6, δ) : 8,38 (t, 1H, NH-CO) ; 7,34 (m, 12H, 2 x Ph + Ph-Cl) ; 7,16 (m, 1H, Ph-Cl) ; 7,03 (m, 1H, Ph-Cl) ; 6,68 (bs, 1H, OH) ; 6,61 (s, 2H, CH=CH) ; 3,20 (m, 2H, CH₂-NHCO) ; 3,00 (bs, 4H, pipérazine) ; 2,58 (bs, 4H, pipérazine) ; 2,43 (m, 2H, CH₂-N) ; 1,66 (m, 2H, CH₂).

Analyse élémentaire pour C₂₇H₃₀ClN₃O₂, fumarate : % Calc : C, 64,19 ; H, 5,91 ; N, 7,24 ; % obtenus : C, 64,13 ; H, 5,90 ; N, 7,27.

### Exemple 10 : N-[3-{4-(2-cyanophényl)-1-pipérazinyl}-propyl]-α-hydroxy-α-phényl-benzèneacétamide, chlorhydrate, monohydrate (Composé 10) :

Le composé 10 est préparé d'une manière analogue à celle de l'exemple 1, sauf que la 1-(2-cyanophényl)-pipérazine est utilisée à la place de la 1-(3-méthylphényl)-pipérazine ; p.f. : 168° C.

RMN-¹H (400 MHz, DMSO d6, δ) : 10,91 (bs, 1H, ⁺NH) ; 8,43 (t, 1H, NH-CO) ; 7,78 (m, 1H, Ph-CN) ; 7,66 (m, 1H, Ph-CN) ; 7,34 (m, 12H, 2 x Ph + Ph-CN) ; 3,57 (m, 4H, ⁺NH-CH₂, + pipérazine) ; 3,35 (m, OH + pipérazine) ; 3,18 (m, 2H, CH₂-NHCO) ; 3,10 (m, 4H, pipérazine) ; 1,93 (m, 2H, CH₂).

Analyse élémentaire pour C₂₈H₃₀N₄O₂, HCl, H₂O : % Calc : C, 66,07 ; H, 6,53 ; N, 11,01 ; % obtenus : C, 65,91 ; H, 6,50 ; N, 11,01.

### Exemple 11 : N-[3-{4-(2,5-diméthylphényl)-1-pipérazinyl}-propyl]-α-méthoxy-α-phényl-benzèneacétamide, chlorhydrate, monohydrate (Composé 11) :

0,51 g (1 mmol) de N-[3-{4-(2,5-diméthylphényl)-1-pipérazinyl}-propyl]-α-hydroxy-α-phényl-benzéneacétamide (Exemple 7) est introduit dans un ballon tricol anhydre équipé d'une arrivée d'azote et de bouchons de type "septa". Du DMF sec (10 ml) est ajouté et la solution est refroidie à 0-5° C à l'aide d'un bain de glace. De l'hydrure de sodium (0,08 g, 2 mmol) est ensuite ajouté en une fois et le mélange est laissé à réchauffer jusqu'à température ambiante. Après une heure, 0,06 ml (1 mmol) d'iodure de méthyle est introduit goutte à goutte à l'aide d'une seringue dans le ballon. Après avoir mélangé pendant deux autres heures, le mélange est concentré et le résidu dissous dans 25 ml d'acétate d'éthyle. Cette solution est rincée successivement avec de l'eau (2 x 20 ml) et une solution saturée de NaCl (20 ml). La phase organique est séchée sur du sulfate de sodium et évaporée sous pression réduite de façon à obtenir une huile qui est purifiée par chromatographie flash (éluent : dichlorométhane / méthanol : 95/5). L'évaporation des phases collectées sous pression réduite donne 0,18 g (38 %) de la base du Composé 11. Celle-ci est transformée en son sel de chlorhydrate tel que décrit ci-dessus ; p.f. : 125-126° C.

RMN-¹H (400 MHz, DMSO d6, δ) : 10,82 (bs, 1H, ⁺NH) ; 8,57 (t, 1H, NH-CO) ; 7,36 (m, 10H, 2 x Ph) ; 7,07 (d, 1H, Ph-CH₃) ; 6,82 (m, 2H, Ph-CH₃) ; 3,38 (m, 2H, ⁺NH-CH₂) ; 3,21 (m, 2H, CH₂-NHCO) ; 3,17-2,80 (m, 8H, pipérazine) ; 2,98 (s, 3H, OCH₃) ; 2,26 (s, 3H, CH₃) ; 2,21 (s, 3H, CH₃) ; 1,90 (m, 2H, CH₂).

Analyse élémentaire pour C₃₀H₃₇N₃O₂, HCl , H₂O : % Calc : C, 68,49 ; H, 7,66 ; N, 7,99 ; % obtenus : C, 68,24 ; H, 7,52 ; N, 7,99.

### Exemple 12 : α-benzyloxy-N-[3-{4-(2,5-diméthylphényl)-1-pipérazinyl}-propyl]-α-phényl-benzèneacétamide (Composé 12) :

Le composé 12 est préparé d'une manière analogue à celle de l'exemple 11 sauf que du bromure de benzyle est utilisé à la place de l'iodure de méthyle dans l'étape d'O-alkylation ; p.f. : 171° C.

RMN-¹H (400 MHz, DMSO d6, δ) : 8,53 (m, 1H, NH-CO) ; 7,32 (m, 10H, 2 x Ph) ; 7,07 (d, 1H, Ph-CH₃, J = 7,5 Hz) ; 6,82 (d, 2H, Ph-CH₃) ; 3,54 (m, 2H, CH₂-NHCO) ; 3,28-3,10 (m, 8H, pipérazine) ; 2,27 (s, 3H, CH₃) ; 2,18 (s, 3H, CH₃) ; 2,10 (m, 2H, CH₂).

### Exemple 13 : N-[3-{4-(2-méthoxyphényl)-1-pipérazinyl}-propyl]-α-phényl-benzèneacétamide, dichlorhydrate (Composé 13) :

0,424 g (2 mmol) d'acide diphénylacétique, 0,498 g (2 mmol) de 3-[4-(2-méthoxyphényl)-1-pipérazinyl]-propyl-1-amine (Wu, Y.H. et al., J. Med. Chem., 12:876 (1969)) et 0,297 g (2,2 mmol) d'hydroxybenzotriazole sont dissous dans 15 ml de THF. La solution est refroidie jusqu'à 0-5° C avant addition de 0,453 g (2,2 mmol) de dicyclohexylcarbodiimide. On continue à agiter à température ambiante pendant 15 heures. Le mélange résultant est filtré, les matières insolubles sont rincées avec le minimum de THF et le filtrat est évaporé sous pression réduite. Le résidu huileux est dissous dans 50 ml d'acétate d'éthyle et rincé successivement avec H₂O (2 x 25 ml), NaOH 0,1 N (25 ml) et une solution saturée de chlorure de sodium. La phase organique est séchée sur du sulfate de sodium et évaporée jusqu'à siccité. Le résidu est soumis à une chromatographie flash sur gel de silice (éluent : dichlorométhane / méthanol : 95/5). L'évaporation des phases collectées sous pression réduite donne 0,505 g (55 %) du composé titre qui est mis sous forme de sel par l'utilisation d'une solution anhydre de chlorure d'hydrogène dans le diéthyléther ; p.f.: 198-199° C.

RMN-¹H (100 MHz, CDCl₃, δ [base libre]) : 7,30 (m, 11H, 2 x Ph + NH-CO) ; 6,90 (m, 4H, Ph-OCH₃) ; 4,88 (s, 1H, CH) ; 3,90 (s, 3H, OCH₃) ; 3,40 (m, 2H, CH₂-NH-CO) ; 3,15-2,50 (m, 8H, pipérazine) ; 2,50 (m, 2H, NH-CH₂) ; 1,70 ( m, 2H, CH₂).

Analyse élémentaire pour C₂₈H₃₃N₃O₂, 2 HCl : % Calc : C, 65,11 ; H, 6,83 ; N, 8,14 ; % obtenus : C, 64,82 ; H, 6,90 ; N, 8,01.

### Exemple 14 : α-hydroxy-N-[3-{4-(2-méthoxyphényl)-1-pipérazinyl}-propyl]-α-phényl-benzèneacétamide, dichlorhydrate, 0,25 H₂O (Composé 14) :

Le composé 14 est préparé d'une manière analogue à celle de l'exemple 13 sauf que l'acide benzylique est utilisé à la place de l'acide diphénylacétique ; p.f.: 191-192° C.

RMN-¹H (400 MHz, DMSO d6, δ) : 10,93 (bs, 1H, ⁺NH) ; 8,43 (t, 1H, NH-CO) ; 7,36 (m, 10H, 2 x Ph) ; 6,95 (m, 4H, Ph-OCH₃) ; 4,85 (bs, 1H, OH); 3,80 (s, 3H, OCH₃) ; 3,45 (m, 2H, ⁺NH-CH₂) ; 3,21 (m, 2H, CH₂-NHCO) ; 3,40-2,95 (m, 8H, pipérazine) ; 1,92 (m, 2H, CH₂).

Analyse élémentaire pour C₂₈H₃₃N₃O₃, 2 HCl, 0,25 H₂O : % Cale: C, 62,63 ; H, 6,66 ; N, 7,82 ; O, 9,68 ; % obtenus : C, 62,59 ; H, 6,88 ; N, 7,93 ; O, 9,59.

### Exemple 15 : N-[3-{4-(2-méthoxyphényl)-1-pipérazinyl}-propyl]-9H-xanthène-9-carboxamide, dichlorhydrate (Composé 15) :

Le composé 15 est préparé d'une manière analogue à celle de l'exemple 13 sauf que l'acide 9H-xanthène-9-carboxylique est utilisé à la place de l'acide diphénylacétique ; p.f. : 257-258° C.

RMN-¹H (100 MHz, CDCl₃, δ [base libre]) : 7,50-6,80 (m, 12H, xanthène + Ph-OCH₃) ; 6,27 (m, 1H, NH-CO) ; 4,92 (s, 1H, CH) ; 3,85 (s, 3H, OCH₃) ; 3,25 (m, 2H, CH₂-NH-CO) ; 3,00-2,30 (m, 8H, pipérazine) ; 2,25 (m, 2H, NH-CH₂) ; 1,60 (m, 2H, CH₂).

Analyse élémentaire pour C₂₈H₃₁N₃O₃ , 2 HCl : % Calc : C, 63,40 ; H, 6,27 ; N, 7,92 ; % obtenus : C, 63,22 ; H, 6,30 ; N, 7,74.

### Exemple 16 : 9-hydroxy-N-[3-{4-(2-méthoxyphényl)-1-pipérazinyl}-propyl]-9H-fluorène-9-carboxamide, dichlorhydrate, monohydrate (Composé 16) :

Le composé 16 est préparé d'une manière analogue à celle de l'exemple 13 stuf que l'acide 9-hydroxy-9-fluorène carboxylique est utilisé à la place de l'acide diphénylacétique ; p.f. : 186-187° C.

RMN-¹H (100 MHz, CDCl₃, δ [base libre]) : 7,70-7,25 (m, 8H, fluorène) ; 6,97 (m, 4H, Ph-OCH₃) ; 6,30 (m, 1H, NH-CO) ; 3,85 (s, 3H, OCH₃) ; 3,30 (m, 2H, CH₂-NH-CO) ; 2,90-2,30 (m, 8H, pipérazine) ; 2,20 (m, 2H, NH-CH₂) ; 1,65 (m, 3H, OH + CH₂).

Analyse élémentaire pour C₂₈H₃₁N₃O₃, 2 HCl, H₂O : % Cale: C, 61,31 ; H, 6;43 ; N, 7,66 ; % obtenus : C, 61,32 ; H, 6,25 ; N, 7,64.

### Exemple 17 : 2-chloro-α-(2-chlorophényl)-α-hydroxy-N-[3-{4-(2-méthoxyphényl)-1-pipérazinyl}-propyl]-α-phényl-benzèneacétamide, monochlorhydrate, monohydrate (Composé 17) :

Le composé 17 est préparé d'une manière analogue à celle de l'exemple 13 sauf que l'acide 2,2'-dichlorobenzylique est utilisé à la place de l'acide diphénylacétique ; p.f. : 185-186° C.

RMN-¹H (100 MHz, CDCl₃, δ [base libre]) : 7,75 (m, 1H, NH-CO) ; 7,30 (m, 8H, 2 x Ph-Cl) ; 6,90 (m, 4H, Ph-OCH₃) ; 3,88 (s, 3H, OCH₃) ; 3,52 (m, 2H, CH₂-NH-CO) ; 3,10 - 2,40 (m, 8H, pipérazine) ; 2,50 (m, 2H, NH-CH₂) ; 1,80 (m; 2H, CH₂).

Analyse élémentaire pour C₂₈H₃₁Cl₂N₃O₃, HCl , H₂O : % Calc : C, 57,69 ; H, 5,88 ; N, 7,21 ; % obtenus : C, 57,42 ; H, 5,84 ; N, 6,67.

### Exemple 18 : (±)-α-cyclohexyl-N-[3-{4-(2-méthoxyphényl)-1-pipérazinyl}-propyl]-benzèneacétamide, dichlorhydrate, monohydrate (Composé 18) :

Le composé 18 est préparé d'une manière analogue à celle de l'exemple 13 sauf que l'acide (±)-cyclohexylphényle acétique est utilisé à la place de l'acide diphénylacétique ; p.f. : 160-161° C.

RMN-¹H (400 MHz, DMSO d6, δ) : 10,94 (bs, 1H, ⁺NH) ; 8,32 (m, 1H, NH-CO) ; 7,28 (m, 5H, Ph) ; 6,95 (m, 4H, Ph-OCH₃) ; 3,80 (s, 3H, OCH₃) ; 3,42 (m, 2H, ⁺NH-CH₂) ; 3,42-2,90 (m, 8H, pipérazine) ; 3,05 (m, 2H, CH₂-NH-CO) ; 2,00-0,68 (m, 13H, cyclohexyle + CH₂).

Analyse élémentaire pour C₂₈H₃₉N₃O₂, 2 HCl, H₂O : % Calc : C, 62,21 ; H, 7,83 ; N, 7,77 ; % obtenus : C, 62,09 ; H, 7,89 ; N, 7,60.

### Exemple 19 : (±)-α-hydroxy-N-[3-{4-(2-méthoxyphényl)-1-pipérazinyl}-propyl]-benzèneacétamide, dichlorhydrate, monohydrate (Composé 19) :

Le composé 19 est préparé d'une manière analogue à celle de l'exemple 13 sauf que l'acide (±)-mandélique est utilisé à la place de l'acide diphénylacétique ; p.f. : 169° C.

RMN-¹H (400 MHz, DMSO d6, δ) : 10,94 (bs, 1H, ⁺NH) ; 8,27 (m, 1H, NH-CO) ; 7,35 (m, 5H, Ph) ; 6,95 (m, 4H, Ph-OCH₃) ; 5,70 (bs, OH) ; 4,95 (s, 1H, CH) ; 3,80 (s, 3H, OCH₃) ; 3,42 (m, 2H, ⁺NH-CH₂) ; 3,25-3,02 (m, 10H, pipérazine + CH₂-NH-CO) ; 1,90 (m, 2H, CH₂).

Analyse élémentaire pour C₂₂H₂₉N₃O₃, 2 HCl, H₂O : % Calc : C. 55,70 ; H, 7,01 ; N, 8.86 ; % obtenus : C, 55,23 ; H, 6,90 ; N, 8,84.

### Exemple 20 : N-[3-{4-(2,5-diméthylphényl)-1-pipérazinyl}-propyl]-α-phényl-benzèneacétamide, 1,5 HCl, H₂O (Composé 20) :

### 20.1) 3-[4-(2,5-diméthylphényl)-1-pipérazinyl]-propyl-1-amine :

Cet intermédiaire est synthétisé en utilisant la même procédure que celle décrite par Wu, Y.H. et al., J. Med. Chem., 12:876 (1969) ; p.f. : 102° C.

RMN-¹H (100 MHz, CDCl₃, δ) : 7,10 (d, 1H, Ph-CH₃, J = 7,5 Hz) ; 6,80 (m, 2H, Ph-CH₃) ; 3,10 (m, 12H, CH₂-N + CH₂-NH₂ + pipérazine) ; 2,30 (s, 3H, CH₃) ; 2,26 (s, 3H, CH₃) ; 1,70 (m, 2H, CH₂) ; 1,62 (s, NH₂).

### 20.2) N-[3-{4-(2,5-diméthylphényl)-1-pipérazinyl}-propyl]-α-phénylbenzèneacétamide, 1,5 HCl, H₂O :

Le composé 20 est préparé d'une manière analogue à celle de l'exemple 13 sauf que la 3-[4-(2,5-diméthylphényl)-1-pipérazinyl]-propyl-1-amine est utilisée à la place de la 3-[4-(2-méthoxyphényl)-1-pipérazinyl]-propyl-1-amine ; p.f. : 151° C.

RMN-¹H (400 MHz, DMSO d6, δ) : 10,96 (bs, 1H, ⁺NH) ; 8,62 (m, 1H, NH-CO) ; 7,27 (m, 10H, 2 x Ph) ; 7,06 (d, 1H, Ph-CH₃, J = 7,5 Hz) ; 6,82 (d, 1H, Ph-CH₃) ; 4,99 (s, 1H, CH) ; 3,44 (m, 2H, ⁺NH-CH₂) ; 3,18 (m, 2H, CH₂-NH-CO) ; 3,12 (m, 8H, pipérazine) ; 2,26 (s, 3H, CH₃) ; 2,19 (s, 3H, CH₃) ; 1,91 (m, 2H, CH₂).

Analyse élémentaire pour C₂₉H₃₅N₃O, 1,5 HCl, H₂O : % Calc : C, 67,72 ; H, 7,55 ; N, 8,17 ; O, 6,22 ; % obtenus : C, 67,56 ; H, 7,51 ; N, 8,17 ; O, 6,48.

### Exemple 21 : 4-chloro-α-(4-chlorophényl)-N-[3-{4-(2,5-diméthylphényl)-1-pipérazinyl}-propyl]-benzèneacétamide, fumarate (Composé 21) :

Le composé 21 est préparé d'une manière analogue à celle de l'exemple 20 sauf que l'acide 4,4'-dichloro-diphénylacétique est utilisé à la place de l'acide diphénylacétique ; p.f. : 139° C.

RMN-¹H (400 MHz, DMSO d6, δ) : 8,36 (m, 1H, NH-CO) ; 7,34 (m, 8H, 2 x Ph-Cl) ; 7,01 (d, 1H, Ph-CH₃, J = 8 Hz) ; 6,79 (s, 1H, Ph-CH₃) ; 6,75 (d, 1H, Ph-CH₃) ; 6,61 (s, 2H, CH=CH) ; 4,95 (s, 1H, CH) ; 3,13 (m, 2H, CH₂-NH-CO) ; 2,81 (bs, 4H, pipérazine) ; 2,52 (bs, 4H, pipérazine) ; 2,38 (m, 2H, CH₂-N) ; 2,23 (s, 3H, CH₃) ; 2,17 (s, 3H, CH₃) ; 1,62 (m, 2H, CH₂).

Analyse élémentaire pour C₂₉H₃₃Cl₂N₃O, fumarate : % Calc : C, 63,26 ; H, 5,95 ; N, 6,71 ; % obtenus : C, 63,60 ; H, 6,03 ; N, 6,78.

### Exemple 22 : (±)-N-[3-{4-(2,5-diméthylphényl)-1-pipérazinyl}-propyl]-α-hydroxy-α-méthyl-benzèneacétamide, 1,25 HCl, 0,75 H₂O (Composé 22) :

Le composé 22 est préparé d'une manière analogue à celle de l'exemple 20 sauf que l'acide (±)-atrolactique est utilisé à la place de l'acide diphénylacétique ; p.f. : 155° C.

RMN-¹H (400 MHz, DMSO d6, δ) : 10,82 (bs, 1H, ⁺NH) ; 8,16 (m, 1H, NH-CO) ; 7,56 (m, 2H, Ph) ; 7,34 (m, 2H, Ph) ; 7,24 (m, 1H, Ph) ; 7,06 (d, 1H, Ph-CH₃, J = 8 Hz) ; 6,82 (m, 2H, Ph,-CH₃) ; 4,87 (bs, OH); 3,39 (m, 2H, ⁺NH-CH₂) ; 3,17-2,97 (m, 10H, pipérazine + CH₂-NH-CO) ; 2,26 (s, 3H, Ph-CH₃) ; 2,20 (s, 3H, Ph-CH₃) ; 1,86 (m, 2H, CH₂) ; 1,64 (s, 3H, CH₃).

Analyse élémentaire pour C₂₄H₃₃N₃O₂, 1,25 HCl, 0,75 H₂O : % Calc : C, 63,41 ; H, 7,93 ; N, 9,24 ; O, 9,68 ; % obtenus : C, 63,56 ; H, 7,83 ; N, 9,15 ; O, 9,46.

### Exemple 23 : N-[3-{4-(2,5-diméthylphényl)-1-pipérazinyl}-propyl]-α-méthyl-α-phényl-benzèneacétamide, 1,5 HCl, 1,25 H₂O (Composé 23) :

Le composé 23 est préparé d'une manière analogue à celle de l'exemple 20 sauf que l'acide 2,2-diphénylpropionique est utilisé à la place de l'acide diphénylacétique ; p.f. : 90° C.

RMN-¹H (400 MHz, DMSO d6, δ) : 7,67 (m, 1H, NH-CO) ; 7,26 (m, 10H, 2 x Ph) ; 7,07 (d, 1H, Ph-CH₃, J = 8 Hz) ; 6,82 (m, 2H, Ph-CH₃) ; 6,56 (bs, 1H, ⁺NH) ; 3,39 (m, 2H, ⁺NH-CH₂) ; 3,20-2,97 (m, 10H, pipérazine + CH₂-NH-CO) ; 2,26 (s, 3H, Ph-CH₃) ; 2,21 (s, 3H, Ph-CH₃) ; 1,88 (m, 5H, CH2 + CH₃).

Analyse élémentaire pour C₃₀H₃₇N₃O, 1,5 HCl, 1,25 H₂O : % Calc : C, 67,62 ; H, 7,76 ; N, 7,89 ; O, 6,76 ; % obtenus : C, 67,82 ; H, 7,80 ; N, 7,51 ; O, 6,57.

### Exemple 24 : N-[3-{4-(2,5-diméthylphényl)-1-pipérazinyl}-propyl]-α-éthylthio-α-phényl-benzèneacétamide, 0,5 fumarate (Composé 24) :

Le composé 24 est préparé d'une manière analogue à celle de l'exemple 20 sauf que l'acide 2-éthylthio-2,2-diphénylacétique est utilisé à la place de l'acide diphénylacétique ; p.f. : 157° C.

RMN-¹H (400 MHz, DMSO d6, δ) : 7,94 (m, 1H, NH-CO) ; 7,32 (m, 10H, 2 x Ph) ; 7,01 (d, 1H, Ph- CH₃, J = 7,4 Hz) ; 6,75 (m, 2H, Ph-CH₃) ; 6,60 (s, 1H, 1/2 CH=CH) ; 3,60 (bs, COOH) ; 3,19 (m, 2H, CH₂-NH-CO) ; 2,77 (bs, 4H, pipérazine) ; 2,50 (m, 4H, pipérazine) ; 2,33 (m, 2H, CH₂-N) ; 2,24 (s, 3H, Ph-CH₃) ; 2,16 (m, 5H, Ph-CH₃ + S-CH₂) ; 1,62 (m, 2H, CH₂) ; 0,99 (t, 3H, CH₃-CH₂-S, J = 7,5 Hz).

Analyse élémentaire pour C₃₁H₃₉N₃OS, 0,5 fumarate : % Calc : C, 70,81 ; H, 7,38 ; N, 7,51 ; S, 5,73 ; % obtenus : C, 70,69 ; H, 7,33 ; N, 7,11 ; S, 5,96.

### Exemple 25 : N-[3-{4-(2,5-diméthylphényl)-1-pipérazinyl}-propyl]-α-hydroxy-4-méthoxy-α-(4-méthoxyphényl)-benzèneacétamide, HCl, 0,5 H₂O (Composé 25) :

Le composé 25 est préparé d'une manière analogue à celle de l'exemple 20 sauf que l'acide 4,4'-diméthoxybenzylique (Toda, F. et al. Chem. Letts., pp. 373-376 (1990)) est utilisé à la place de l'acide diphénylacétique ; p.f. : 173° C.

RMN-¹H (400 MHz, DMSO d6, δ): 11,19 (bs, 1H, ⁺NH) ; 8,37 (m, 1H, NH-CO) ; 7,70 (bs, OH) ; 7,29 (d, 4H, 2 x Ph-OCH₃, J = 8,6 Hz) ; 7,05 (d, 1H, Ph-CH₃, J = 7,7 Hz) ; 6,87 (d, 4H, 2 x Ph-OCH₃) ; 6,83 (m, 2H, Ph-CH₃) ; 3,73 (s, 6H, 2 x OCH₃) ; 3,42 (m, 2H, ⁺NH-CH₂) ; 3,30-2,95 (m, 10H, pipérazine + CH₂-NH-CO) ; 2,26 (s, 3H, Ph-CH₃) ; 2,20 (s, 3H, Ph-CH₃) ; 1,94 (m, 2H, CH₂).

### Exemple 26 : N-[3-{4-(2,5-diméthylphényl)-1-pipérazinyl}-propyl]-2-éthyl-2-phényl-butyramide, dichlorhydrate, monohydrate (Composé 26) :

Le composé 26 est préparé d'une manière analogue à celle de l'exemple 20 sauf que l'acide 2-éthyl-2-phénylbutyrique est utilisé à la place de l'acide diphénylacétique ; p.f.: 195° C.

RMN-¹H (400 MHz, DMSO d6, δ) : 11,12 (bs, 1H, ⁺NH) ; 7,65 (m, 1H, NH-CO); 7,28 (m, 10H, 2 x Ph) ; 7,06 (d, 1H, Ph-CH₃, J = 8 Hz) ; 6,82 (m, 2H, Ph-CH₃) ; 3,40 (m, 2H, ⁺NH-CH₂) ; 3,05 (m, 8H, pipérazine); 2,92 (m, 2H, CH₂-NH-CO) ; 2,26 (s, 3H, Ph-CH₃) ; 2,20 (s, 3H, Ph-CH₃) ; 1,91 (m, 6H, 3 x CH₂) ; 0,63 (t, 6H, 2 x CH₃-CH2, J = 7,2 Hz).

Analyse élémentaire pour C₂₇H₃₉N₃O, 2 HCl, H₂O : % Calc : C, 63,27 ; H, 8,46 ; N, 8,20 ; O, 6,24 ; % obtenus : C, 63,67 ; H, 8,57 ; N, 8,19 ; O, 6,43.

### Exemple 27 : (±)-N-[3-{4-(2,5-diméthylphényl)-1-pipérazinyl}-propyl]-2-hydroxy-2-phényl-butyramide, fumarate (Composé 27) :

Le composé 27 est préparé d'une manière analogue à celle de l'exemple 20 sauf que l'acide α-hydroxy-α-phényl-butyrique est utilisé à la place de l'acide diphénylacétique ; p.f. : 179° C.

RMN-¹H (400 MHz, DMSO d6, δ) : 8,03 (m, 1H, NH-CO) ; 7,55 (d, 2H, Ph, J = 7,5 Hz) ; 7,30 (m, 2H, Ph) ; 7,22 (m, 1H, Ph) ; 7,02 (d, 1H, Ph-CH₃, J = 7,5 Hz) ; 6,81 (s, 1H, Ph-CH₃) ; 6,76 (d, 1H, Ph-CH₃) ; 6,60 (s, 2H, CH=CH) ; 3,10 (m, 2H, CH₂-NHCO) ; 2,86 (bs, 4H, pipérazine) ; 2,58 (bs, 4H, pipérazine) ; 2,41 (m, 2H, CH₂-N) ; 2,24 (s, 3H, CH₃); 2,21 (m, 1H, CH₂-CH₃) ; 2,17 (s, 3H, CH₃) ; 1,86 (m, 1H, CH₂-CH₃) ; 1,60 (m, 2H, CH₂); 0,79 (t, 3H, CH₂-CH₃, J = 7,2 Hz).

Analyse élémentaire pour C₂₅H₃₅N₃O₂, fumarate : % Calc : C, 66,27 ; H, 7,48 ; N, 7,99 ; % obtenus : C, 66,28 ; H, 7,35 ; N, 7,98.

### Exemple 28 : (±)-N-[3-{4-(2,5-diméthylphényl)-1-pipérazinyl}-propyl]-2-hydroxy-2-phényl-hexanamide, fumarate (Composé 28) :

### 28.1) Acide (±)-2-hydroxy-2-phényl-hexanoïque :

Cet intermédiaire est décrit par Newcomb M., et al. (J. Org. Chem., 43:3963 (1978)) ; p.f. : 102° C.

RMN-¹H (100 MHz, CDCl₃, δ) : 7,52 (m, 5H, Ph) ; 5,75 (bs, OH + COOH); 2,10 (m, 2H, CH₂₋C-OH) ; 1,33 (m, 4H, CH₂-CH₂) ; 0,90 (m, 3H, CH₃).

### 28.2) (±)-N-[3-{4-(2,5-diméthylphényl)-1-pipérazinyl}-propyl]-2-hydroxy-2-phényl-hexanamide, fumarate :

Le composé 28 est préparé d'une manière analogue à celle de l'exemple 20 sauf que l'acide (±)-2-hydroxy-2-phényl-hexanoïque est utilisé à la place de l'acide diphénylacétique ; p.f. : 147° C.

RMN-¹H (400 MHz, DMSO d6, δ) : 8,01 (m, 1H, NH-CO); 7,55 (d, 2H, Ph, J = 7,5 Hz) ; 7,25 (m, 3H, Ph) ; 7,02 (d, 1H, Ph-CH₃, J = 7,5 Hz) ; 6,81 (s, 1H, Ph-CH₃) ; 6,76 (d, 1H, Ph-CH₃) ; 6,60 (s, 2H, CH=CH); 5,85 (bs, OH + COOH) ; 3,10 (m, 2H, CH₂-NHCO) ; 2,85 (bs, 4H, pipérazine) ; 2,57 (bs, 4H, pipérazine) ; 2,39 (m, 2H, CH₂-N) ; 2,24 (s, 3H, Ph-CH₃) ; 2,17 (s, 3H, Ph-CH₃) ; 2,14 (m, 1H, 1/2 CH₂-C-OH) ; 1,84 (m, 1H, 1/2 CH₂-C-OH) ; 1,59 (m, 2H, CH₂) ; 1,25 (m, 4H, CH₂-CH₂-CH₃) ; 1,18 (t, 3H, CH₂-CH₃, J = 6,9 Hz).

Analyse élémentaire pour C₂₇H₃₉N₃O₂, fumarate : % Calc : C, 67,25 ; H, 7,83 ; N, 7,59 ; % obtenus : C, 66,76 ; H, 7,69 ; N, 7,58.

### Exemple 29 : (±)-2-chloro-N-[3-{4-(2,5-diméthylphényl)-1-pipérazinyl}-propyl]-α-hydroxy-α-méthyl-benzèneacétamide, fumarate, monohydrate (Composé 29) :

### 29.1) (±)-2-chloro-α-hydroxy-α-méthyl-benzèneacétonitrile :

Ce composé est synthétisé en utilisant la même procédure que celle décrite par Gassman, P.G. et al., Tetrahedron Lett., 40:3773 (1978).

RMN-¹H (100 MHz, CDCl₃, δ) : 7,50 (m, 4H, Ph) ; 3,78 (bs, 1H, OH) ; 2,06 (s, 3H, CH₃).

### 29.2) Acide (±)-2-chloro-α-hydroxy-α-méthyl-benzèneacétique :

Un mélange de 2,3 g (12,7 mmol) de (±)-2-chloro-α-hydroxy-α-méthyl-benzèneacétronitrile et 20 ml de HCl concentré est chauffé à 60°C pendant 3 heures puis est mis au reflux pendant 2 heures. Après refroidissement, le mélange réactionnel est concentré sous pression réduite et le résidu est séparé entre diéthyléther (50 ml) et NaOH 1N (50 ml). La phase organique est extraite deux fois avec 25 ml de NaOH 1N, et toutes les phases basiques sont réunies ensemble avant acidification avec HCl concentré. Le produit est extrait avec 2 x 50 ml de diéthyléther, puis la solution est séchée sur sulphate de sodium et évaporée sous vide pour donner 930 mg (36 %) d'une poudre blanche ; p.f. : 110-111° C.

RMN-¹H (100 MHz, CDCl₃, δ) : 7,50 (m, 4H, Ph) ; 6,47 (bs, 2H, OH +COOH) ; 1,90 (s, 3H, CH₃).

### 29.3) (±)-2-chloro-N-[3-{4-(2,5-diméthylphényl)-1-pipérazinyl}-propyl]-α-hydroxy-α-méthyl-benzèneacétamide, fumarate, monohydrate :

Le composé 29 est préparé d'une manière analogue à celle de l'exemple 20 sauf que l'acide (±)-2-chloro-α-hydroxy-α-méthyl-benzèneacétique est utilisé à la place de l'acide diphénylacétique ; p.f. : 84° C.

RMN-¹H (400 MHz, DMSO d6, δ) : 7,90 (m, 1H, NH-CO) ; 7,62 (m, 1H, Ph-Cl) ; 7,34 (m, 3H, Ph-Cl) ; 7,03 (d, 1H, Ph-CH₃, J = 7,5 Hz) ; 6,78 (m, 2H, Ph-CH₃) ; 6,61 (s, 2H, CH=CH) ; 6,02 (bs, OH + COOH); 3,19 (m, 2H, CH₂-NHCO) ; 2,90 (bs, 4H, pipérazine) ; 2,76 (bs, 4H, pipérazine) ; 2,62 (m, 2H, CH₂-N) ; 2,24 (s, 3H, CH₃) ; 2,18 (s, 3H, CH₃) ; 1,72 (m, 5H, CH₂ + CH₃).

Analyse élémentaire pour C₂₄H₃₂ClN₃O₂, fumarate, H₂O : % Calc : C, 59,62 ; H, 6,79 ; N, 7,45 ; % obtenus : C, 59,54 ; H, 6,47 ; N, 7,10.

### Exemple 30 : (±)-4-chloro-N-[3-{4-(2,5-diméthylphényl)-1-pipérazinyl}-propyl]-α-hydroxy-α-méthyl-benzèneacétamide, 1,25 HCl, 0,5 H₂O (Composé 30) :

### 30.1) Acide (±)-4-chloro-α-hydroxy-α-méthyl-benzèneacétique :

Ce composé est préparé d'une manière analogue à celle de l'intermédiaire 29.2 sauf que le (±)-4-chloro-α-hydroxy-α-méthyl-benxèneacétonitrile (Gassrnan, P.G. et al., Tetrahedron Lett., 40:3773 (1978)) est utilisé à la place du (±)-2-chloro-α-hydroxy-α-méthyl benzèneacétonitrile.

RMN-¹H (100 MHz, CDCl₃, δ) : 7,45 (m, 4H, Ph) ; 6,80 (bs, 2H, OH + COOH) ; 1,80 (s,. 3H, CH₃).

### 30.2) (±)-4-chloro-N-[3-{4-(2,5-diméthylphényl)-1-pipérazinyl}-propyl]-α-hydroxy-α-méthyl-benzèneacétamide, 1,25 HCI, 0,5 H₂O :

Le composé 30 est préparé d'une manière analogue à celle de l'exemple 20 sauf que l'acide (±)-4-chloro-α-hydroxy-α-méthyl-benzèneacétique est utilisé à la place de l'acide diphénylacétique ; p.f. : 161,5° C.

RMN-¹H (400 MHz, DMSO d6, δ) : 10,95 (bs, 1H, ⁺NH) ; 8,20 (m, 1H, NH-CO) ; 7,56 (m, 2H, Ph-Cl) ; 7,39 (m, 2H, Ph-Cl) ; 7,06 (d, 1H, Ph-CH₃, J = 8 Hz); 6,82 (m, 2H, Ph-CH₃) ; 5,70 (bs, OH) ; 3,44 (m, 2H, ⁺NH-CH₂) ; 3,16- 2,99 (m, 10H, pipérazine + CH₂-NH-CO) ; 2,26 (s, 3H, Ph-CH₃); 2,20 (s, 3H, Ph-CH₃) ; 1,87 (m, 2H, CH₂) ; 1,62 (s, 3H, CH₃).

Analyse élémentaire pour C₂₄H₃₂ClN₃O₂, 1,25 HCl, 0,5 H₂O : % Calc : C, 59,49 ; H, 7,12 ; N, 8,67 ; % obtenus : C, 59,46 ; H, 7,01 ; N, 8,51.

### Exemple 31 : (±)-3-chloro-N-[3{4-(2,5-diméthylphényl)-1-pipérazinyl}-propyl]-α-hydroxy-α-méthyl-benzèneacétamide, fumarate (Composé 31) :

### 31.1) Ethyl-ester de l'acide (±)-3-chloro-α-hydroxy-α-méthyl-benzèneacétique :

Dans un ballon tricot anhydre équipé d'une arrivée d'azote, d'un entonnoir, et d'un condensateur à reflux sont introduits 400 mg (0,016 a.g.) de poudre de magnésium, quelques gouttes d'une solution de 3-bramochlorobenzène (1,94 ml, 16,5 mmol) dans 10 ml de diéthyléther sec, et un crystal d'iode. Le mélange est doucement chauffé jusqu'à ce que la réaction commence, puis le reliquat de 3-bromochlorobenzène est ajouté goutte à goutte à un débit qui maintient le reflux. L'agitation est poursuivie jusqu'à disparition complète du magnésium (environ 1 heure). Le bromure de 3-chlorophénylmagnésium ainsi formé est ensuite amené via une tubulure dans une solution préalablement refroidie (-78° C) de 1,64 ml (15 mmol) de pyruvate d'éthyle dans 15 ml de diéthyléther sec. Après que cette addition soit complète, on laisse la température de réaction lentement augmenter jusqu'à 20° C et le mélange est agité toute la nuit. Le mélange réactionnel est refroidi brutalement à 0° C par l'addition de 3N HCl (10 ml) et extrait avec 2 x 20 ml de diéthyléther. L'extrait organique est ensuite rincé avec de l'eau (2 x 20 ml), une solution saturée de chlorure de sodium (20 ml), et séché sur du sulphate de sodium. L'évaporation du solvant sous pression réduite permet l'obtention d'une huile jaune qui est purifiée sur colonne de chromatographie (éluent : pétrole léger (bp. 40-65° C) / acétate d'éthyle : 95/5). 2,16 g (57 %) d'une huile pure incolore sont obtenus.

RMN-¹H (100 MHz, CDCl₃, δ) : 7,60 - 7,25 (m, 4H, Ph); 4,23 (q, 2H, CH₂, J = 7,0 Hz) ; 3,89 (s, 1H, OH) ; 1,75 (s, 3H, CH₃) ; 1,29 (t, 3H, CH₂-CH₃).

### 31.2) Acide (±)-3-chloro-α-hydroxy-α-méthyl-benzèneacétique :

A une solution de 2,16 g (9,44 mmol) d'éthyl-ester d'acide (±)-3-chloro-α-hydroxy-α-méthyl-benzèneacétique dans 20 ml d'éthanol refroidi (0-5° C) est ajoutée goutte à goutte une solution de 1,24 g (18,8 mmol) de KOH dans 10 ml de H₂O. L'agitation est poursuivie pendant 3 heures à 25° C, et le solvant est évaporé. Le résidu est séparé entre H₂O et diéthyléther. Après décantation, la couche aqueuse est acidifiée à 0° C avec 3N HCI et extraite avec 2 x 50 ml de diéthyléther. L'extrait diéthyléther est séché sur sodium de sulphate et le solvant est évaporé sous pression réduite de façon à obtenir 1,76 g (93 %) d'une poudre blanche p.f. : 110-111° C.

RMN-¹H (100 MHz, CD₃OD, δ) : 7,80-7,42 (m, 4H, Ph) ; 1,89 (s, 3H, CH₃).

### 31.3) (±)-3-chloro-N-[3-{4-(2,5-diméthylphényl)-1-pipérazinyl}-propyl]-α-hydroxy-α-méthyl-benzèneacétamide, fumarate :

Le composé 31 est préparé d'une manière analogue à celle de l'exemple 20 sauf que l'acide (±)-3-chloro-α-hydroxy-α-méthyl-benzèneacétique est utilisé à la place de l'acide diphénylacétique : p.f. : 80-81° C.

RMN-¹H (400 MHz, DMSO d6, δ) : 8,17 (m, 1H, NH-CO) ; 7,57 (s, 1H, Ph-Cl) ; 7,49 (d, 1H, Ph-Cl, J = 7,63 Hz) ; 7,34 (m, 2H, Ph-Cl) ; 6,61 (s, 2H, -CH=CH-) ; 3,10 (m, 2H, CH₂-NHCO) ; 2,86 (bs, 4H, pipérazine) ; 2,58 (bs, 4H, pipérazine) ; 2,40 (m, 2H, CH₂-N) ; 2,24 (s, 3H, Ph-CH₃) ; 2,18 (s, 3H, Ph-CH₃) ; 1,59 (m, 5H, CH₂ + CH₃).

Analyse élémentaire pour C₂₄H₃₂ClN₃O₂, fumarate : % Cale : C, 61,59 ; H, 6,65 ; N, 7,70 ; % obtenus : C, 61,18 ; H, 6,60 ; N, 7,60.

### Exemple 32 : (±)-α-amino-N-[3-{4-(2,5-diméthylphényl)-1-pipérazinyl}-propyl]-benzèneacétamide, difumarate (Composé 32) :

### 32.1) {±)-α-tert-butoxycarbonylamino-N-[3-{4-(2,5-diméthylphényl)-1-pipérazinyl}-propyl]-benzèneacétamide :

Ce composé est préparé d'une manière analogue à celle de l'exemple 20 sauf que la (±)-Boc-phénylglycine est utilisée à la place de l'acide diphénylacétique.

RMN-¹H (100 MHz, CDCl₃, δ) : 7,35 (m, 6H, Ph + NH-CO) ; 7,01 (d, 1H, Ph-CH₃, J = 7,5 Hz) ; 6,85 (s, 1H, Ph-CH₃) ; 6,79 (d, 1H, Ph-CH₃) ; 5,90 (m, 1H, NH-Boc) ; 5,10 (d, 1H, CH) ; 3,38 (m, 2H, CH₂-NHCO) ; 3,00-2,30 (m, 10H, pipérazine + CH₂-N) ; 2,35 (s, 3H, CH₃) ; 2,25 (s, 3H, CH₃) ; 1,65 (m, 2H, CH₂) ; 1,40 (s, 9H, tBu).

### 32.2) {±)-α-amino-N-[3-{4-(2,5-diméthylphényl)-1-pipérazinyl}-propyl]-benzèneacétamide, difumarate :

Une solution de 1,47 g (3 mmol) de (±)-α-tert-butoxycarbonylamino-N-[3-{4-(2,5-diméthylphényl)-1-pipérazinyl}-propyl]-benzèneacétamide dans 20 ml de 1,4-dioxane est refroidie à 0° C avant addition de 7,5 ml (30 mmol) d'une solution de 4N HCl dans 1,4-dioxane. Après agitation toute la nuit à la température ambiante, le mélange réactionnel est concentré sous vide. Le résidu est mis en suspension dans 50 ml d'acétate d'éthyle et rincé avec 2 x 50 ml de NaOH 1N. Après séchage sur sulphate de sodium anhydre, la phase organique est concentrée pour donner 1,10 g (95 %) d'une huile incolore. Celle-ci est convertie en son sel de fumarate tel que décrit ci-dessus ; p.f. : 122-123° C.

RMN-¹H (400 MHz, DMSO d6, δ) : 9,90 (bs, NH₃⁺) ; 8,52 (m, 1H, NH-CO) ; 7,47 (m, 5H, Ph) ; 7,03 (d, 1H, Ph-CH₃, J = 7,5 Hz) ; 6,79 (s, 1H, Ph-CH₃) ; 6,75 (d, 1H, Ph-CH₃) ; 6,55 (s, 4H, 2 x -CH=CH-) ; 4,82 (s, 1H, CH) ; 3,12 (m, 2H, CH₂-NHCO) ; 2,80 (bs, 4H, pipérazine) ; 2,50 (bs, 4H, pipérazine) ; 2,32 (m, 2H, CH₂-N) ; 2,23 (s, 3H, CH₃) ; 2,17 (s, 3H, CH₃); 1,59 (m, 2H, CH₂).

Analyse élémentaire pour C₂₃H₃₂N₄O, difumarate : % Calc : C, 60,77 ; H, 6,58 ; N, 9,14 ; % obtenus : C, 60.61 ; H, 6,51 ; N, 8,87.

### Exemple 33 : (±)-N-[3-{4-(2,5-diméthylphényl)-1-pipérazinyl}-propyl]-α-(méthylsulfonylamino)-benzèneacétamide, HCl, 0,5 H₂O (Composé 33) :

### 33.1) Acide (±)-α-(méthylsulfonylamino)-benzèneacétique :

A une suspension agitée de 3,78 g (25 mmol) de (±)-phénylglycine dans 10 ml H₂O est ajoutée goutte à goutte une solution de NaOH 1N (25 ml) jusqu'à dissolution complète. Sous agitation forte, 2,7 ml (35 mmol) de chlorure de méthanesulfonyle sont ajoutés suivis par NaOH 1N en petite quantité pour maintenir l'alcalinité du mélange à environ pH 9. Après stabilisation du pH, l'agitation est poursuivie à température ambiante pendant une heure. Le mélange réactionnel est ensuite acidifié avec HCl 4N et extrait avec 2 x 50 ml d'acétate d'éthyle. La phase organique est rincée avec 50 ml HCl 1N suivi par 2 x 25 ml d'une solutation saturée de chlorure de sodium et finalement séchée sur sulfate de sodium. La filtration et l'évaporation du solvant permettent l'obtention d'une huile jaune qui cristallise lentement lorsqu'elle est laissée toute la nuit à température ambiante. Les cristaux jaunes sont filtrés et rincés avec un mélange de pétrole léger (bp, 40-65° C) et de diéthyléther pour donner 1,87 g (32 %) d'une poudre blanche ; p.f. =117° C.

RMN-¹H (100 MHz, CDCl₃, δ) : 9,79 (bs, 1H, COOH) ; 7,40 (bs, 5H, Ph) ; 5,93 (d, 1H, SO₂NH, J = 7,0 Hz) ; 5,25 (d, 1H, CH) ; 2,74 (s, 3H, CH₃).

### 33.2) (±)-N-[3-{4-(2,5-diméthylphényl)-1-pipérazinyl}-propyl]-α-(méthylsulfonylamino)-benzèneacétamide, HCl, 0,5 H₂O :

Le composé 33 est préparé d'une manière analogue à celle de l'exemple 20 sauf que l'acide (±)-α-(méthylsulfonylamino)-benzèneacétique est utilisé à la place de l'acide diphénylacétique ; p.f. : 151-152° C.

RMN-¹H (400 MHz, DMSO d6, δ) : 11,05 (bs, 1H, ⁺NH) ; 8,64 (m, 1H, NH-CO) ; 7,98 (m, 1H, NHSO₂) ; 7,50 (d, 2H, Ph, J = 7,55 Hz) ; 7,36 (m, 3H, Ph) ; 7,06 (d, 1H, Ph-CH₃, J = 7,5 Hz) ; 6,82 (m, 2H, Ph-CH₃) ; 5,03 (d, 1H,CH, J = 6,8 Hz) ; 3,48 (m, 2H, ⁺NH-CH₂) ; 3,09 (m, 10H, pipérazine + CH₂-NH-CO) ; 2,76 (s, 3H, CH₃-SO₂) ; 2,26 (s, 3H, Ph-CH₃) ; 2,20 (s, 3H, Ph-CH₃) ; 1,90 (m, 2H, CH₂) ; 1,62 (s, 3H, CH₃).

Analyse élémentaire pour C₂₄H₃₄ClN₄O₃S, HCl, 0,5 H₂O : % Calc : C, 57,19 ; H, 7,20 ; N, 11,11 ; S, 6,36 ; % obtenus : C, 57,27 ; H, 7,09 ; N, 11,04 ; S, 6,09.

### Exemple 34 : (±)-N-[3-{4-(2,5-diméthylphényl)-1-pipérazinyl}-propyl]-2-hydroxy-2-méthyl-4-méthoxy-benzènepropionamide, fumarate (Composé 34) :

### 34.1) (±)-2-hydroxy-2-méthyl-4-méthoxy-benzènepropionitrile :

Ce composé est synthétisé en utilisant la même procédure que celle décrite par Gassman, P.G. et al., Tetrahedron Lett., 40:3773 (1978).

RMN-¹H (100 MHz, CDCl₃, δ) : 7,10 (m, 4H, Ph) ; 3,81 (s, 3H, OCH₃) ; 2,98 (AB, 2H, CH₂, J_{AB} = 14 Hz) ; 2,70 (bs, 1H, OH) ; 1,64 (s, 3H, CH₃).

### 34.2) Acide (±)-2-hydroxy-2-méthyl-4-méthoxy-benzènepropionique

Ce composé est préparé d'une manière analogue à celle de l'intermédiaire 29.2 sauf que le (±)-2-hydroxy-2-méthyl-4-méthoxy-benzènepropionitrile est utilisé à la place du (±)-2-chloro-α-hydroxy-α-méthyl-benzèneacétronitrile.

RMN-¹H (100 MHz, CDCl₃, δ) : 7,00 (m, 4H, Ph) ; 5,66 (bs, 2H, OH + COOH) ; 3,79 (s, 3H, OCH₃) ; 3,00 (AB, 2H, CH₂, J_{AB} = 13,7 Hz) ; 1,51 (s, 3H, CH₃).

### 34.3) (±)-N-[3-{4-(2,5-diméthylphényl)-1-pipérazinyl}-propyl]-2-hydroxy-2-méthyl-4-méthoxy-benzènepropionamide, fumarate :

Le composé 34 est préparé d'une manière analogue à celle de l'exemple 20 sauf que l'acide (±)-2-hydroxy-2-méthyl-4-méthoxy-benzènepropionique est utilisé à la place de l'acide diphénylacétique ; p.f. : 142° C.

RMN-¹H (400 MHz, DMSO d6, δ) : 7,66 (m, 1H, NH-CO); 7,08-6,73 (m, 7H, Ph-CH₃ + Ph-OCH₃) ; 6,61 (s, 2H, CH=CH) ; 5,30 (bs, OH + COOH); 3,69 (s, 3H, OCH₃) ; 3,16 (m, 1H, 1/2 CH₂-NHCO) ; 2,95(m, 1H, 1/2 CH₂-NHCO) ; 2,82 (bs, 4H, pipérazine) ; 2,78 (AB, 2H, CH₂-PhOCH₃, J_{AB} = 13,35 Hz) ; 2,50 (bs, 4H, pipérazine) ; 2,32 (m, 2H, CH₂-N) ; 2,25 (s, 3H, CH₃) ; 2,17 (s, 3H, CH₃) ; 1,47 (m, 2H, CH₂) ; 1,26 (s, 3H, CH₃).

Analyse élémentaire pour C₂₆H₃₇N₃O₃, fumarate : % Calc : C, 64,85 ; H, 7,44 ; N, 7,56 ; % obtenus : C, 64,59 ; H, 7,42 ; N, 7,49.

### Exemple 35 : N-[3-{4-(2,5-diméthyiphényl)-lpipérazinyl)-propyl]-N-méthyl-α-phényl-benzèneacétamide, 0,75 fumarate (Composé 35) :

### 35.1) N-(3-hydroxypropyl)-N-méthyl-α-phényl-benzèneacétamide :

Le composé titre est préparé d'une manière analogue à celle de l'intermédiaire 1.1 sauf que la N-méthyl-3-hydroxypropylamine (Koepke, S.R. et al., J. Org. Chem. 44:2718 (1979)) est utilisée à la place du 3-amino-1-propanol.

RMN-¹H (400 MHz, CDCl₃, δ) : 7,29 (m, 10H, 2 x Ph) ; 5,24 (s, 1H, CH) ; 3,58 (m, 2H, CH₂-NCO) ; 3,48 (m, 2H, CH₂OH) ; 3,00 (s, 3H, CH₃) ; 1,71 (m, 2H, CH₂).

### 35.2) N-(3-iodopropyl)-N-méthyl-α-phényl-benzèneacétamide :

Le composé titre est préparé d'une manière analogue à celle de l'intermédiaire 1.2 sauf que le N-(3-hydroxypropyl)-N-méthyl-α-phényl-benzèneacétamide est utilisé à la place du α-hydroxy-N-(3-hydroxypropyl)-α-phényl-benzèneacétamide.

RMN-¹H (400 MHz, CDCl₃, δ) : 7,32 (m, 10H, 2 x Ph) ; 5,18 (s, 1H, CH) ; 3,50 (m, 2H, CH₂-NCO); 3,12 (m, 2H, CH₂-I) ; 3,00 (s, 3H, CH₃) ; 2,10 (m, 2H, CH₂).

### 35.3) N-[3-{4-(2,5-diméthylphényl)-1-pipérazinyl)-propyl]-N-méthyl-α-phényl-benzèneacétamide, 0,75 fumarate :

Un mélange de 2 g (5 mmol) de N-(3-iodopropyl)-N-méthyl-α-phényl-benzèneacétamide et 970 mg (5 mmol) de 1-(2,5-diméthylphényl)-pipérazine dans 15 ml d'acétonitrile est chauffé sous reflux pendant 4 heures. Après refroidissement, le solvant est évaporé sous pression réduite, et le résidu est traité tel que décrit dans l'exemple 1 excepté pour la colonne de chromatographie (éluent : acétate d'éthyle / éthanol : 90/10). La mise sous forme de sel est effectuée en présence d'un équivalent de l'acide fumarique dans de l'éthanol au reflux pour donner le composé titre sous forme de solide blanc ; p.f. : 192° C.

RMN-¹H (400 MHz, DMSO d6, δ) : 7,25 (m, 10H, 2 x Ph) ; 7,02 (d, 1H, Ph-CH₃, J = 7,5 Hz) ; 6,77 (m, 2H, Ph-CH₃) ; 6,61 (s, 1,5H, 0,75 CH=CH) ; 5,52 (s, 0,5H, CH) ; 5,45 (s, 0,5H, CH) ; 3,37 (m, 2H, CH₂-N(CH₃)-CO) ; 2,98 (s, 1,5H, NCH₃) ; 2,86 (s, 1,5H, NCH₃) ; 2,82 (bs, 4H, pipérazine) ; 2,60 (bs, 4H, pipérazine) ; 2,40 (m, 1H, CH₂-N) ; 2,34 (m, 1H, CH₂-N) ; 2,23 (s, 3H, Ph-CH₃) ; 2,17 (s, 3H, Ph-CH₃) ; 1,67 (m, 2H, CH₂).

Analyse élémentaire pour C₃₀H₃₇N₃O, 0,75 fumarate : % Calc : C, 73,04 ; H, 7,43 ; N, 7,74 ; % obtenus : C, 72,54 ; H, 7,30 ; N, 7,29.

### Exemple 36 : (±)-N-[3-{4-(2-(1-hydroxypropyl)-phényl)-1-pipérazinyl}-propyl]-α-phényl-benzèneacétamide, fumarate (Composé 36) :

### 36.1) 2-(4-benzyl-1-pipérazinyl)-benzaldéhyde :

4,24 ml (40 mmol) de 2-fluorobenzaldéhyde, 8,34 ml (48 mmol) de N-benzylpipérazine et 6,62 g (48 mmol) de K₂CO₃ dans 60 ml de DMF sont chauffés à 150° C pendant 7 heures. Après refroidissement, le mélange est couvert avec 200 ml d'acétate d'éthyle, et la phase organique est rincée successivement avec 2 x 50 ml de H₂O et 2 x 50 ml d'une solution saturée de NaCl. Après séchage sur Na₂SO₄, l'acétate d'éthyle est évaporé pour obtenir une huile marron qui est purifiée par chromatographie sur gel de silice (éluent : pétrole léger (bp. 40-65° C) / acétate d'éthyle : 9/1 puis 8/2). L'évaporation des fractions appropriées sous pression réduite donne 10,91 g (91 %) d'un solide blanc ; p.f. : 60° C.

RMN-¹H (100 MHz, CDCl₃, δ) : 10,32 (s, 1H, CHO) ; 7,78 (m, 1H, Ph-CHO) ; 7,47 (m, 1H, Ph-CHO) ; 7,32 (s, 5H, Ph) ; 7,10 (m, 2H, Ph-CHO) ; 3,60 (s, 2H, CH₂-Ph) ; 3,12 (m, 4H, pipérazine) ; 2,68 (m, 4H, pipérazine).

### 36.2) (±)-2-(4-benzyl-1-pipérazinyl)-α-éthyl-benzèneméthanol :

Dans un ballon tricol anhydre équipé d'une avivée d'azote est introduite une solution de 1,8 ml ( 5,3 mmol, solution 3M dans le diéthyléther) de bromure d'éthylmagnésium dans 10 ml de diéthyléther sec. A cette solution préalablement refroidie (0 à 5° C) est ajouté goutte à goutte 1 g (3,5 mmol) de 2-(4-benzyl-1-pipérazinyl)-benzaldéhyde dilué dans 5 ml de diéthyléther sec, et l'agitation est poursuivie 30 minutes à température ambiante. Le mélange réactionnel est ensuite hydrolysé à 0° C par une solution concentrée de chlorure d'ammonium et finalement dilué avec 50 ml d'acétate d'éthyle. Après décantation, la phase organique est rincée successivement avec 2 x 20 ml d'eau et 20 ml d'une solution concentrée de NaCl. Après séchage sur Na₂SO₄, l'acétate d'éthyle est évaporé sous vide pour donner 880 mg (81 %) d'une huile incolore qui cristallise lentement à température ambiante ; p.f. : 97° C.

RMN-¹H (100 MHz, CDCl₃, δ) : 7,25 (m, 9H, 2 x Ph) ; 6,55 (bs, OH) ; 4,70 (t, 1H, CH, J = 7 Hz) ; 3,55 (s, 2H, CH₂-Ph) ; 2,98 (m, 4H, pipérazine) ; 2,60 (m, 4H, pipérazine) ; 1,78 (m, 2H, CH₂-CH₃) ; 1,00 (t, 3H, CH₂-CH₃, J = 7 Hz).

### 36.3) (±)-2-(1-pipérazinyl)-α-éthyl-benzèneméthanol :

456 mg (1,5 mmol) de 2-(4-benzyl-1-pipérazinyl)-α-éthyl-benzèneméthanol sont dissous dans 30 ml d'éthanol et hydrogénés sur Pd/C 10 % à 40 PSI 40° C dans un appareil Parr. Après 4 heures, le mélange réactionnel est filtré au travers de Celite, et le filtrat est concentré sous pression réduite pour donner 330 mg (100 %) de composé titre sous forme de solide blanc ; p.f. : 95° C.

RMN-¹H (100 MHz, CDCl₃, δ) : 7,21 (m, 4H, Ph) ; 4,72 (t, 1H, CH, J = 7 Hz) ; 3,00 (m, 8H, pipérazine) ; 2,30 (bs, OH) ; 1,80 (m, 2H, CH₂-CH₃) ; 1,05 (t, 3H, CH₂-CH₃, J = 7 Hz).

### 36.4) N-(3-hydroxypropyl)-α-phényl-benzèneacétamide :

Le composé titre est préparé d'une manière analogue à celle de l'intermédiaire 1.1 sauf que l'acide diphénylacétique est utilisé à la place de l'acide benzylique ; p.f. : 85° C.

RMN-¹H (400 MHz, CDCl₃, δ) : 7,26 (m, 10H, 2 x Ph) ; 6,18 (m, 1H, NH-CO) ; 4,92 (s, 1H, CH) ; 3,55 (m, 2H, CH₂-NHCO) ; 3,41 (m, 2H, CH₂OH) ; 3,18 (bs, OH) ; 1,60 (m, 2H, CH₂).

### 36.5) N-(3-iodopropyl)-α-phényl-benzèneacétamide :

Le composé titre est préparé d'une manière analogue à celle de l'intermédiaire 1.2 sauf que le N-(3-hydroxypropyl)-α-phényl-benzèneacétamide est utilisé à la place du α-hydroxy-N-(3-hydroxypropyl)-α-phényl-benzèneacétamide ; p.f. : 114° C.

RMN-¹H (400 MHz, CDCl₃, δ) : 7,30 (m, 10H, 2 x Ph) ; 5,75 (m, 1H, NH-CO) ; 4,92 (s, 1H, CH) ; 3,38 (q, 2H, CH₂-NHCO, J = 6,53 Hz) ; 3,09 (t, 2H, CH₂I, J = 6,75 Hz) ; 2,02 (m, 2H, CH₂).

### 36.6) (±)-N-[3-{4-(2-(1-hydroxypropyl)-phényl)-1-pipérazinyl}-propyl]-α-phényl-benzèneacétamide, fumarate :

710 mg (3,22 mmol) de (±)-2-(1-pipérazinyl)-α-éthyl-benzèneméthanol et 1,21 g (3,22 mmol) de N-(3-iodopropyl)-α-phényl-benzèneacétamide sont mis au reflux dans 20 ml d'acétonitrile pendant 4 heures. Après refroidissement, le solvant est éliminé sous pression réduite, et le résidu est traité tel que décrit dans l'exemple 1 sauf pour la colonne de chromatographie (éluent : acétate d'éthylelméthanol : 98/2). L'évaporation des fractions collectées sous pression réduite donne 0,74 g (49 %) de la base du composé titre qui est convertie en son sel de fumarate en chauffant avec un équivalent d'acide fumarique dans l'éthanol absolu ; p.f. : 76° C.

RMN-¹H (400 MHz, DMSO d6, δ) : 8,29 (m, 1H, NH-CO) ; 7,41-7,09 (m, 14H, 2 x Ph + Ph-N) ; 6,61 (s, 2H, CH=CH) ; 4,92 (s, 1H, CH) ; 4,85 (m, 1H, CH(OH)) ; 3,13 (m, 2H, CH₂-NHCO) ; 2,95 (m, 2H, pipérazine) ; 2,69 (m, 2H, pipérazine) ; 2,50 (bs, 4H, pipérazine) ; 2,37 (m, 2H, CH₂N) ; 1,55 (m, 4H, CH₂ + CH₂-CH₃) ; 0,86 (t, 3H, CH₂-CH₃, J = 7,4 Hz).

Analyse élémentaire pour C₃₀H₃₇N₃O₂, fumarate : % Cale : C, 69,49 ; H, 7,03 ; N, 7,15 ; % obtenus : C, 69,31 ; H, 7,22 ; N, 7,33.

### Exemple 37 : (±)-N-[3-{4-(2-(1-méthoxypropyl)-phényl)-1-pipérazinyl}-propyl]-α-phényl-benzèneacétamide, fumarate (Composé 37) :

### 37.1) (±)-1-benzyl-4-[2-(1-méthoxypropyl)-phényl]-pipérazine :

Dans un ballon tricol anhydre équipé d'une arrivée d'azote sont dissous 1,55 g (5 mmol) d'intermédiaire 36.1 dans 50 ml de DMF sec. 220 mg (5,5 mmol) d'hydrure de sodium (une dispersion 60 % dans l'huile) sont ajoutés en une fois et le mélange réactionnel est agité toute la nuit à température ambiante avant addition de 0,22 ml (5,5 mmol) de iodure de méthyle dilué avec 2 ml de DMF sec. L'agitation est poursuivie pendant 2 autres heures, le mélange est concentré sous pression réduite et le résidu fractionné entre 50 ml d'acétate d'éthyle et **25** ml de NaOH 1N. Après décantation, la phase organique est rincée successivement avec **25** ml de NaOH 1N et **25** ml d'une solution concentrée de chlorure de sodium. La filtration et l'évaporation du solvent donnent une huile qui est purifiée par chromatographie flash (éluent : pétrole léger (bp. 40-65° C) / acétate d'éthyle : 80/20). Les fractions pures sont collectées et concentrées pour donner 0,71 g (68 %) d'une huile incolore.

RMN-¹H (100 MHz, CDCl₃, δ) : 7,30 (m, 9H, 2 x Ph) ; 4,70 (m, 1H, CH) ; 3,60 (s, 2H, CH₂-Ph) ; 3,19 (s, 3H, OCH₃) ; 3,10-2,40(m, 8H, pipérazine) ; 1,70 (m, 2H, CH₂-CH₃) ; 0,92 (t, 3H, CH₂-CH₃, J = 7,0 Hz).

### 37.2) (±)-1-[2-(1-méthoxypropyl)-phényl]-pipérazine :

L'hydrogénation est effectuée telle que décrite pour l'intermédiaire 36.3.

RMN-¹H (100 MHz, CDCl₃, δ) : 7,28 (m, 4H, Ph); 4,70 (m, 1H, CH) ; 3,18 (s, 3H, OCH₃) ; 3,12-2,70 (m, 8H, pipérazine) ; 1,70 (m, 2H, CH₂-CH₃) ; 0,92 (t, 3H, CH₂-CH₃, J = 7,0 Hz).

### 37.3) (±)-N-[3-{4-(2-(1-méthoxypropyl)-phényl)-1-pipérazinyl)-propyl]-α-phényl-benzèneacétamide, fumarate :

Le composé 37 est préparé d'une manière analogue à celle de l'exemple 36 sauf que la (±)-1-[2-(1-méthoxypropyl)-phényl]-pipérazine est utilisée à la place du (±)-2-(1-pipérazinyl)-α-éthyl-benzèneméthanol.

RMN -¹H (400 MHz, DMSO d6, δ) 8,31 (m, 1H, NH-CO) ; 7,22 (m, 14H, 2 x Ph + Ph-N) ; 6,61 (s, 2H, CH=CH) ; 4,93 (s, 1H, CH) ; 4,59 (m, 1H, CH(OCH₃)) ; 3,14 (m, 2H, CH₂-NHCO) ; 3,07 (s, 3H, OCH₃) ; 2,95-2,50 (m, 8H, pipérazine) ; 2,43 (m, 2H, CH₂N) ; 1,60 (m, 4H, CH₂ + CH₂-CH₃) ; 0,86 (t, 3H, CH₂-CH₃, J = 7,33 Hz).

### Exemple 38 : (±)-N-[3-{4-(2,5-diméthylphényl)-1-pipérazinyl}-propyl]-α-méthyl-benzèneacétamide, fumarate (Composé 38) :

Le composé titre est préparé d'une manière analogue à celle de l'exemple 20 sauf que l'acide (±)-2-phénylpropionique est utilisé à la place de l'acide diphénylacétique ; p.f. : 136° C.

RMN-¹H (400 MHz, DMSO d6, δ) : 7,97 (m, 1H, NH-CO) ; 7,25 (m, 5H, Ph) ; 7,02 (d, 1H, Ph-CH₃, J = 7,5 Hz) ; 6,80 (s, 1H, Ph-CH₃) ; 6,78 (d, 1H, Ph-CH₃) ; 6,60 (s, 2H, CH=CH) ; 3,58 (m, 1H, CH) ; 3,07 (m, 2H, CH₂-NHCO) ; 2,82 (bs, 4H, pipérazine) ; 2,57 (bs, 4H, pipérazine) ; 2,39 (m, 2H, CH₂-N) ; 2,23 (s, 3H, Ph-CH₃) ; 2,17 (s, 3H, Ph-CH₃) ; 1,59 (m, 2H, CH₂) ; 1,32 (d, 3H, CH₃, J = 7 Hz).

Analyse élémentaire pour C₂₄H₃₃N₃O, fumarate : % Calc : C, 67,86 ; H, 7,52; N, 8,48 ; % obtenus : C, 67,51 ; H, 7,44 ; N, 8,19.

### Exemple 39 : (±)-N-[3-{4-(2,5-diméthylphényl)-1-pipérazinyl}-propyl]-α-hydroxyméthyl-benzèneacétamide, monochlorhydrate, 0,5 H₂O (Composé 39) :

Le composé 39 est préparé d'une manière analogue à celle de l'exemple 20 sauf que l'acide (±)-tropique est utilisé à la place de l'acide diphénylacétique ; p.f. : 168-169° C.

RMN-¹H (400 MHz, DMSO d6, δ) : 10,77 (bs, 1H, ⁺NH) ; 8,34 (m, 1H, NH-CO) ; 7,28 (m, 5H, Ph) ; 7,06 (d, 1H, Ph-CH₃, J = 7,6 Hz) ; 6,82 (m, 2H, Ph-CH₃) ; 4,48 (bs, 1H, OH) ; 3,96 (m, 1H, CH) ; 3,61 (m, 1H, 1/2 CH₂-OH) ; 3,53 (m, 1H, 1/2 CH₂-OH) ; 3,45 (m, 2H, ⁺NH-CH₂) ; 3,17 (m, 2H, CH₂-NH-CO) 3,10 (m, 10H, pipérazine) ; 2,26 (s, 3H, Ph-CH₃) ; 2,20 (s, 3H, Ph-CH₃) ; 1,87 (m, 2H, CH₂).

Analyse élémentaire pour C₂₄H₃₃N₃O₂, HCl, 0,5 H₂O : % Calc : C, 65,36 ; H, 8,00 ; N, 9,53 ; % obtenus : C, 65,72 ; H, 7,85; N, 9,31.

### Exemple 40 : (±)-N-[3-{4-(2,5-diméthylphényl)-1-pipérazinyl}-propyl]-α-hydroxy-α-méthyl-4-méthoxy-benzèneacétamide, 1,25 fumarate, 0,6 H₂O (Composé 40) :

### 40.1) Ethyl-ester de l'acide (±)-α-hydroxy-α-méthyl-4-méthoxy-benzèneacétique :

L'intermédiaire 40.1 est préparé d'une manière analogue à celle de l'intermédiaire 31.1 sauf que la 4-bromoanisole est utilisée à la place du 3-bromochlorobenzène.

RMN-¹H (100 MHz, CDCl₃, δ) : 7,51-6,82 (m, 4H, Ph) ; 4,25 (q, 2H, CH₂, J = 7,0 Hz) ; 3,81 (s, 3H, OCH₃) ; 3,65 (s, 1H, OH) ; 1,82 (s, 3H, CH₃) ; 1,30 (t, 3H, CH₂-CH₃).

### 40.2) Acide (±)-α-hydroxy-α-méthyl-4-méthoxy-benzèneacétique :

L'intermédiaire 40.2 est préparé d'une manière analogue à celle de l'intermédiaire 31.2 sauf que l'éthyl-ester de l'acide (±)-α-hydroxy-α-méthyl-4-méthoxy-benzèneacétique est utilisé à la place de l'éthyl-ester de l'acide (±)-3-chloro-α-hydroxy-α-méthyl-benzèneacétique ; p.f. : 129-130° C.

RMN-¹H (100 MHz, CDCl₃, δ) : 7,52-6,85 (m, 4H, Ph) ; 4,70 (bs, 2H, OH + CO₂H) ; 3,81 (s, 3H, OCH₃) ; 1,75 (s, 3H, CH₃).

### 40.3) (±)-N-[3-{4-(2,5-diméthylphényl)-1-pipérazinyl}-propyl]-α-hydroxy-α-méthyl-4-méthoxy-benzèneacétamide, 1,25 fumarate, 0,6 H₂O :

Le composé 40 est préparé d'une manière analogue à celle de l'exemple 20 sauf que l'acide (±)-α-hydroxy-α-méthyl-4-méthoxy-benzèneacétique est utilisé à la place de l'acide diphénylacétique; p.f. : 90-91°C.

RMN-¹H (400 MHz, DMSO d6, δ) : 8,12 (m, 1H, NH-CO) ; 7,45 (d, 2H, Ph-OCH₃, J = 8,7 Hz) ; 7,06 (d, 1H, Ph-CH₃, J = 7,57 Hz) ; 6,86 (d, 2H, Ph-OCH₃) ; 6,81 (s, 1H, Ph-CH₃) ; 6,76 (d, 1H, Ph-CH₃) ; 6,60 (s, 2,5H, 1,25 -CH=CH-) ; 3,71 (s, 3H, OCH₃) ; 3,10 (m, 2H, CH₂-NHCO) ; 2,86 (bs, 4H, pipérazine) ; 2,59 (bs, 4H, pipérazine) ; 2,41 (m, 2H, CH₂-N) ; 2,24 (s, 3H, Ph-CH₃) ; 2,17 (s, 3H, Ph-CH₃) ; 1,59 (m, 5H, CH₂, CH₃).

Analyse élémentaire pour C₂₅H₃₅N₃O₃, 1,25 fumarate, 0,6 H₂O : % Calc : C, 61,97 ; H, 7,14 ; N, 7,23; % obtenus : C, 62,41 ; H, 7,02 ; N, 7,22.

### Exemple 41 : (±)-N-[3-{4-(2,5-diméthylphényl)-1-pipérazinyl}-α-hydroxy-α-méthyl-4-méthyl-benzèneacétamide, dichlorhydrate (Composé 41) :

### 41.1) Ethyl-ester de l'acide (±)-α-hydroxy-α-méthyl-4-méthyl-benzèneacétique :

L'intermédiaire 41.1 est préparé d'une manière analogue à celle de l'intermédiaire 31.1 sauf que le 4-bromotoluène est utilisé à la place du 3-bromocblorobenzène.

RMN-¹H (100 MHz, CDCl₃, δ) : 7,51-7,11 (m, 4H, Ph) ; 4,24 (q, 2H, CH₂-CH₃, J = 7,0 Hz) ; 3,78 (s, 1H, OH) ; 2,40 (s, 3H, Ph-CH₃) ; 1,75 (s, 3H, CH₃) ; 1,30 (t, 3H, CH₂-CH₃).

### 41.2) Acide (±)-α-hydraxy-α-méthyl-4-méthyl-benzèneacétique

L'intermédiaire 41.2 est préparé d'une manière analogue à celle de l'intermédiaire 31.2 sauf que l'éthyl-ester de l'acide (±)-α-hydroxy-α-méthyl-4-méthyl-benzèneacétigue est utilisé à la place de l'éthyl-ester de l'acide (±)-3-chloro-α-hydroxy-α-méthyl-benzèneacétique ; p.f. : 105-106° C.

RMN-¹H (100 MHz, CDCl₃, δ) : 7,55-7,15 (m, 4H, Ph) ; 4,80 (bs, 2H, OH + CO₂H) ; 2,41 (s, 3H, Ph-CH₃) ; 1,81 (s, 3H, CH₃).

### 41.3) (±)-N-[3-{4-(2,5-diméthylphényl)-1-plpérazinyl}-propyl]-α-hydroxy-α-méthyl-4-méthyl-benzèneacétamide, dichlorhydrate :

Le composé 41 est préparé de manière analogue à celle de l'exemple 20 sauf que l'acide (±)-α-hydroxy-α-méthyl-4-méthyl-benzèneacétique est utilisé à la place de l'acide diphénylacétique ; p.f. : 145-147° C.

RMN-¹H (400 MHz, DMSO d6, δ) : 10,93 (bs, 1H, ⁺NH) ; 8,12 (m, 1H, NH-CO) ; 7,42 (d, 2H, Ph-C(OH), J = 8 Hz) ; 7,20 (d, 2H, Ph-C(OH)) ; 7,06 (d, 1H, Ph-CH₃, J = 7,9 Hz) ; 6,82 (m, 2H, Ph-CH₃) ; 5,23 (bs, OH) ; 3,41 (m, 2H, ⁺NH-CH₂) ; 3,10 (m, 10H, pipérazine + CH₂-HNCO) ; 2,26 (s, 6H, 2 x Ph-CH₃) ; 2,20 (s, 3H, Ph-CH₃) ; 1,86 (m, 2H, CH₂) ; 1,61 (s, 3H, CH₃).

Analyse élémentaire pour C₂₅H₃₅N₃O₂, 2 HCl : % Calc : C, 62,23 ; H, 7,73 ; N, 8,71; % obtenus : C, 62,24 ; H, 7,94 ; N, 8,76.

### Exemple 42 : (±)-N-[3-{4-(2,5-diméthylphényl)-1-pipérazinyl}-propyl]-α-hydroxy-α-méthyl-1-naphtalèneacétamide, fumarate (Composé 42) :

### 42.1) Ethyl-ester de l'acide (±)-α-hydroxy-α-méthyl-1-naphtalèneacétique :

L'intermédiaire 42.1 est préparé d'une manière analogue celle de à l'intermédiaire 31.1 sauf que le 1-bromonaphtalène est utilisé à la place du 3-bromochlorobenzène.

RMN-¹H (100 MHz, CDCl₃, δ) : 8,30-7,35 (m, 7H, naphtalène) ; 4,19 (q, 2H, CH₂, J =7 Hz) ; 3,69 (s, 1H, OH) ; 2,01 (s, 3H, CH₃) ; 1,09 (t, 3H, CH₂-CH₃).

### 42.2) Acide (±)-α-hydroxy-α-méthyl-1-naphtalèneacétique :

L'intermédiaire 42.2 est préparé d'une manière analogue à celle de l'intermédiaire 31.2 sauf que l'éthyl-ester de l'acide (±)-α-hydroxy-α-méthyl-1-naphtalèneacétique est utilisé à la place l'éthyl-ester de l'acide (±)-3-chloro-α-hydroxy-α-méthyl-benzèneacétique ; p.f.: 152-153° C.

RMN-¹H (100 MHz, CD₃OD, δ) : 8.54 (m, 1H, naphtalène) ; 8.10-7.50 (m, 6H, naphtalène) ; 2.18 (s, 3H, CH₃).

### 42.3) (±)-N-[3-{4-(2,5-diméthylphényl)-1-pipérazinyl}-propyl]-α-hydroxy-α-méthyl-1-naphtalèneacétamide, fumarate :

Le composé 42 est préparé d'une manière analogue à celle de l'exemple 20 sauf que l'acide (±)-α-hydroxy-α-méthyl-1-naphtalèneacétique est utilisé à la place de l'acide diphénylacétique ; p.f. : 136-137° C.

RMN-¹H (400 MHz, DMSO d6, δ) : 8,41 (d, 1H, naphtalène) ; 8,29 (m, 1H, NH-CO) ; 7,89 (d, 1H, naphtalène, J = 7,39 Hz) ; 7,84 (d, 1H, naphtalène, J = 8,10 Hz) ; 7,63 (d, 1H, naphtalène, J = 7,21 Hz) ; 7,45 (m, 3H, naphtalène) ; 7,01 (d, 1H, Ph-CH₃, J = 7,52 Hz) ; 6,79 (s, 1H, Ph-CH₃) ; 6.74 (d, 1H, Ph-CH₃) ; 6,60 (s, 2H, -CH=CH-); 6,25 (bs, 1H, OH) ; 3,19 (m, 2H, CH₂-NHCO) ; 2,84 (bs, 4H, pipérazine) ; 2,56 (bs, 4H, pipérazine) ; 2,43 (m, 2H, CH₂-N) ; 2,22 (s, 3H, Ph-CH₃) ; 2,17 (s, 3H, Ph-CH₃) ; 1,83 (s, 3H, CH₃) ; 1,66 (m, 2H, CH₂).

Analyse élémentaire pour C₂₈H₃₅N₃O₂, fumarate : % Calc : C, 68,43 ; H, 7,00 ; N, 7,48 ; % obtenus : C, 68,58 ; H, 7,16 ; N, 7,53.

### Exemple 43 : (±)-N-[3-{4-(2,5-diméthylphényl)-1-pipérazinyl}-propyl]-α-hydroxy-α-méthyl-2-naphtalèneacétamide, 0,75 fumarate (Composé 43) :

### 43.1) Ethyl-ester de l'acide (±)-α-hydroxy-α-méthyl-2-naphtalèneacétique :

L'intermédiaire 43.1 est préparé d'une manière analogue à celle de l'intermédiaire 31.1 sauf que le 2-bromonaphtalène est utilisé à la place du 3-bromochlorobenzène.

RMN-¹H (100 MHz, CDCl₃, δ) : 8,10-7,40 (m, 7H, naphtalène) ; 4,25 (q, 2H, CH₂, J = 7 Hz) ; 3,95 (s, 1H, OH) ; 1,90 (s, 3H, CH₃) ; 1,29 (t, 3H, CH₂-CH₃).

### 43.2) Acide (±)-α-hydroxy-α-méthyl-2-naphtalèneacétique :

L'intermédiaire 43.2 est préparé d'une manière analogue à celle de l'intermédiaire 31.2 sauf que l'éthyl-ester de l'acide (±)-α-hydroxy-α-méthyl-2-naphtalèneacétique est utilisé à la place de l'éthyl-ester de l'acide (±)-3-chloro-α-hydroxy-α-méthyl-benzèneacétique ; p.f.: 169-170° C.

RMN-¹H (100 MHz, CD₃OD, δ) : 8,75-7,56 (m, 7H, naphtalène) ; 2,05 (s, 3H, CH₃).

### 43.3) (±)-N-[3-{4-(2,5-diméthylphényl)-1-pipérazinyl}-propyl]-α-hydroxy-α-méthyl-2-naphtalèneacétamide, 0,75 fumarate :

Le composé 43 est préparé d'une manière analogue à celle de l'exemple 20 sauf que l'acide (±)-α-hydroxy-α-méthyl-2-naphtalèneacétique est utilisé à la place de l'acide diphénylacétique ; p.f. : 132-133° C.

RMN-¹H (400 MHz, DMSO d6, δ) : 8,17 (m, 1H, NH-CO); 8,03 (s, 1H, naphtalène) ; 7,89 (m, 3H, naphtalène) ; 7,71 (dd, 1H, naphtalène, J₁₂ = 8,60 Hz, J₁₃ = 1,42 Hz) ; 7,47 (m, 2H, naphtalène) ; 7,02 (d, 1H, Ph-CH₃, J = 7,57 Hz) ; 6,80 (s, 3H, Ph-CH₃) ; 6,76 (d, 1H, Ph-CH₃) ; 6,61 (s, 1,5H, 0,75 x -CH=CH-) ; 3,12 (m, 2H, CH₂-NHCO) ; 2,82 (bs, 4H, pipérazine) ; 2,54 (bs, 4H, pipérazine) ; 2,39 (m, 2H, CH₂-N) ; 2,24 (s, 3H, Ph-CH₃) ; 2,16 (s, 3H, Ph-CH₃) ; 1,73 (s, 3H, CH₃) ; 1,16 (m, 2H, CH₂).

Analyse élémentaire pour C₂₈H₃₅N₃O₂, 0,75 fumarate : % Calc : C, 69,90 ; H, 7,19 ; N, 7,89 ; % obtenus : C, 69,64 ; H, 7,12 ; N, 7,56.

### Exemple 44 : (±)-N-[3-{4-(2,5-diméthylphényl)-1-pipérazinyl}-propyl]-α-hydroxy-α-méthyl-1,1'-biphényl-3-acétamide, fumarate (Composé 44) :

### 44.1) Ethyl-ester de l'acide (±)-α-hydroxy-α-méthyl-1,1'-biphényl-3-acétique :

L'intermédiaire 44.1 est préparé d'une manière analogue à celle de l'intermédiaire 31.1 sauf que le 3-bromobiphényle est utilisé à la place du 3-bromochlorobenzène.

RMN-¹H (100 MHz, CDCl₃, δ) : 7,80-7,35 (m, 9H, biphényl) ; 4,25 (q, 2H, CH₂, J = 7,0 Hz) ; 3,83 (s, 1H, OH) ; 1,81 (s, 3H, CH₃) ; 1,29 (t, 3H, CH₂-CH₃).

### 44.2) Acide (±)-α-hydroxy-α-méthyl-1,1'-biphényl-3-acétique :

L'intermédiaire 44.2 est préparé d'une manière analogue à celle de l'intermédiaire 31.2 sauf que l'éthyl-ester de l'acide (±)-α-hydroxy-α-méthyl-1,1'-biphényl-3-acétique est utilisé à la place de l'éthyl-ester de l'acide (±)3-chloro-α-hydroxy-α-méthyl-benzèneacétique ; p.f. : 118-119° C.

RMN-¹H (100 MHz, CDCl₃, δ) : 7,82-7,35 (m, 9H, biphényl) ; 6,20 (bs, 2H, OH, CO₂H) ; 1,90 (s, 3H, CH₃).

### 44.3) (±)-N-[3-{4-(2,5-diméthylphényl)-1-pipérazinyl}-propyl]-α-hydroxy-α-méthyl-1,1'-biphényl-3-acétamide, fumarate :

Le composé 44 est préparé d'une manière analogue à celle de l'exemple 20 sauf que l'acide (±)-α-hydroxy-α-méthyl-1,1'-biphényl-3-acétique est utilisé à la place de l'acide diphénylacétique; p.f.: 90-91° C.

RMN-¹H (400 MHz, DMSO d6, δ) : 8,19 (m, 1H, NH-CO) ; 7,83 (s, 1H, biphényl) ; 7,63-7,34 (m, 8H, biphényl) ; 7,02 (d, 1H, Ph-CH₃, J = 7,54 Hz) ; 6,79 (s, 1H, Ph-CH₃) ; 6,75 (d, 1H, Ph-CH₃) ; 6,61 (s, 2H, -CH=CH-) ; 3,12 (m, 2H, CH₂-NHCO) ; 2,89 (bs, 4H, pipérazine) ; 2,50 (bs, 4H, pipérazine) ; 2,39 (m, 2H, CH₂-N) ; 2,23 (s, 3H, Ph-CH₃) ; 2,15 (s, 3H, Ph-CH₃) ; 1,68 (s, 3H, CH₃) ; 1,16 (m, 2H, CH₂).

Analyse élémentaire pour C₃₀H₃₇N₃O₂, fumarate : % Calc : C, 69,49 ; H, 7,03 ; N, 7,15 ; % obtenus : C, 69,65 ; H, 6,94 ; N, 7,38.

### Exemple 45 : S-(+)-N-[3-{4-(2,5-diméthylphényl)-1-pipérazinyl}-propyl]-α-hydroxy-α-méthyl-benzèneacétamide, fumarate (Composé 45) :

Le composé 45 est préparé d'une manière analogue à celle de l'exemple 20 sauf que l'acide S-(+)-atrolactique est utilisé à la place de l'acide diphénylacétique ; p.f. : 190-191°C ([α]D = + 10,75 (c = 0,12, EtOH ; temp = 20° C)).

RMN-¹H (400 MHz, DMSO d6, δ) : 8,09 (m, 1H, NH-CO) ; 7,53 (m, 2H, Ph) ; 7,31 (m, 2H, Ph) ; 7,22 (m, 1H, Ph) ; 7,02 (d, 1H, Ph-CH₃, J = 7,57 Hz) ; 6,81 (s, 1H, Ph-CH₃) ; 6,76 (d, 1H, Ph-CH₃) ; 6,60 (s, 2H, -CH=CH-) ; 3,10 (m, 2H, CH₂-NHCO) ; 2,86 (bs, 4H, pipérazine) ; 2,58 (bs, 4H, pipérazine) ; 2,41 (m, 2H, CH₂-N) ; 2,24 (s, 3H, Ph-CH₃) ; 2,17 (s, 3H, Ph-CH₃) ; 1,61 (s, 3H, CH₃) ; 1,59 (m, 2H, CH₂).

Analyse élémentaire pour C₂₄H₃₃N₃O₂, fumarate : % Calc : C, 65,73 ; H, 7,29 ; N, 8,21 ; % obtenus : C, 65,78 ; H, 7,51 ; N, 8,07.

### Exemple 46 : R-(-)-N-[3-{4-(2,5-diméthylphényl)-1-pipérazinyl}-propyl]-α-hydroxy-α-méthyl-benzèneacétamide, 0,8 fumarate (Composé 46) :

Le composé 46 est préparé d'une manière analogue à celle de l'exemple 20 sauf que l'acide R-(-)-atrolactique est utilisé à la place de l'acide diphénylacétique ; p.f. : 190-191° C ([α]D = -7,25 (c = 0,10, EtOH ; temp = 20° C)).

Analyse élémentaire pour C₂₄H₃₃N₃O₂, 0,8 fumarate : % Calc : C, 66,89 ; H, 7,47 ; N, 8,60 ; % obtenus : C, 66,43 ; H, 7,25 ; N, 8,56.

### Exemple 47 : (±)-N-[3-{4-(2,5-diméthylphényl)-1-pipérazinyl}-propyl]-α-benzylaminocarbonyl-benzèneacétamide, fumarate (Composé 47) :

### 47.1) Benzyl-ester de l'acide (±)-α-benzylaminocarbonyl-benzèneacétique :

4,05 g (15 mmol) du monobenzyl-ester de l'acide phénylmalonique, 1,64 ml (15 mmol) de benzylamine et 2,23 g (16,5 mmol) d'hydroxybenzotriazole sont dissous dans 40 ml de dichlorométhane. Après refroidissement jusqu'à 0-5° C, 3,4 g (16,5 mmol) de dicyclohexylcarbodiimide sont ajoutés en une fois, et l'agitation est poursuivie à 20° C pendant 4 heures. Le précipité blanc formé durant la réaction est filtré, et le filtrat est successivement rincé avec NaOH 1N (2 x 50 ml) et une solution saturée de chlorure de sodium (50 ml). Après séchage sur du sulphate de sodium, le solvant est évaporé, et le résidu est précipité en présence de dichlorométhane. Une filtration donne 3,19 g (59 %) d'une poudre blanche ; p.f. : 150-151° C.

RMN-¹H (100 MHz, CD₃OD, δ) : 7,70-7,35 (m, 15H, Ph) ; 5,35 (s, 2H, CH₂-O) ; 5,05 (s, 1H, CH); 4,51 (d, 2H, CH2-N).

### 47.2) Acide (±)-α-benzylaminocarbonyl-benzèneacétique :

Dans une bouteille Parr sont introduits 1 g (2,78 mmol) de benzyl-ester de l'acide (±)-α-benzylaminocarbonyl-benzèneacétique dissous dans un mélange de 40 ml d'éthanol et 30 ml d'acide acétique et 200 mg de Pd/C 10 %. L'hydrogénation est effectuée sous 15 PSI H₂ à 30° C. Après 15 heures d'agitation, le mélange est filtré au travers de Celite, et le filtrat est concentré sous pression réduite. Le résidu est fractionné entre 30 ml de diéthyléther et 30 ml d'une solution saturée de NaHCO₃. L'extrait aqueux est décanté et rincé avec 30 ml de diéthyléther. Après acidification avec HCI 3N à 0° C, la phase aqueuse est extraite avec 2 x 30 ml de diéthyléther. La solution éthérée est séchée sur du sulfate de sodium, filtrée, et le solvant évaporé pour donner 451 mg (60 %) d'une poudre blanche ; p.f.: 132-133° C.

RMN-¹H (100 MHz, CD₃OD, δ) : 7,70-7,35 (m, 10H, Ph) ; 4,80 (s, 1H, CH); 4,57 (m, 2H, CH₂-N).

### 47.3) (±)-N-[3-{4-(2,5-diméthylphényl)-1-pipérazinyl}-propyl]-α-benzylaminocarbonyl-benzèneacétamide, fumarate :

Le composé 47 est préparé d'une manière analogue à celle de l'exemple 20 sauf que l'acide (±)-α-benzylaminocarbonyl-benzèneacétique est utilisé à la place de l'acide diphénylacétique ; p.f.: 90-91° C.

RMN-¹H (400 MHz, DMSO d6, δ) ; 8,69 (m, 1H, NH-CH₂Ph) ; 8,26 (m, 1H, NH-CO); 7,32 (m, 10H, 2 x Ph) ; 7,02 (d, 1H, Ph-CH₃, J = 7,51 Hz) ; 6,80 (s 1H, Ph-CH₃) ; 6,76 (d, 1H, Ph-CH₃) ; 6,61 (s, 2H, -CH=CH-); 4,47 (s, 1H, CH) ; 4,31 (m, 2H, NH-CH₂-Ph) ; 3,15 (m, 2H, CH₂-NHCO) ; 2,83 (bs, 4H, pipérazine) ; 2,58 (bs, 4H, pipérazine) ; 2,42 (m, 2H, CH₂-N) ; 2,23 (s, 3H, Ph-CH₃) ; 2,17 (s, 3H, Ph-CH₃) ; 1,63 (m, 2H, CH₂).

Analyse élémentaire pour C₃₁H₃₈N₄O₂, fumarate : % Calc: C, 68,38 ; H, 6,89 ; N, 9,11 ; % obtenus : C, 68,70 ; H, 7,06 ; N, 8,81.

### Exemple 48 : (±)-N-[3-{4-{2,5-diméthylphényl)-1pipérazinyl}-propyl]-α-(4-méthylphényl)-sulfonylamino-benzèneacétamide, 1,5 HCl, 0,5 H₂O (Composé 48) :

### 48.1) Acide (±)-(4-méthylphényl)-sulfonylamino-benzèneacétique :

L'intermédiaire 48.1 est préparé d'une manière analogue à celle de l'intermédiaire 33.1 sauf que le chlorure de p-toluènesulfonyle est utilisé à la place du chlorure de méthanesulfonyle ; p.f. : 188-189° C.

RMN-¹H (100 MHz, CD₃OD, δ) : 7,65 (m, 4H, p-toluène) ; 7,42 (s, 5H, Ph) ; 5,09 (d, 1H, CH); 2,55 (s, 3H, CH₃).

### 48.2) (±)-N-[3-{4-(2,5-diméthylphényl)-1-pipérazinyl}-propyl]-α-(4-méthylphényl)-sulfonylamino-benzèneacétamide, 1,5 HCl, 0,5 H₂O :

Le composé 48 est préparé d'une manière analogue à l'exemple 20 sauf que l'acide (±)-(4-méthylphényl)-sulfonylamino-benzèneacétique est utilisé à la place de l'acide diphénylacétique ; p.f. : 180-181° C.

RMN-¹H (400 MHz, DMSO d6, δ) : 11,19 (bs, 1H, ⁺NH) ; 8,54 (m, 2H, NH-CO, NH-SO₂) ; 7,63 (m, 2H, Ph-SO₂) ; 7,26 (m, 7H, Ph-SO₂, Ph) ; 7,06 (d, 1H, Ph-CH₃, J = 7,05 Hz) ; 6,83 (bs, 2H, Ph-CH₃) ; 4,92 (d, 1H, CH, J = 8,80 Hz); 3,37 (m, 2H, ⁺NH-CH₂) ; 3,12 (bs, 4H, pipérazine) ; 2,97 (m, 6H, pipérazine, CH₂-NHCO) ; 2,35 (s, 3H, CH₃-PhSO₂) ; 2,26 (s, 3H, Ph-CH₃) ; 2,20 (s, 3H, Ph-CH₃) ; 1,77 (m, 2H, CH₂).

Analyse élémentaire pour C₃₀H₃₈N₄O₃S, 1,5 HCl, 0,5 H₂O : % Cale : C, 60,21 ; H, b,82 ; N, 9,36 ; S, 5,36 ; % obtenus : C, 60,59 ; H, 6,81 ; N, 9,21 ; S, 5,69.

### Exemple 49 : (±)-N-[3-{4-(2,5-diméthylphényl)-1-pipérazinyl}-propyl]-α-benzylcarbonylamino-benzèneacétamide, fumarate, 0,5 H₂O (Composé 49) :

### 49.1) Acide (±)-α-benzylcarbonylamino-benzèneacétique :

L'intermédiaire 49.1 est préparé d'une manière analogue à celle de l'intermédiaire 33.1 sauf que le chlorure de phénylacétyle est utilisé à la place du chlorure du méthanesulfonyle.

RMN-¹H (100 MHz, CDCl₃, δ) : 8,80 (bs, 1H, CO₂H) ; 7,30 (bs, 10H, 2 x Ph) ; 5,52 (d, 1H, NHCO, J = 7,0 Hz) ; 3,60 (m, 3H, CH + CH₂).

### 49.2) (±)-N-[3-{4-(2,5-diméthylphényl)-1-pipérazinyl}-propyl]-α-benzylcarbonylamino-benzèneacétamide, fumarate, 0,5 H₂O :

Le composé 49 est préparé d'une manière analogue à celle de l'exemple 20 sauf que l'acide (±)-α-benzylcarbonylamino-benzèneacétique est utilisé à la place de l'acide diphénylacétique ; p.f. : 158-159° C.

RMN-¹H (400 MHz, DMSO d6, δ) : 8,73 (d, 1H, CH-NH-CO, J = 8,0 Hz) ; 8,32 (m, 1H, NH-CO) ; 7,42-7,20 (m, 10H, 2 x Ph) ; 7,02 (d, 1H, Ph-CH₃, J = 7,52 Hz) ; 6,80 (s, 1H, Ph-CH₃) ; 6,75 (d, 1H, Ph-CH₃) ; 6,61 (s, 2H, -CH=CH-) ; 5,43 (d, 1H, CH) ; 3,56 (s, 2H, CH₂-Ph) ; 3,10 (m, 2H, CH₂-NHCO) ; 2,80 (bs, 4H, pipérazine) ; 2,50 (bs, 4H, pipérazine) ; 2,34 (m, 2H, CH₂-N) ; 2,23 (s, 3H, Ph-CH₃) ; 2,17 (s, 3H, Ph-CH₃) ; 1,58 (m, 2H, CH₂).

Analyse élémentaire pour C₃₁H₃₈N₄O₂, fumarate, 0,5 H₂O : % Calc : C, 67,40 ; H, 6,95 ; N, 8,98 ; % obtenus : C, 67,57 ; H, 6,86 ; N, 8,98.

### Exemple 50 : (±)-N-[3-{4-(2,5-diméthylphényl)-1-pipérazinyl}-propyl]-α-benzylaminocarbonyl-α-méthyl-benzèneacétamide, fumarate, 0,5 H₂O (Composé 50) :

### 50.1) Diéthyl-ester de l'acide (±)-α-méthyl-phénylmalonique :

L'intermédiaire 50.1 est préparé d'une manière analogue à celle de l'exemple 11 sauf que le diéthyl-ester de l'acide phénylmalonique est utilisé à la place du (±)-N-[3-{4-(2,5-diméthylphényl)-1-pipérazinyl)-propyl]-α-hydroxy-α-phényl-benzèneacétamide.

RMN-¹H (100 MHz, CDCl₃, δ) : 7,39 (bs, 5H, Ph) ; 4,28 (q, 4H, OCH₂, J = 7,0 Hz) ; 1,88 (s, 3H, CH₃) ; 1,26 (t, 3H, CH₂-CH₃).

### 50.2) Monoéthyl-ester de l'acide (±)-α-méthyl-phénylmalonique :

L'intermédiaire 50.2 est préparé d'une manière analogue à l'intermédiaire 31.2 sauf que le diéthyl-ester de l'acide (±)-α-méthyl-phénylmalonique est utilisé à la place de l'éthyl-ester de l'acide (±)-3-chloro-α-hydroxy-α-méthyl-benzèneacétique.

RMN-¹H (100 MHz, CDCl₃, δ) : 9,38 (bs, 1H, CO₂H) ; 7,38 (m, 5H, Ph) ; 4,25 (q, 2H, OCH₂, J = 7,0 Hz) ; 1,90 (s, 3H, CH₃) ; 1,28 (t, 3H, CH₂-CH₃).

### 50.3) Ethyl-ester de l'acide (±)-α-benzylaminocarbonyl-α-méthyl-benzèneacétique :

L'intermédiaire 50.3 est préparé d'une manière analogue à celle de l'intermédiaire 47.1 sauf que le monoéthyl-ester de l'acide (±)-α-méthyl-phénylmalonique est utilisé à la place du monobenzyl-ester de l'acide phénylmalonique.

RMN-¹H (100 MHz, CDCl₃, δ) : 7,30 (m, 10H, 2 x Ph) ; 6,82 (m, 1H, NH-CO) ; 4,48 (m, 2H, CH₂) ; 4,27 (q, 2H, OCH₂, J = 7,0 Hz) ; 1,87 (s, 3H, CH₃) ; 1,26 (t, 3H, CH₂-CH₃).

### 50.4) Acide(±)-α-benzylaminocarbonyl-α-méthyl-benzènescétique :

L'intermédiaire 50.4 est préparé d'une manière analogue à celle de l'intermédiaire 31.2 sauf que l'éthyl-ester de l'acide (±)-α-benzylaminocarbonyl-α-méthyl-benzèneacétique est utilisé à la place de l'éthyl-ester de l'acide (±)-3-chloro-α-hydroxy-α-méthyl-benzèneacétique.

RMN-¹H (100 MHz, CDCl₃, δ) : 7,25 (m, 10H, 2 x Ph) ; 6,26 (m, 1H, NH-CO) ; 4,45 (m, 2H, CH₂) ; 1,95 (s, 3H, CH₃).

### 50.5) (±)-N-[3-{4-(2,5-diméthylphényl)-1-pipérazinyl}-propyl]-α-benzylaminocarbonyl-α-méthyl-benzèneacétamide, fumarate, 0,5 H₂O :

Le composé 50 est préparé d'une manière analogue à celle de l'exemple 20 sauf que l'acide (±)-α-benzylaminocarbonyl-α-méthyl-benzèneacétique est utilisé à la place de l'acide diphénylacétique p.f. : 159-160° C.

RMN-¹H (400 MHz, DMSO d6, δ) : 8,40 (m, 1H, PhCH₂-NH-CO) ; 7,93 (m, 1H, NH-CO) ; 7,26 (m, 10H, 2 x Ph) ; 7,02 (d, 1H, Ph-CH₃, J = 7,52 Hz) ; 6,79 (s, 1H, Ph-CH₃) ; 6,75 (d, 1H, Ph-CH₃) ; 6,61 (s, 2H, -CH=CH-) ; 4,33 (d, 2H, CH₂-Ph, J = 5,85 Hz) ; 3,19 (m, 2H, CH₂-NHCO) ; 2,79 (bs, 4H, pipérazine) ; 2,54 (bs, 4H, pipérazine) ; 2,39 (m, 2H, CH₂-N) ; 2,24 (s, 3H, Ph-CH₃) ; 2,17 (s, 3H, Ph-CH₃) ; 1,71 (s, 3H, CH₃); 1,63 (m, 2H, CH₂).

Analyse élémentaire pour C₃₂H₄₀N₄O₂, fumarate, 0,5 H₂O : % Calc : C, 67,80 ; H, 7,11 ; N, 8,78 ; % obtenus : C, 68,20 ; H, 7,08 ; N, 8,91.

### Exemple 51 : (±)-N-[3-{4-(2,5-diméthylphényl)-1-pipérazinyl}-propyl]-α-méthoxy-α-méthyl-benzèneacétamide, 0,5 fumarate (Composé 51) :

Le composé 51 est préparé d'une manière analogue à celle de l'exemple 11 sauf que la (±)-N-[3-{4-(2,5-diméthylphényl)-1-pipérazinyl}-propyl]-α-hydroxy-α-méthyl-benzèneacétamide est utilisée à la place de la (±)-N-[3-{4-(2,5-diméthylphényl)-1-pipérazinyl)-propyl]-α-hydroxy-α-phényl-benzèneacétamide ; p.f. : 172,5-173,5° C.

RMN-¹H (400 MHz, DMSO d6, δ) : 8,16 (m, 1H, NH-CO) ; 7,35 (m, 5H, pH) ; 7,02 (d, 1H, Ph-CH₃, J = 7,54 Hz) ; 6,80 (s, 1H, Ph-CH₃) ; 6,75 (d, 1H, Ph-CH₃) ; 6,60 (s, 1H, 0,5 x -CH=CH-) ; 3,13 (m, 5H, CH₂-NHCO + OCH₃) ; 2,84 (bs, 4H, pipérazine) ; 2,54 (bs, 4H, pipérazine) ; 2,37 (m, 2H, CH₂-N) ; 2,23 (s, 3H, Ph-CH₃) ; 2,18 (s, 3H, Ph-CH₃) ; 1,64 (s, 3H, CH₃) ; 1,61 (m, 2H, CH₂).

Analyse élémentaire pour C₂₅H₃₅N₃O₂, 0,5 fumarate : % Calc : C, 69,35 ; H, 7,98 ; N, 8,99 ; % obtenus : C, 69,34 ; H, 7,99 ; N, 8,69.

### Exemple 52 : (±)-N-[3-{4-(2,5-diméthylphényl)-1-pipérazinyl}-propyl]-α-benzyloxy-α-méthyl-benzèneacétamide, 0,5 fumarate (Composé 52) :

Le composé 52 est préparé d'une manière analogue à celle de l'exemple 11 sauf que la (±)-N-[3-{4-(2,5-diméthylphényl)-1-pipérazinyl}-propyl)-α-hydroxy-α-méthyl-benzèneacétamide et le bromure de benzyle sont utilisés à la place de la (±)-N-[3-{4-(2,5-diméthylphényl)-1-pipérazinyl}-propyl]-α-hydroxy-α-phényl-benzèneacétamide et de l'iodure de méthyle ; p.f. : 153-154° C.

RMN-¹H (400 MHz, DMSO d6, δ) : 7,94 (m, 1H, NH-CO) ; 7,50-7,26 (m, 10H, 2 x Ph) ; 7,00 (d, 1H, Ph-CH₃, J = 7,46 Hz) ; 6,74 (d, 1H, Ph-CH₃) ; 6,71 (s, 1H, Ph-CH₃) ; 6,61 (s, 1H, 0,5 x -CH=CH-) ; 4,35 (AB, 2H, CH₂-Ph, J_{AB} = 12,25 Hz) ; 3,17 (m, 2H, CH₂-NHCO) ; 2,73 (bs, 4H, pipérazine) ; 2,50 (bs, 4H, pipérazine) ; 2,34 (m, 2H, CH₂-N); 2,22 (s, 3H, Ph-CH₃) ; 2,15 (s, 3H, Ph-CH₃) ; 1,74 (s, 3H, CH₃) ; 1,61 (m, 2H, CH₂).

Analyse élémentaire pour C₃₁H₃₉N₃O₂, 0,5 fumarate : % Calc : C, 72,90 ; H, 7,60 ; N, 7,73 ; % obtenus : C, 73,01 ; H, 7,46 ; N, 7,27.

### Exemple 53 : (±)-3,4-dichloro-N-[3-{4-(2,5-diméthylphényl)-1-pipérazinyl}-propyl]-α-hydroxy-α-méthyl-benzèneacétamide, fumarate (Composé 53) :

Le composé 53 est préparé d'une manière analogue à celle de l'exemple 31 sauf que le 1-bromo-3,4-dichlorobenzène est utilisé à la place du 3-bromvchlorobenzène ; p.f. : 174-175° C.

RMN-¹ H (400 MHz, DMSO d6, δ) : 8,19 (m, 1H, NH-CO) ; 7,73 (d, 1H, Ph-Cl, J = 1,17 Hz) ; 7,59 (d, 1H, PhCl, J = 8,44 Hz) ; 7,50 (dd, 1H, PhCl) ; 7,02 (d, 1H, Ph-CH₃, J = 7,52 Hz) ; 6,81 (s, 1H, Ph-CH₃) ; 6,76 (d, 1H, Ph-CH₃) ; 6,60 (s, 2H, -CH=CH-) ; 3,10 (m, 2H, CH₂-NHCO) ; 2,85 (bs, 4H, pipérazine) ; 2,56 (bs, 4H, pipérazine) ; 2,38 (m, 2H, CH₂-N) ; 2,24 (s, 3H, Ph-CH₃) ; 2,17 (s, 3H, Ph-CH₃) ; 1,59 (m, 5H, CH₃ + CH₂).

Analyse élémentaire pour C₂₄H₃₁Cl₂N₃O₂, fumarate : % Calc : C, 57,93 ; H, 6,08; N, 7,24 ; % obtenus : C, 57,99 ; H, 6.24 ; N, 7,03.

### Exemple 54 : (±)-N-[3-{4-(2,5-diméthylphényl)-1-pipérazinyl}-propyl]-2-hydroxy-2-méthyl-propionamide, fumarate (Composé 54) :

Le composé 54 est préparé d'une manière analogue à celle de l'exemple 20 sauf que l'acide 2-hydroxyisobutyrique est utilisé à la place de l'acide diphénylacétique ; p.f. : 150-151° C.

RMN-¹H (400 MHz, DMSO d6, δ) : 7,85 (m, 1H, NH-CO) ; 7,02(d, 1H, Ph-CH₃, J = 7,52 Hz) ; 6,81 (s, 1H, Ph-CH₃) ; 6,76 (d, 1H, Ph-CH₃) ; 6,60 (s, 2H, -CH=CH-) ; 3,13 (m, 2H, CH₂-NHCO) ; 2,88 (bs, 4H, pipérazine) ; 2,67 (bs, 4H, pipérazine) ; 2,38 (m, 2H, CH₂-N) ; 2,24 (s, 3H, Ph-CH₃) ; 2,18 (s, 3H, Ph-CH₃) ; 1,66 (m, 2H, CH₂) ; 1,24 (s, 6H, 2 x CH₃).

Analyse élémentaire pour C₁₉H₃₁N₃O₂, fumarate : % Calc : C, 61,45 ; H, 7,85 ; N, 9,35 ; % obtenus : C, 61,00 ; H, 7,89 ; N, 9,22.

### Exemple 55 : (±)-N-[3-{4-(2-(cyclopropylhydroxyméthyl)-phényl)-1-pipérazinyl}-propyl]-α-phényl-benzèneacétamide, 1,25 fumarate, 0,5 H₂O (Composé 55) :

Le composé 55 est préparé d'une maniëre analogue à celle de l'exemple 36 sauf que le bromure de cyclopropylmagnésium est utilisé à la place du bromure d'éthylmagnésium ; p.f. : 151-152° C.

RMN-¹H (400 MHz, DMSO d6, δ) : 8,31 (m, 1H, NH-CO); 7,49-7,09 (m, 14H, 3 x Ph) ; 6,61 (s, 2,5H, 1,25 x -CH=CH-) ; 4,92 (s, 1H, Ph-CH) ; 4,51 (d, 1H, CH-(OH), J = 6,71 Hz) ; 3,13 (m, 2H, CH₂-NHCO) ; 3,00 (bs, 2H, pipérazine) ; 2,72 (bs, 2H, pipérazine) ; 2,58 (bs, 4H, pipérazine) ; 2,41 (m, 2H, CH₂-N) ; 1,63 (m, 2H, CH₂) ; 1,05 (m, 1H, CH) ; 0,36 (m, 4H, cyclopropane).

Analyse élémentaire pour C₃₁H₃₇N₃O₂, 1,25 fumarate, 0,5 H₂O : % Calc : C, 67,80 ; H, 6,80 ; N, 6,59 ; % obtenus : C, 68,21 ; H, 6,82 ; N, 6,12.

### Exemple 56 : (±)-N-[3-{4-(2-(propyl)-phényl)-1-pipérazinyl}-propyl]-α-hydroxy-α-phényl-benzèneacétamide, 0,75 fumarate (Composé 56) :

### 56.1) (Z,E)-1-benzyl-4-[2-(1-propényl)-phényl]-pipérazine :

Dans un ballon tricol anhydre pourvu d'une arrivée d'azote contenant 80 ml de THF sec sont introduits 5,7 g (15,3 mmol) de bromure d'éthyltriphénylphosphonium. A ce mélange sont ajoutés goutte à goutte, à 20° C, 8,45 ml (16,9 mmol) d'une solution de phényllithium (2 M dans cyclohexaneldiéthyléther : 7,3). L'agitation est poursuivie pendant une heure et à ce mélange d'un rouge foncé, refroidi à 0° C, est ajoutée goutte à goutte une solution de 4,3 g (15,3 mmol) de l'intermédiaire 36.1 dans 30 ml de THF sec. Après agitation toute la nuit, le précipité est filtré et rincé avec de l'acétate d'éthyle. Le filtrat est concentré sous vide, et le résidu huileux marron est soumis à chromatographie sur gel de silice (éluent : pétrole léger (bp. 40-65° C) / acétate d'éthyle : 90/10) pour donner 2,71 g (60 %) d'une huile pure jaune pâle.

RMN-¹H (100 MHz, CDCl₃, δ): 7,45-6,90 (m, 9H, 2 x Ph) ; 6,60 (m, 1H, Ph-CH =CH-) ; 6,20 (m, 1/2 CH, 1/2 x Ph-CH=CH-) ; 5,79 (m, 1/2 CH, 1/2 x Ph-CH=CH-) ; 3,60 (m, 2H, CH₂-Ph) ; 3,00 (m, 4H, pipérazine) ; 2,60 (m, 4H, pipérazine) ; 1,90 (m, 3H CH₃).

### 56.2) 1-[2-(1-propényl)-phényl]-pipérazine

Un mélange de 2,71 g (9,3 mmol) de (Z,E)-1-benzyl-4-[2-(1-propényl)-phényl]-pipérazine et 100 mg de Pd/C 10 % dans 30 ml d'éthanol sont hydrogénés sous 40 PSI H₂ à 40° C pendant 15 heures dans un appareil Parr. Puis le mélange réactionnel est filtré sur une couche de Celite, et le solvant est évaporé sous pression réduite pour donner 1,78 g (94 %) d'une poudre blanche ; p.f. : 145-146° C.

RMN-¹H (100 MHz, CDCl₃, δ) : 7,18 (m, 4H, Ph) ; 4,55 (bs, NH) ; 3,30-2,90 (m, 8H, pipérazine) ; 2,60 (m, 2H, CH₂-Ph) ; 1,68 (m, 2H, CH₂) ; 1,00 (t, 3H, CH₃, J = 7,0 Hz).

### 56.3) (±)-N-[3-{4-(2-(propyl)-phényl)-1-pipérazinyl}-propyl]-α-hydroxy-α-phényl-benzèneacétamide, 0,75 fumarate :

Le composé 56 est préparé d'une manière analogue à celle de l'exemple 1 sauf que la (±)-1-[2-(1-propényl)-phényl]-pipérazine est utilisée à la place de la 1-(3-méthylphényl)-pipérazine ; p.f. : 152-153° C.

RMN-¹H (400 MHz, DMSO d6, δ) : 8,37 (m, 1H, NH-CO); 7,40-6,99 (m, 14H, 3 x Ph) ; 6,60 (s, 1,5H, 0,75 x -CH=CH-) ; 3,20 (m, 2H, CH₂-NHCO) ; 2,84 (m, 4H, pipérazine) ; 2,59 (m, 6H, pipérazine + Ph-CH₂) ; 2,44 (m, 2H, CH₂-N) ; 1,67 (m, 2H, CH₂) ; 1,60 (m, 2H, CH₂-CH₃) ; 0,92 (t, 3H, CH₃, J = 7,46 Hz).

Analyse élémentaire pour C₃₀H₃₇N₃O₂, 0,75 fumarate : % Cale: C, 70,94 ; H, 7,22 ; N, 7,52; % obtenus : C, 70,71; H, 7,28 ; N, 7,07.

### Essai de liaison au récepteur α₁-adrénergique :

L'affinité de composés variés de l'invention pour les récepteur α₁-adrénergiques est déterminée en mesurant l'inhibition de la liaison de la [³H]prazosine (DuPont NEN, Les Ulis, France) au cortex cérébral de rat selon une méthode dérivée de celle de Morrow et Creese, Mol. Pharmacol. 29:321 (1986). Les cortex cérébraux de rats mâles Sprague Dawley sont homogénéisés à 4° C dans Tris-HCl 50 mM pH = 7,7 et centrifugés à 50 000 g pendant 30 minutes à 4° C. Les culots sont à nouveau mis en suspension dans le même tampon et centrifugés à nouveau à 50 000 g pendant 30 minutes à 4° C.

L'inhibition compétitive de la liaison de 0,2 nM [³H]prazosine est effectuée en triple avec des composés tests non étiquetés de l'invention dans des concentrations variant de 10⁻¹¹M à 10⁻²M. Des membranes de cortex cérébral de rat (5 mg (humide) / ml) sont incubées pendant 30 minutes à 25° C dans Tris-HCl 50 mM, pH = 7,7. La [³H]prazosine liée est séparée de la [³H]prazosine libre par filtration immédiate au travers de filtres en fibres de verre Whatman GF/B en utilisant des récupérateurs de cellules Brandel. Les filtres sont rincés trois fois avec le même tampon à 0-4° C et on teste leur radioactivité par spectrométrie liquide à scintillation.

Les liaisons spécifiques sont obtenues en soustrayant les liaisons non-spécifiques (déterminées en présence de 10 µM de phentolamine (Sigma Chemical Co., St. Louis MO)) des liaisons totales. Les données relatives aux liaisons sont analysées par analyse par régression non-linéaire itérative assistée par ordinateur, en utilisant le programme Ligand (Munson, P.J., Rodbard, D. Anal. Biochem. 107:220 (1980)).

L'IC₅₀ (concentration nécessaire pour inhiber 50 % des liaisons spécifiques) de chacun des composés tests est donnée en Tableau I.

**TABLEAU I**

| **AFFINITÉ DE LIAISON DU RÉCEPTEUR α**_{**1-**}**ADRÉNERGIQUE** | | | |
|---|---|---|---|
| **Composé No.** | **Ki (nM)** | **Composé No.** | **Ki (nM)** |
| 1 | 51 | 29 | 67 |
| 2 | 30 | 30 | 44 |
| 3 | 60 | 31 | 43 |
| 4 | 66 | 32 | 51 |
| 5 | 60 | 33 | 40 |
| 6 | 480 | 34 | 45 |
| 7 | 30 | 35 | 90 |
| 8 | 16 | 36 | 170 |
| 9 | 28 | 38 | 30 |
| 10 | 27 | 39 | 27 |
| 11 | 38 | 40 | 38 |
| 13 | 30 | 41 | 27 |
| 14 | 27 | 42 | 22 |
| 15 | 27 | 43 | 29 |
| 16 | 41 | 44 | 73 |
| 17 | 54 | 45 | 13 |
| 18 | 55 | 46 | 48 |
| 19 | 60 | 47 | 14 |
| 20 | 27 | 48 | 29 |
| 21 | 64 | 49 | 50 |
| 22 | 25 | 50 | 50 |
| 23 | 98 | 51 | 110 |
| 24 | 69 | 52 | 51 |
| 25 | 29 | 54 | 280 |
| 26 | 74 | 55 | 300 |
| 27 | 31 | 56 | 25 |
| 28 | 36 | | |

### Essai de contraction de l'urètre :

Les urètres sont extraites de lapins mâles New Zealand, et nettoyées des tissus connexes environnants. Des bandes d'urètres de la prostate (3 mm de large, 10-15 mm de long) sont suspendues (1 g de tension) dans des bains pour organes contenant 10 ml de solution physiologique (pH 7,4) à 37° C sous une atmosphère de 95 % O₂ / 5 % CO₂ ; NaCl (118 mM) ; KCl (4,7 mM) ; CaCl₂ (2,5 mM) ; KH₂PO₄ (1,2 mM) ; MgSO₄ (1,2 mM); NaHCO₃ (25 mM) ; glucose (11 mM). Des réponses contractiles sont mesurées en utilisant des transducteurs à déplacement de force ("force displacement transducers") couplés à un polygraphe Gould 8000S. Une période d'équilibration ou équilibrage d'une heure est prévue avant l'expérimentation. Après l'équilibrage, les préparations sont sensibilisées par une concentration submaximale de phényléphrine. Quarante cinq minutes après la sensibilisation, une courbe concentration-effet pour la phényléphrine est établie. Afin de déterminer l'effet des composés tests, différentes préparations provenant du même animal sont utilisées en parallèle. Une préparation sert de contrôle alors que les autres recoivent une concentration d'un composé test introduit dans le bain 30 minutes avant la phényléphrine (Auguet, M., et al., European J. Pharmacol. 217:153 (1995)).

Les constantes de dissociation (K_{b}) des composés tests sont déterminées en utilisant l'équation suivante : K_{b} = [B] / (rapport dosique - 1); dans laquelle [B] est la concentration du composé test et (rapport dosique) est l'EC₅₀ de la phényléphrine en présence du composé test divisée par l'EC₅₀ de la préparation de contrôle. Ces résultats sont ensuite exprimés comme le logarithme négatif du K_{b}. Les valeurs d'EC₅₀ sont calculées à partir de la réponse contractile maximum à la phényléphrine par analyse par ordinateur en utilisant une régression linéaire sur les valeurs de tension moyennes. La réponse contractile maximale de chaque préparation qui a été soumise au composé test est déterminée en comparant la réponse provoquée par l'application de la concentration sensibilisante de phényléphrine avec la réponse provoquée dans la préparation de contrôle (qui n'a pas reçu les composés tests).

Les résultats obtenus pour les composés test sont donnés en Tableau II.

**TABLEAU II**

| **INHIBITION DE LA CONTRACTION DE L'URÈTRE** | |
|---|---|
| **Composé N°** | **-log K**_{**b**} |
| 2 | 8 |
| 7 | 8,1 |
| 8 | 8,2 |
| 9 | 8,1 |
| 10 | 8,2 |
| 11 | 6,9 |
| 13 | 8,2 |
| 14 | 7,8 |
| 15 | 7,9 |
| 16 | 7,8 |
| 17 | 7,7 |
| 20 | 7,5 |
| 22 | 8,3 |
| 25 | 7,6 |
| 27 | 7,6 |
| 38 | 7,5 |
| 39 | 7,3 |
| 42 | 7,68 |
| 45 | 8,3 |
| 46 | 7,5 |
| 47 | 7,07 |
| 48 | 7,02 |
| 50 | 6,92 |

### Essai de contraction de la veine portale et de l'aorte de rat :

L'aorte et la veine portale sont extraites de rats mâles Sprague Dawley (275 - 350 g, Charles River, France) après dislocation cervicale et nettoyées des tissus connexes environnants. Des bandes longitudinales (une par animal) de la veine portale (3 mm de large, 10 - 15 mm de long) et des anneaux dénudés de l'endothélium (≡ 2 mm de large) de l'aorte sont suspendus (tension : veine portale = 0,5 g ; aorte = 2 g) dans des bains pour organes contenant 10 ml de solution physiologique (composée de NaCl (118 mM), KCl (4,7 mM), CaCl₂ (2,5 mM), KH₂PO₄ (1,2 mM), MgSO₄ (1,2 mM), NaHCO₃ (25 mM), glucose (11 mM)) à 37° C et gazée avec 95 % O₂ / 5 % CO₂. Des réponses contractiles sont mesurées en utilisant des transducteurs à déplacement de force soit couplés à un polygraphe Gould 8000S, soit connectés à un système de récupération de données (IOS, Dei Lierre, Mitry-Mory, France). Une période d'équilibrage ou équilibration d'une heure est prévue avant l'expérimentation. Après l'équilibration, les préparations sont sensibilisées avec une concentration submaximale de phényléphrine (1 µM pour l'aorte ; 3 µM pour la veine portale). Lorsque la contraction provoquée par la phényléphrine est stable, un défaut de relaxation après addition de carbachol (10 µM) indique une désorganisation réussie de l'endothélium de l'aorte. Soixante-quinze minutes après la sensibilisation, une courbe concentration-effet pour la phényléphrine est établie. Pour déterminer l'effet des composés tests, différentes préparations sont utilisées en parallèle. L'une sert de contrôle, alors que les autres reçoivent les diffférents composés tests introduits dans le bain 60 minutes avant l'administration de phényléphrine.

Les résultats sont exprimés comme des moyennes ± S.E.M. ou pourcentage de contraction. La réponse contractile maximum de chaque préparation qui a été soumise à un composé test est déterminée en comparant la réponse provoquée par l'application de la concentration sensibilisante de phényléphrine avec la réponse provoquée dans la préparation contrôle (qui n'a reçu que la phényléphrine). Les valeurs d'EC₅₀ sont calculées à partir de la réponse contractile maximum par analyse par ordinateur en utilisant une régression linéaire sur les valeurs de tension moyennes. Les constantes de dissociation (K_{b}) des composés tests sont déterminées pour chaque composé test selon l'équation suivante : K_{b} = [B] / (rapport dosique - 1) ; dans laquelle [B] est la concentration du composé test et (rapport dosique) est l'EC₅₀ de l'agoniste en présence du composé test divisée par l'EC₅₀ contrôle. Le tableau III donne le K_{b} du composé 45 et de l'α₁-antagoniste, la Prazosine (Sigma, Clery en Vescin, France).

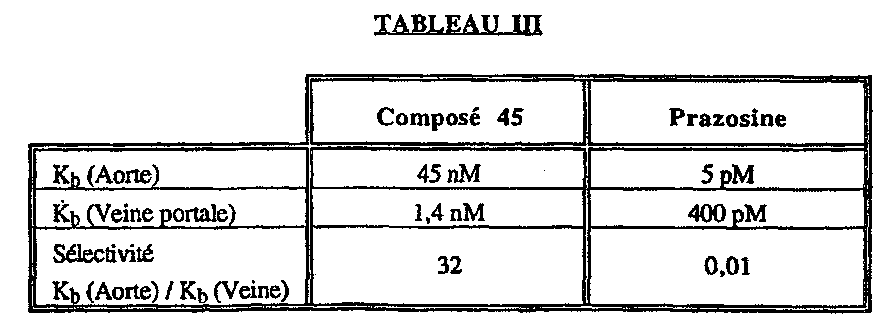

### Essai de contraction de l'aorte et de la veine portale du lapin :

L'aorte et la veine portale sont extraites de lapins mâles New Zealand (2,5 - 3,5 kg, Dombes Romans, France) après dislocation cervicale, et nettoyées des tissus connexes environnants.

Des bandes longitudinales (4 par animal - 3 mm de largeur, 10 à 15 mm de long) ou des anneaux dénudés de l'endothélium (1 mm de large) de l'aorte sont suspendus (tension : veine portale = 0,5 g ; aorte = 2 g) dans des bains pour organes contenant 10 ml de solution physiologique (composée de NaCl (118 mM), KCl (4,7 mM), CaCl₂ (2,5 mM), KH₂PO₄ (1,2 mM), MgSO₄ (1,2 mM), NaHCO₃ (25 mM), glucose (11 mM)) à 37° C et gazés avec 95 % O₂ / 5 % CO₂. Des réponses contractiles sont mesurées en utilisant des transducteurs à déplacement de force couplés à un polygraphe Gould RS3400. Une période d'équilibration ou équilibrage de une heure est prévue avant expérimentation. Après équilibrage, les préparations sont sensibilisées avec une concentration submaximale de phényléphrine (1 µM pour la veine portale ; 3 µM pour l'aorte). Lorsque la contraction provoquée par la phényléphrine est stabilisée, du carbachol (10 µM) est administré dans le but de contrôler la désorganisation de l'endothélium dans les vaisseaux. Soixante-quinze minutes après la sensibilisation, une courbe concentration-effet pour la phényléphrine est établie. Afin de déterminer l'effet des composés tests, différentes préparations sont utilisées en parallèle. L'une sert de contrôle, alors que les autres recoivent différentes concentrations de composé test introduit dans le bain 60 minutes avant la phényléphrine.

Les résultats sont exprimés comme des moyennes ± S.E.M. ou pourcentage de la contraction. La réponse contractile maximale de chaque préparation ayant été soumise à un composé test est déterminée en comparant la réponse déclenchée par l'application de la concentration sensibilisante de phényléphrine avec la réponse déclenchée dans la préparation contrôle (qui n'a reçu que de la phényléphrine). Les valeurs d'EC₅₀ sont calculées à partir de la réponse contractile maximum par analyse par ordinateur en utilisant une régression linéaire sur les valeurs de tension moyennes. Les constantes de dissociation des composés tests (K_{b}) sont déterminées pour chaque antagoniste selon l'équation suivante : K_{b} = [B] / (rapport dosique - 1) ; dans laquelle [B] est la concentration du composé test et (rapport dosique) est l'EC₅₀ de l'agoniste en présence du composé test divisée par l'EC₅₀ contrôle. Le tableau IV donne le K_{b} du composé 45 et de la Prazosine.

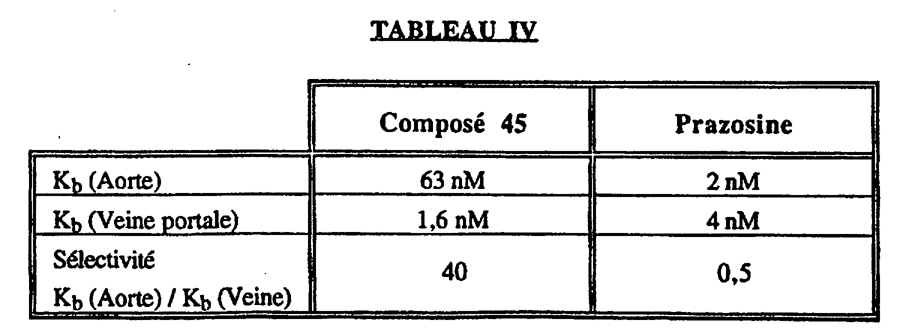

### Essai de contraction de la veine saphène de lapin :

La veine saphène caudale est extraite de lapins mâles New Zealand (2,5 à 3 kg, bombes Romans, France) après dislocation cervicale et nettoyée des tissus connexes environnants. Des anneaux de 2-3 mm de largeur sont découpés sous une lentille de dissection. Les anneaux sont suspendus sous une tension de 1 g à 37° C dans des bains pour organes contenant 10 ml de solution physiologique (composée de NaCl (118 mM), KCl (4,7 mM), CaCl₂ (2,5 mM), KH₂PO₄ (1,2 mM), MgSO₄ (1,2 mM), NaHCO₃ (25 mM), glucose (11 mM)) à 37° C et gazés avec 95 % O₂ / 5 % CO₂. Les réponses contractiles sont mesurées en utilisant des transducteurs à déplacement de force couplés à un polygraphe Gould RS3400. L'endothélium est enlevé en faisant rouler doucement de petits forceps sur la surface luminale des anneaux artériels et en perfusant un flux doux de carbogène pendant 5 minutes au travers de la veine. Une période d'équilibration ou équilibrage d'une heure est prévue avant l'expérimentation. Pendant cette période d'équilibrage, on laisse la tension des anneaux veineux se relâcher et la tension finale est maintenue à 0,5 g. Après équilibrage, les préparations sont sensibilisées par une concentration submaximale (3 µM) de phényléphrine. Lorsque la contraction déclenchée par la phényléphrine est stabilisée, du carbachol (10 µM) est administré afin de contrôler la désorganisation de l'endothélium dans les vaisseaux. Soixante-quinze minutes après la sensibilisation, une courbe concentration-effet pour la phényléphrine est établie. Afin de déterminer l'effet des composés tests, différentes préparations sont utilisées en parallèle. L'une sert de contrôle alors que les autres reçoivent les différents composés tests introduits dans le bain 60 minutes avant l'administration de phényléphrine.

Les résultats sont exprimés comme des moyennes ± S.E.M. ou pourcentage de la contraction. La réponse contractile maximale de chaque préparation ayant été soumise au composé test est déterminée en comparant la réponse déclenchée par l'application de la concentration sensibilisante de phényléphrine avec la réponse déclenchée dans la préparation contrôle (qui n'a reçu que la phényléphrine). Les valeurs d'EC₅₀ sont calculées à partir de la réponse contractile maximum par analyse par ordinateur en utilisant une régression linéaire sur les valeurs de tension moyennes. Les constantes de dissociation du composé test (K_{b}) sont déterminés pour chaque composé test selon l'éguation suivante : K_{b} = [B] / (rapport dosique - 1) ; dans laquelle [B] est la concentration du composé test et (rapport dosique) est l'EC₅₀ de l'agoniste en présence du composé test divisée par l'EC₅₀ contrôle. Le tableau V donne le K_{b} du composé 45 et de la Prazosine.

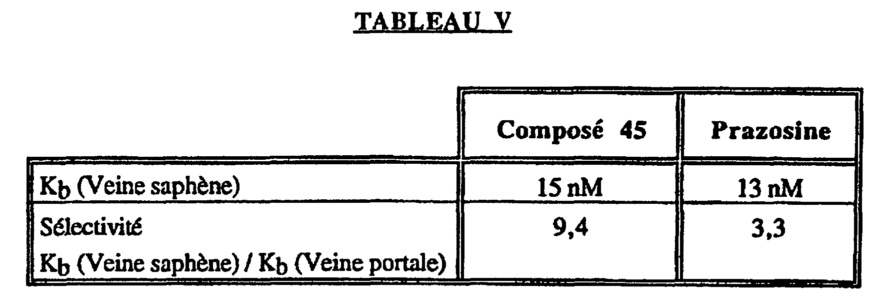

## Revendications

1. Composé répondant
soit à la formule I: 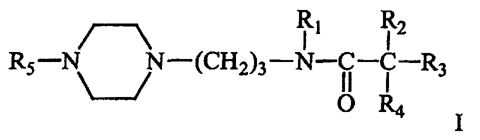 dans laquelle :
R₁ représente un hydrogène ou un alkyle inférieur ;
R₂ représente un hydrogène, un alkyle inférieur, OR₆, S(O)ₘR₆ dans lequel m vaut 0, 1 ou 2, NHS(O)ₙR₆ dans lequel n vaut 1 ou 2, OC(O)R₆, C(O)NR₆R₇, NR₆R₇ ou N(R₇)C(O)R₆ ;
chacun des R₃ et R₄, de façon indépendante l'un de l'autre, représente l'un des radicaux suivants substitués une à quatre fois ou non-substitués : alkyle inférieur, cyclohexyle, phényle, napthyle, benzyle dans leque ledit substituant est un alkyle inférieur, un halogène, OR₆, SR₆, NR₆R₇, CN, NO₂, CF₃ ou phényle, ou R₃ et R_{4,} ensemble avec l'atome de carbone auquel ils sont conjointement liés, forment le radical 9-xanthényle ou 9-fluorényle ;
R₅ représente un phényle substitué une à quatre fois dans lequel ledit substituant est un alkyle inférieur, CN, un halogène, un hydroxy-alkyle inférieur, cyclopropylhydroxyméthyle, un alkoxy inférieur-alkyle inférieur, ou alkoxy inférieur ; et
chacun des R₆ et R₇, de façon indépendante l'un de l'autre, représente un hydrogène ou l'un des radicaux suivants substitués une à quatre fois ou non-substitués : un alkyle inférieur, phényle, benzyle dans lequel ledit substituant est un alkyle inférieur, un halogène, ou un alcoxy inférieur ; ou un de ses sels pharmaceutiquement acceptables,
soit à l'une des formules suivantes:
α-hydroxy-N-[3-{4-(2-méthoxyphényl)-1-pipérazinyl}-propyl]-benzèneacétamide ;
α-amino-N-[3-{4-(2,5-diméthylphényl}-1-pipérazinyl}-propyl]-benzèneacétamide ;
N-[3-(4-(2,5-diméthylphényl)-1-pipérazinyl)-propyl]-α-(méthylsulfonylamino)-benzéneacétamide ;
N-[3-{4-(2,5-diméthylphényl)-1-pipérazinyl}-propyl]-α-benzylaminocarbonyl-benzèneacétamide;
N-[3-{4-(2,5-diméthylphényl)-1-pipérazinyl}-propyl]-α-(4-méthylphényl)-sulfonyl amino-benzèneacétamide;
N-[3-{4-(2,5-diméthylphényl}-1-pipérazinyl}-propyl]-α-benzylcarbonylamino-benzèneacétamide;
étant entendu que le terme "inférieur", lorsqu'il se réfère au radical alkyl ou alkoxy, signifie au plus 6 atomes de carbone.

2. Composé selon la revendication 1, dans lequel R₂ représente un hydrogène, un alkyle inférieur, OR₆ dans lequel R₆ représente un hydrogène ou benzyle, SR₆ dans lequel R₆ représente un alkyle inférieur, NH₂, NHSO₂R₆ dans lequel R₆ représente un alkyle inférieur ou benzyle, OC(O)R₆ dans lequel R₆ représente benzyle, C(O)NR₆R₇ ou N(R₇)C(O)R₆ dans lequel R₇ représente un hydrogène et R₆ représente benzyle ; ou un de ses sels pharmaceutiquement acceptables.

3. Composé selon la revendication 2, dans lequel chacun des R₃ et R₄, de façon indépendante l'un de l'autre, représente l'un des radicaux suivants substitués ou non substitués : un alkyle inférieur, cyclohexyle, phényle, naphtyle, dans lequel ledit substituant est un halogène, OR₆ dans lequel R₆ est un alkyle inférieur, ou phényle ; ou un de ses sels acceptables pharmaceutiquement.

4. Composé selon la revendication 3, dans lequel R₁ représente un hydrogène et R₅ représente le 2,5-diméthyl-phényle ; ou un de ses sels acceptables pharmaceutiquement.

5. Composé selon la revendication 4, dans lequel R₂ représente OR₆ dans lequel R₆ représente un hydrogène, un alkyle inférieur, ou benzyle ; ou un de ses sels acceptables pharmaceutiquement.

6. Composé selon la revendication 4, dans lequel R₃ représente un alkyle inférieur et R₄ représente un phényle ; ou un de ses sels acceptables pharmaceutiquement.

7. Composé selon la revendication 2, dans lequel R₃ et R_{4,} ensemble avec l'atome de carbone auquel ils sont conjointement liés, forment le radical 9-xanthényle ou 9-fluorényle; ou un de ses sels acceptables pharmaceutiquement.

8. Composé selon la revendication 7, dans lequel R₁ représente l'hydrogène et R₅ représente le 2-méthoxy-phényle ou le 2,5-diméthyl-phényle ; ou un de ses sels acceptables pharmaceutiquement.

9. Composé selon la revendication 8, dans lequel R₂ représente OR₆ dans lequel R₆ représente l'hydrogène, un alkyle inférieur ou benzyle; ou un de ses sels acceptables pharmaceutiquement.

10. Composé selon la revendication 1, dans lequel ledit composé répond à la formule : 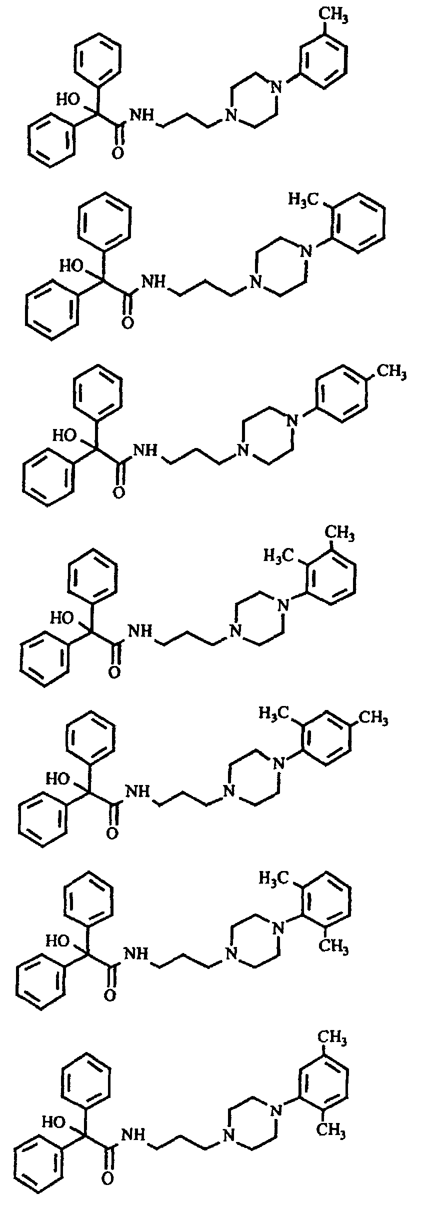 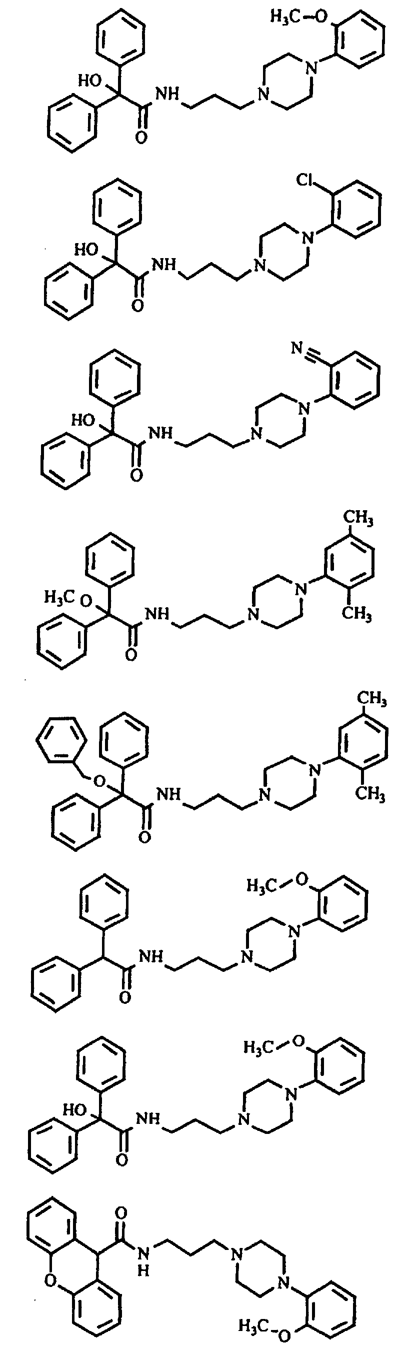 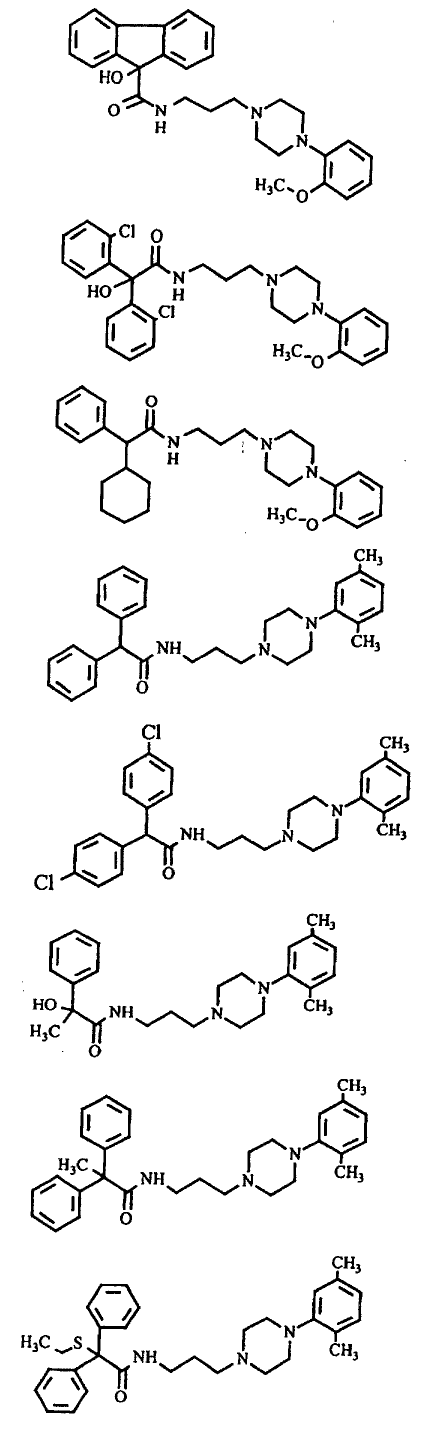 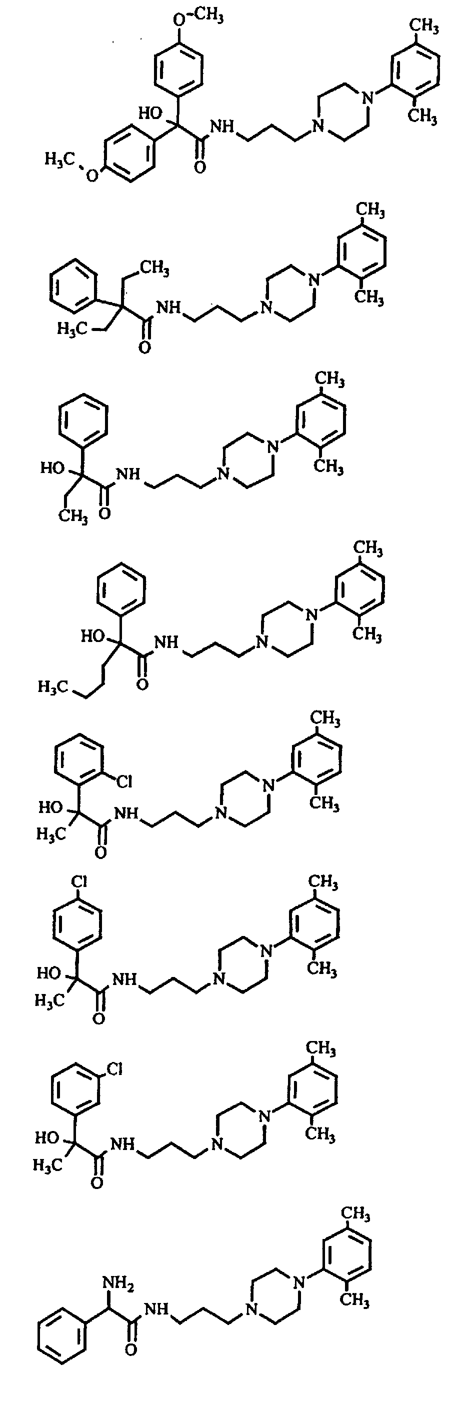 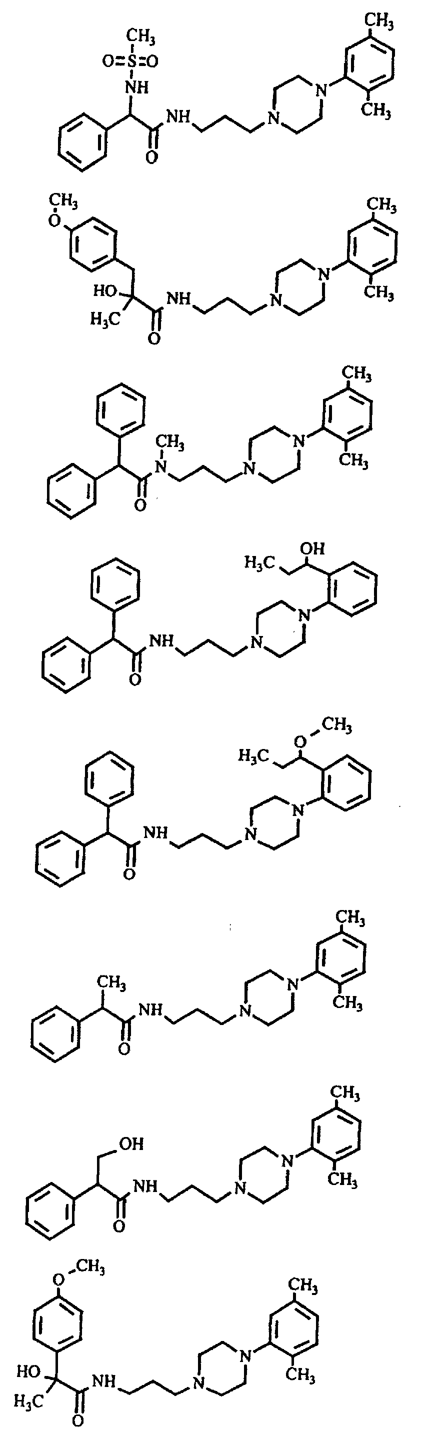 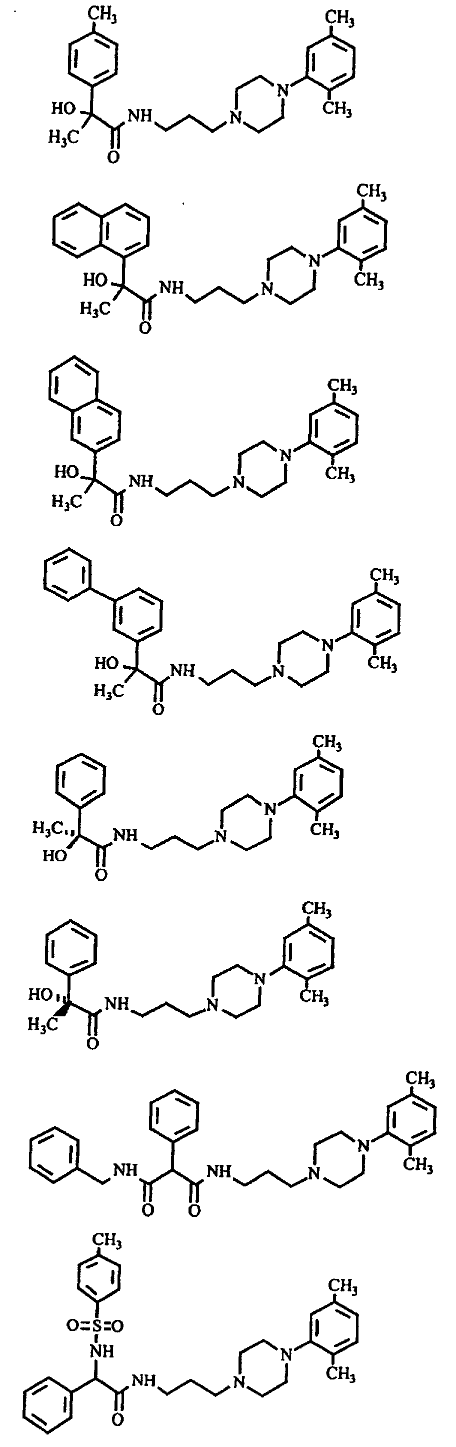 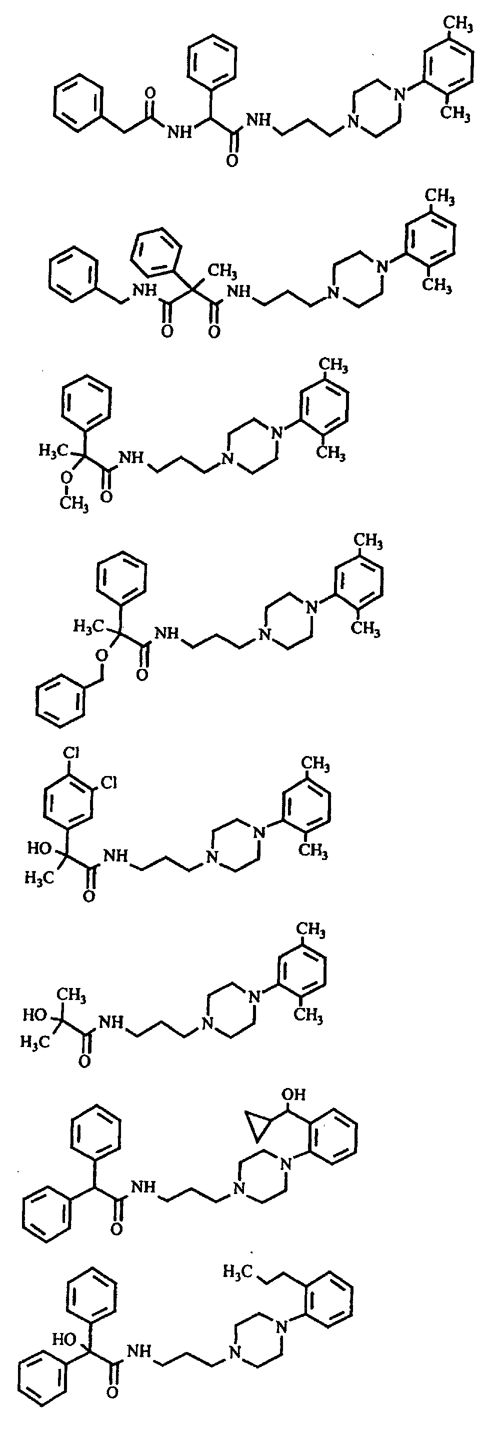 ou un de ses sels acceptables pharmaceutiquement.

11. Composé selon la revendication 1, dans lequel ledit composé répond à la formule : 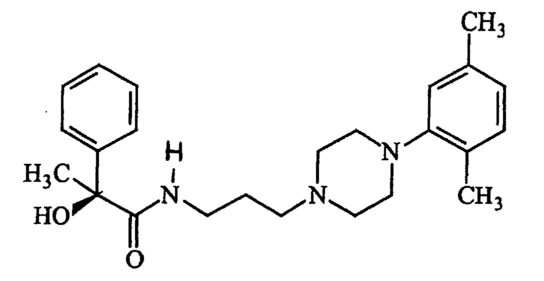 ou un de ses sels acceptables pharmaceutiquement.

12. Composition pharmaceutique comprenant un composé selon l'une des revendications précédentes.

13. Composition pharmaceutique selon la revendication 12 comprenant un composé selon la revendication 4.

14. Composition pharmaceutique selon la revendication 13 comprenant un composé selon les revendications 5 et 6.

15. Composition pharmaceutique selon la revendication 14 comprenant un composé selon la revendication 11.

16. Utilisation d'un composé selon l'une des revendications 1 à 11, pour la préparation d'un médicament destiné à traiter l'hypertension portale.

17. Utilisation d'un composé selon l'une des revendications 1 à 11, pour la préparation d'un médicament destiné à traiter la cirrhose.

## Claims

1. Compound corresponding
**either** to the formula I 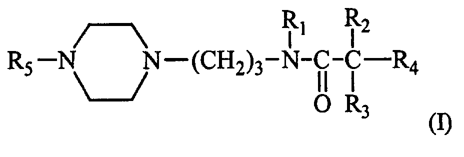 in which :
R₁ represents a hydrogen or a lower alkyl ;
R₂ represents a hydrogen, a lower alkyl, OR₆, S(O)ₘR₆ in which m is 0, 1, or 2, NHS(O)ₙR₆ in which n is 1 or 2, OC(O)R₆, C(O)NR₆R₇, NR₆R₇ or N(R₇)C(O)R₆;
each of R₃ and R₄, independent of each other, represent one of the following substituted one to four times or non-substituted radicals : lower alkyl, cyclohexyl, phenyl, naphthyl, benzyl, in which said substituent is a lower alkyl, a halogen, OR₆, SR₆, NR₆R₇, CN, NO₂, CF₃ or phenyl, or R₃ and R₄ form, together with the carbon atom to which they are jointly linked, 9-xanthenyl or 9-fluorenyl ;
R₅ represents a phenyl substituted one to four times, in which said substituent is a lower alkyl, CN, a halogen, a hydroxy- lower alkyl, cyclopropylhydroxymethyl, a lower alkoxylower alkyl or lower alkoxy ; and
each of R₆ and R₇, independent of each other, represent a hydrogen or one of the following radicals non-substituted or substituted one to four times : a lower alkyl, phenyl or benzyl, in which said substituent is a lower alkyl, a halogen or a lower alkoxy ; or one of the pharmaceutically acceptable salts thereof,
**or** to one of the following formulae :
α-hydroxy-N-[3-{4-(2-methoxyphenyl)-1-piperazinyl}-propyl]-benzeneacetamide ;
α-amino-N-[3-{4-(2,5-dimethylphenyl)-1-piperazinyl}-propyl]-benzeneacetamide ;
N-[3-{4-(2,5-dimethylphenyl)-1-piperazinyl}-propyl]-α-(methylsulfonylamino)-benzèneacétamide ;
N-[3-{4-(2,5-dimethylphenyl)-1-piperazinyl}-propyl]-α-benzylaminocarbonyl-benzeneacetamide ;
N-[3-{4-(2,5-dimethylphenyl)-1-piperazinyl}-propyl]-α-(4-méthylphenyl)-sulfonyl amino-benzeneacetamide ;
N-[3-{4-(2,5-dimethylphenyl)-1-piperazinyl}-propyl]-α-benzylcarbonylamino-benzeneacetamide ;
it being understood that the term "lower" when it refers to an alkyl or an alkoxy radical, means no more than 6 carbon atoms.

2. Compound according to claim 1, in which R₂ represents a hydrogen, a lower alkyl, OR₆ in which R₆ represents a hydrogen or benzyl, SR₆ in which R₆ represents a lower alkyl, NH₂, NHSO₂R₆ in which R₆ represents a lower alkyl or benzyl, OC(O)R₆ in which R₆ represents benzyl, C(O)NR₆R₇ or N(R₇)C(O)R₆ in which R₇ represents a hydrogen and R₆ represents benzyl ; or one of the pharmaceutically acceptable salts thereof.

3. Compound according to claim 2, in which each of R₃ and R₄, independent of each other, represents one of the following substituted or non substituted radicals : lower alkyl, cyclohexyl, phenyl, naphtyl, in which said substituent is a halogen, OR₆ in which R₆ is a lower alkyl, or phenyl ; or one of the pharmaceutically acceptable salts thereof.

4. Compound according to claim 3, in which R₁ represents a hydrogen and R₅ represents 2,5-dimethyl-phenyl; or one of the pharmaceutically acceptable salts therof.

5. Compound according to claim 4, in which R₂ represents OR₆ in which R₆ represents a hydrogen, a lower alkyl, or benzyl ; or one of the pharmaceutically acceptable salts therof.

6. Compound according to claim 4, in which R₃ represents a lower alkyl and R₄ represents phenyl ; or one of the pharmaceutically acceptable salts therof.

7. Compound according to claim 2, in which R₃ and R₄, together with the carbon atom to which they are jointly linked, form 9-xanthenyl or 9-fluorenyl ; or one of the pharmaceutically acceptable salts thereof.

8. Compound according to claim 7, in which R₁ represents hydrogen and R₅ represents 2-methoxy-phenyl or 2,5-dimethyl-phenyl; or one of the pharmaceutically acceptable salts thereof.

9. Compound according to claim 8, in which R₂ represents OR₆ in which R₆ represents hydrogen, a lower alkyl or benzyl ; or one of the pharmaceutically acceptable salts thereof.

10. Compound according to claim 1, in which said compound corresponds to the formula: 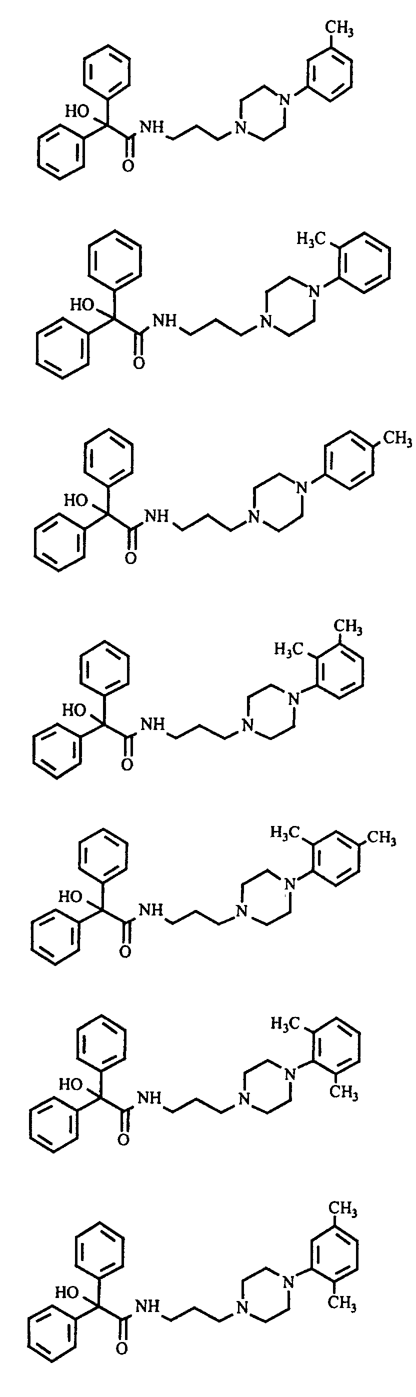 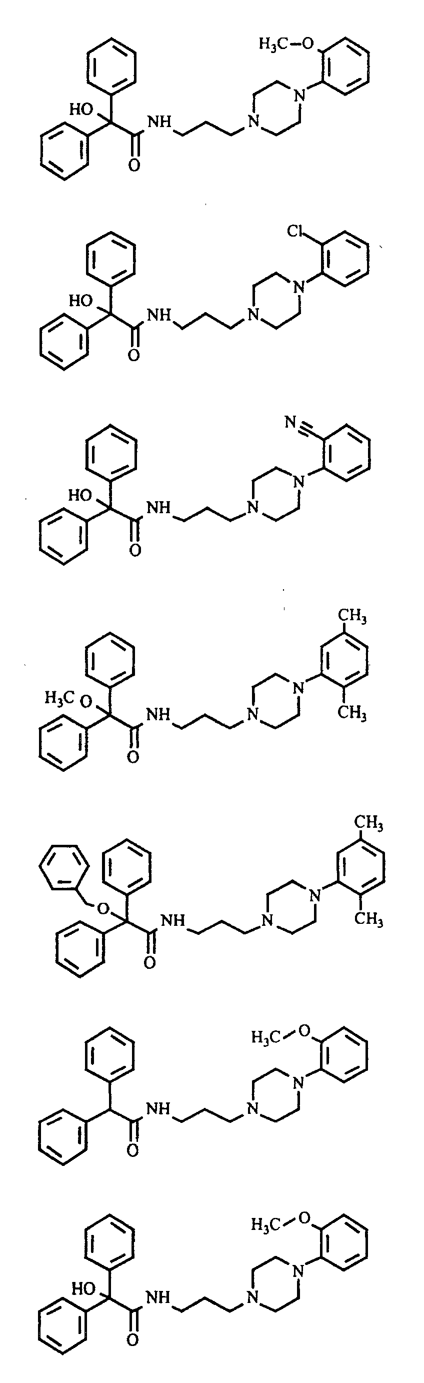 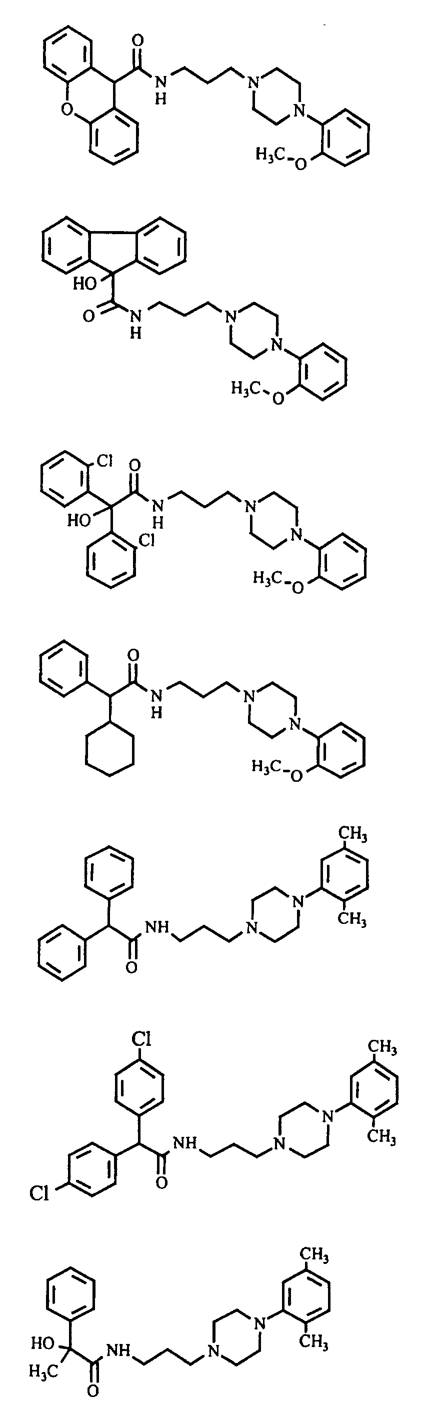 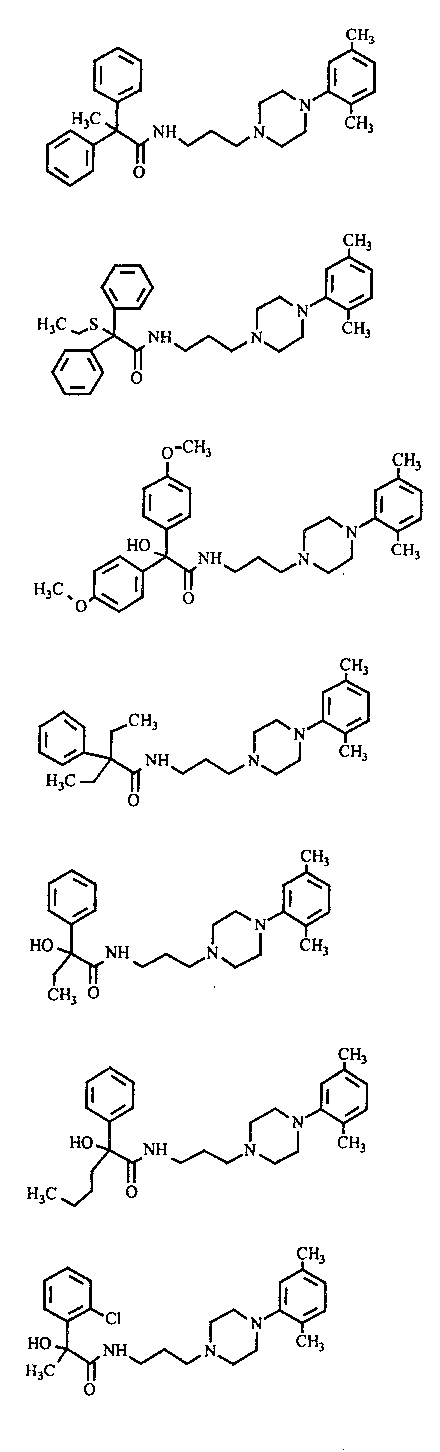 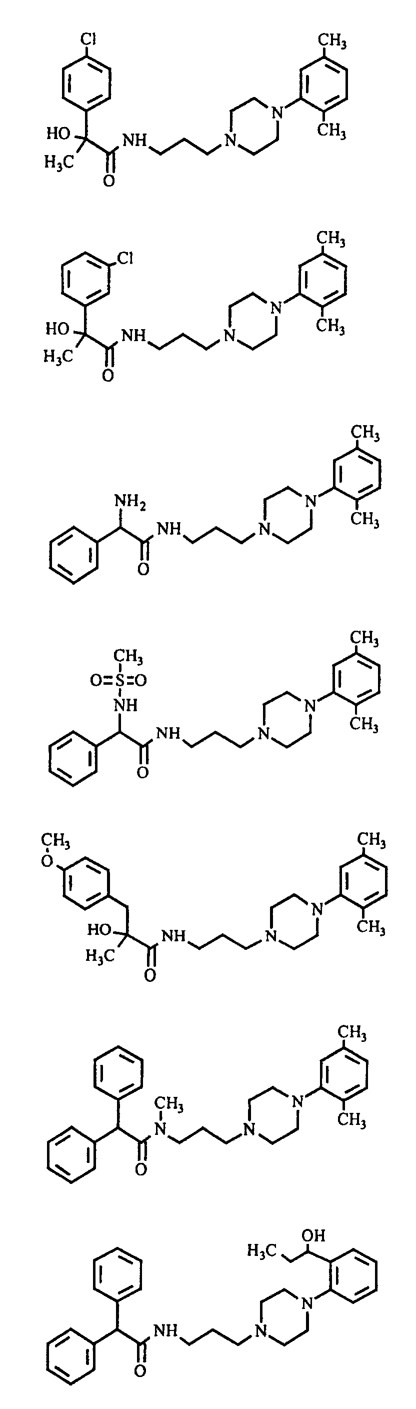 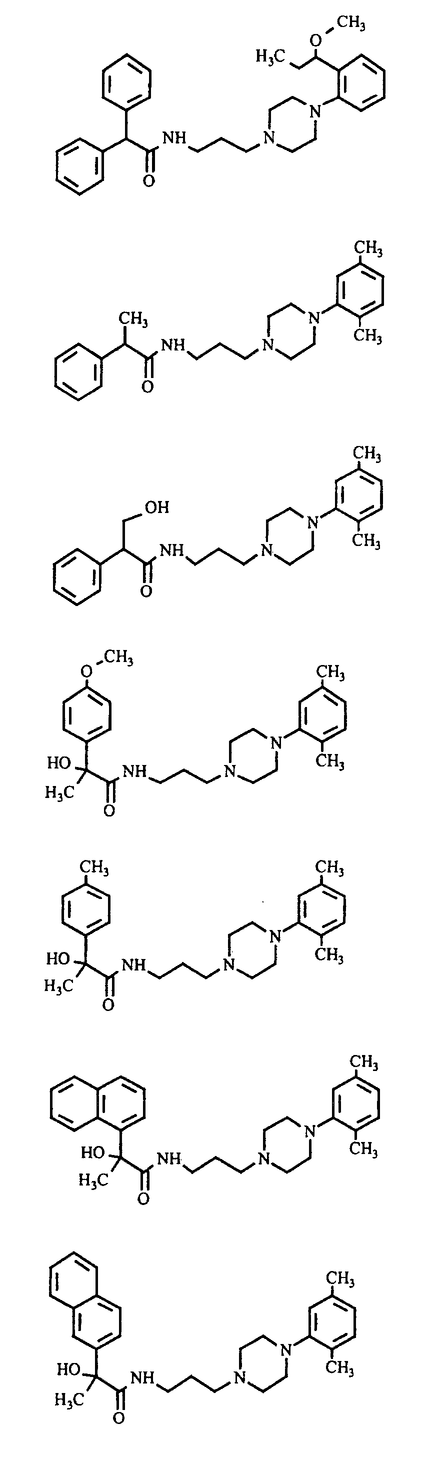 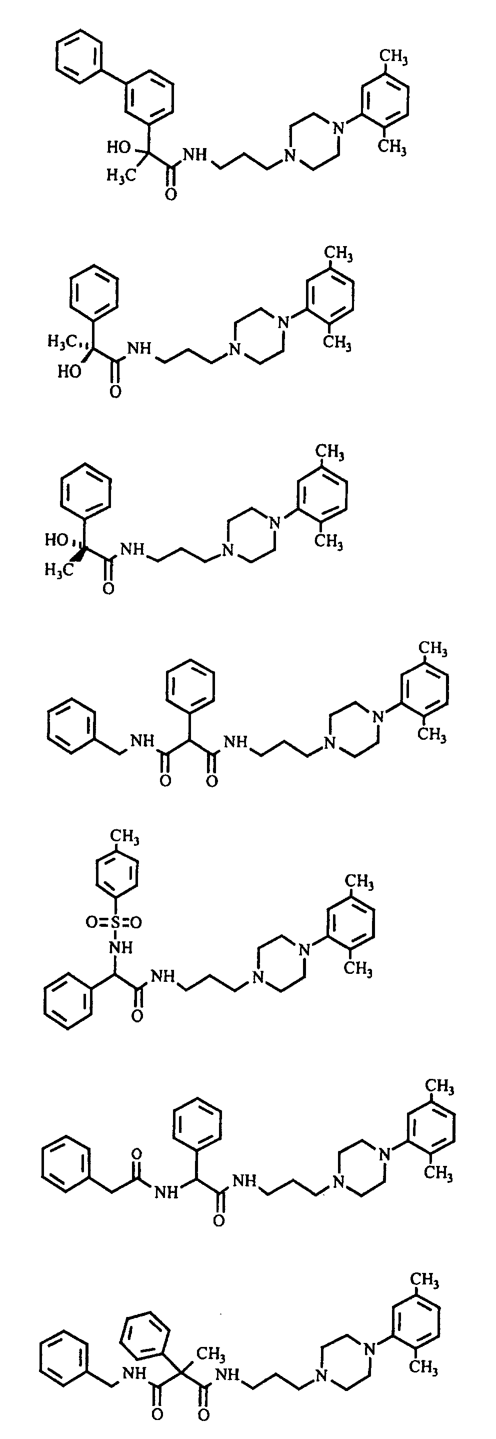 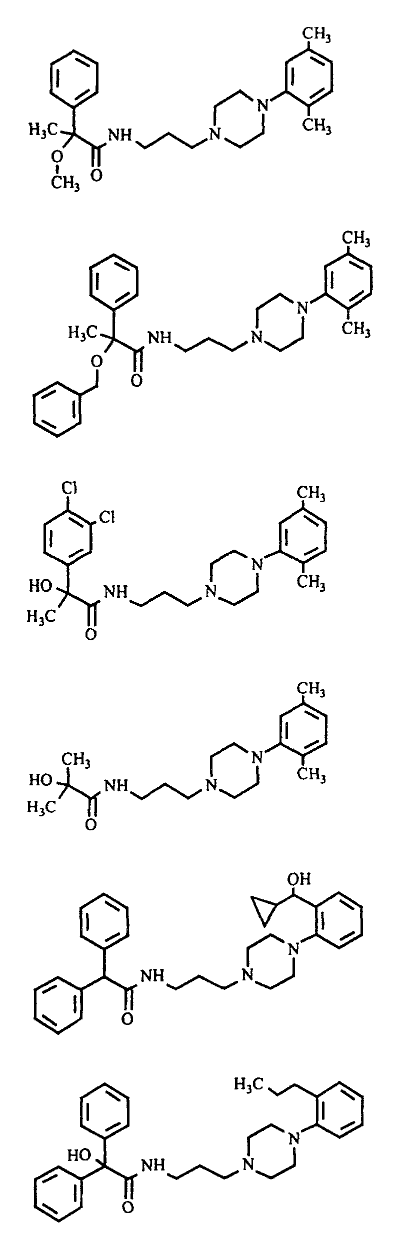 or one of the pharmaceutically acceptable salts thereof.

11. Compound according to claim 1, in which said compound corresponds to the formula: 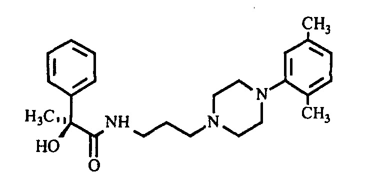 or one of the pharmaceutically acceptable salts thereof.

12. Pharmaceutical composition containing a compound according to any of the preceding claims.

13. Pharmaceutical composition according to claim 12, containing a compound according to claim 4.

14. Pharmaceutical composition according to claim 13, containing a compound according to claims 5 and 6.

15. Pharmaceutical composition according to claim 14, containing a compound according to claim 11.

16. Use of a compound according to one of claims 1 to 11, for the preparation of a medicament intended for the treatment of portal hypertension.

17. Use of a compound according to one claims 1 to 11, for the preparation of a medicament intended for the treatment of cirrhosis.

## Patentansprüche

1. Verbindung, die entweder der Formel I 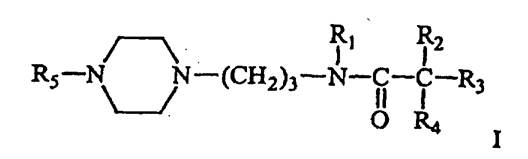 entspricht, in der:
R₁ Wasserstoff oder ein Niederalkyl darstellt ;
R₂ Wasserstoff, ein Niederalkyl, OR₆, S(O)ₘR₆, in dem m 0, 1 oder 2 bedeutet, NHS(O)ₙR₆, in dem n 1 oder 2 bedeutet,
OC (O) R₆, C(O)NR₆R₇, NR₆R₇ oder N(R₇)C(O)R₆ darstellt;
R₃ und R₄ unabhängig voneinander jeweils einen der folgenden ein- bis viermal substituierten oder nichtsubstituierten Reste darstellen: Niederalkyl, Cyclohexyl, Phenyl, Naphtyl, Benzyl, in dem dieser Substituent ein Niederalkyl, ein Halogen, OR₆, SR₆, NR₆R₇, CN, NO₂, CF₃ oder Phenyl ist, oder R₃ und R₄ zusammen mit dem Kohlenstoffatom, an das sie gemeinsam gebunden sind, den Rest 9-Xanthenyl oder 9-Fluorenyl bilden;
R₅ ein ein- bis viermal substituiertes Phenyl darstellt, in dem der Substituent ein Niederalkyl, CN, ein Halogen, ein Hydroxyniederalkyl, Cyclopropylhydroxymethyl, ein Niederalkoxyniederalkyl oder Niederalkoxy ist; und
R₆ und R₇ unabhängig voneinander jeweils Wasserstoff oder einen der folgenden ein- bis viermal substituierten oder nichtsubstituierten Reste darstellen: ein Niederalkyl, Phenyl, Benzyl, in dem dieser Substituent ein Niederalkyl, ein Halogen oder ein Niederalkoxy ist; oder eines ihrer pharmazeutisch verträglichen Salze,
oder die einer der folgenden Formeln entspricht:
α-Hydroxy-N-[3-{4-(2-methoxyphenyl)-1-piperazinyl}propyl] benzolacetamid;
α-Amino-N-[3-{4-(2,5-dimethylphenyl)-1-piperazinyl}propyl) benzolacetamid;
N-[3-{4-(2,5-dimethylphenyl)-1-piperazinyl}propyl]-α-(methylsulfonylamino)benzolacetamid:
N-[3-{4-(2,5-dimethylphenyl)-1-piperazinyl}propyl]-α-benzylaminocarbonylbenzolacetamid;
N-[3-{4-(2,5-dimethylphenyl)-1-piperazinyl}propyl]-α-(4-methylphenyl)sulfonylaminobenzolacetamid;
N-[3-{4-(2,5-dimethylphenyl)-1-piperazinyl}propyl]-α-benzylcarbonylaminobenzolacetamid;
wobei der Begriff "nieder", wenn er sich auf einen Alkyloder Alkoxyrest bezieht, höchstens 6 Kohlenstoffatome bedeutet.

2. Verbindung nach Anspruch 1, in der R₂ Wasserstoff, ein Niederalkyl, OR₆, in dem R₆ Wasserstoff oder Benzyl darstellt, SR₆, in dem R₆ ein Niederalkyl darstellt, NH₂, NHSO₂R₆, in dem R₆ ein Niederalkyl oder Benzyl darstellt, OC(O)R₆, in dem R₆ Benzyl darstellt, C(O)NR₆R₇ oder N(R₇)C(O)R₆ darstellt, in dem R₇ Wasserstoff darstellt und R₆ Benzyl darstellt; oder eines ihrer pharmazeutisch verträglichen Salze.

3. Verbindung nach Anspruch 2, in der R₃ und R₄ unabhängig voneinander jeweils einen der folgenden substituierten oder nichtsubstituierten Reste darstellen: ein Niederalkyl, Cyclohexyl, Phenyl, Naphtyl, in dem dieser Substituent ein Halogen, OR₆, in dem R₆ ein Niederalkyl ist, oder Phenyl ist; oder eines ihrer pharmazeutisch verträglichen Salze.

4. Verbindung nach Anspruch 3, in der R₁ Wasserstoff und R₅ 2,5-Dimethylphenyl darstellen; oder eines ihrer pharmazeutisch verträglichen Salze.

5. Verbindung nach Anspruch 4, in der R₂ OR₆ darstellt, in dem R₆ Wasserstoff, ein Niederalkyl oder Benzyl darstellt; oder eines ihrer pharmazeutisch verträglichen Salze.

6. Verbindung nach Anspruch 4, in der R₃ ein Niederalkyl darstellt und R₄ ein Phenyl darstellt; oder eines ihrer pharmazeutisch verträglichen Salze.

7. Verbindung nach Anspruch 2, in der R₃ und R₄ zusammen mit dem Kohlenstoffatom, an das sie gemeinsam gebunden sind, den Rest 9-Xanthenyl oder 9-Fluorenyl bilden; oder eines ihrer pharmazeutisch verträglichen Salze.

8. Verbindung nach Anspruch 7, in der R₁ Wasserstoff darstellt und R₅ 2-Methoxyphenyl oder 2,5-Dimethylphenyl darstellt; oder eines ihrer pharmazeutisch verträglichen Salze.

9. Verbindung nach Anspruch 8, in der R₂ OR₆ darstellt, in dem R₆ Wasserstoff, ein Niederalkyl oder Benzyl darstellt; oder eines ihrer pharmazeutisch verträglichen Salze.

10. Verbindung nach Anspruch 1, wobei diese Verbindung der folgenden Formel entspricht: 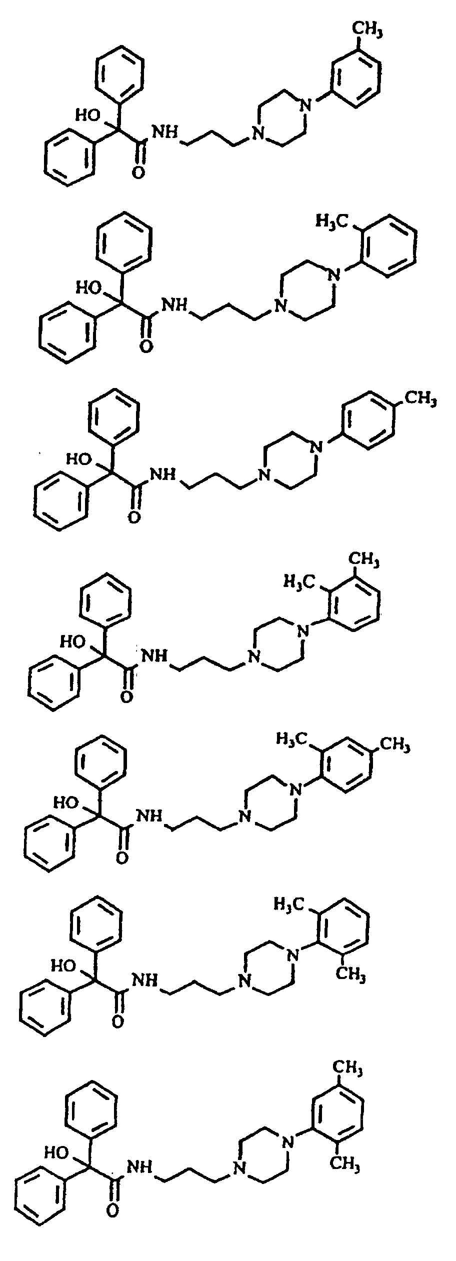 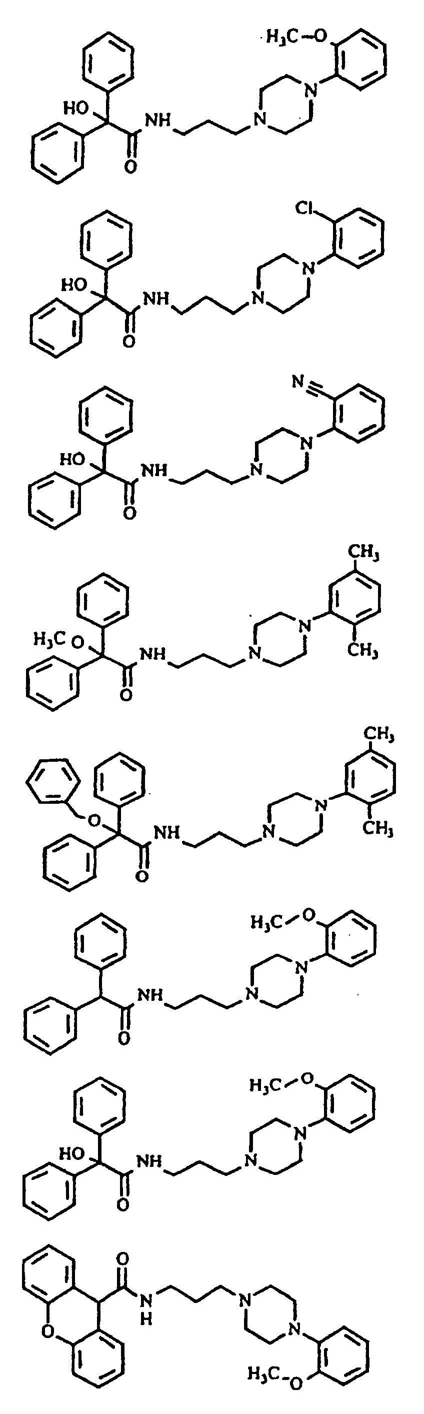 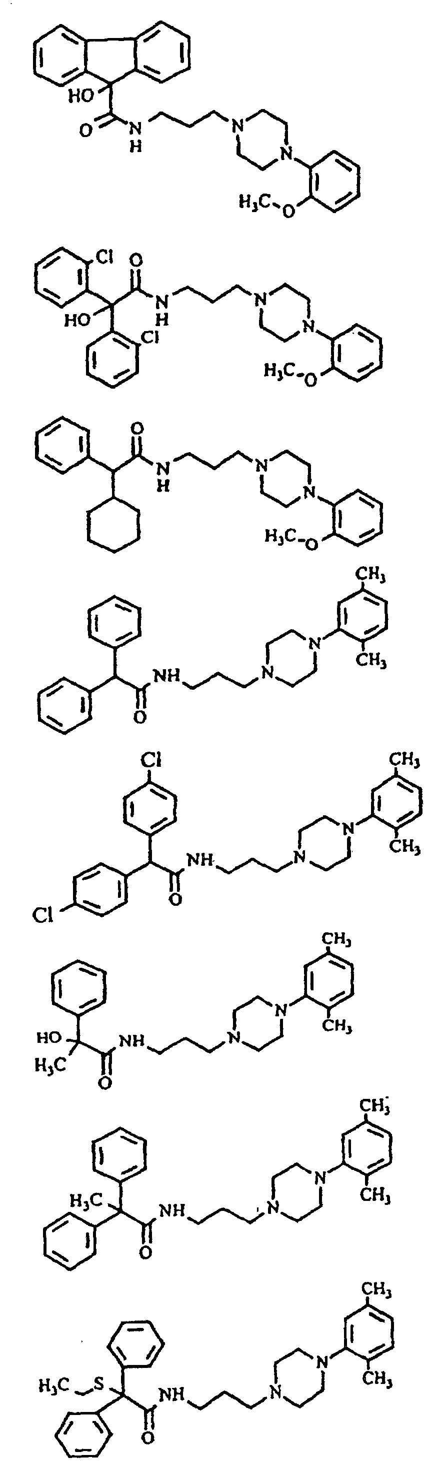 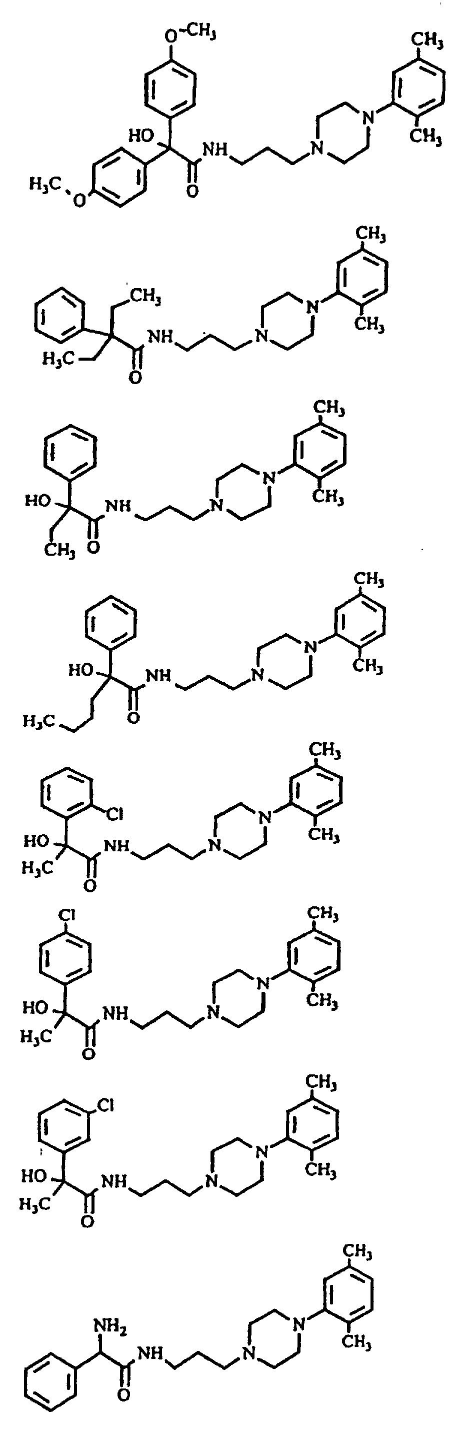 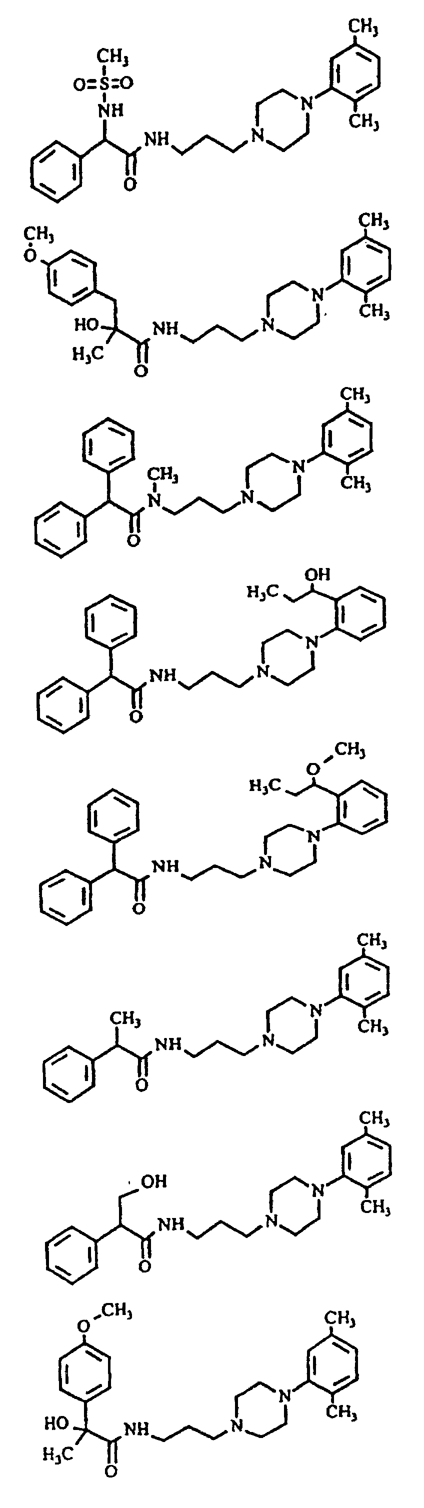 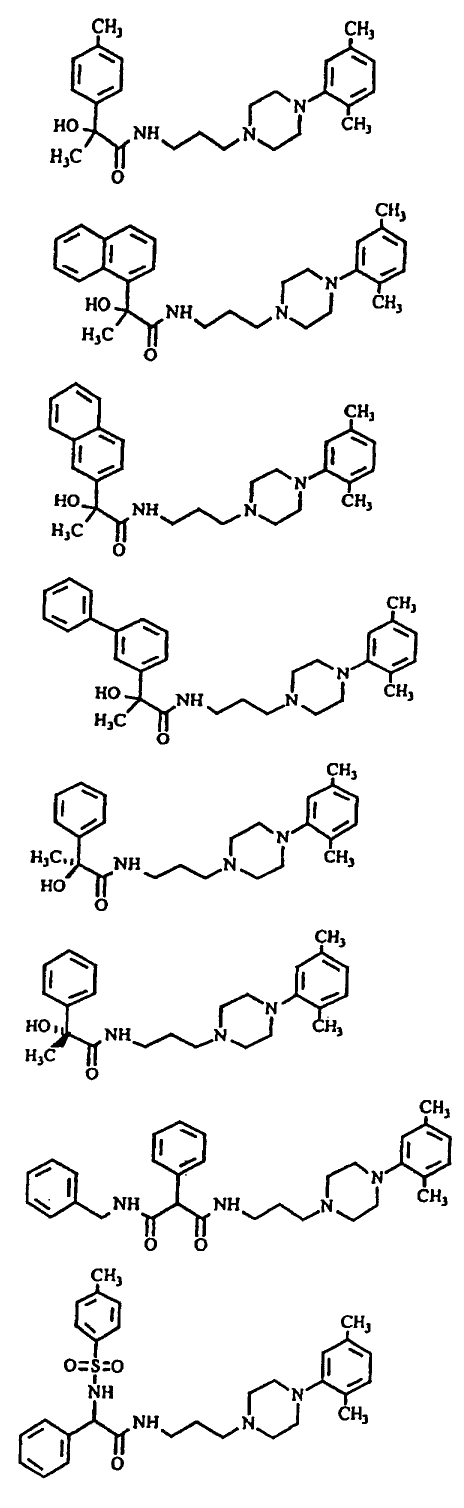 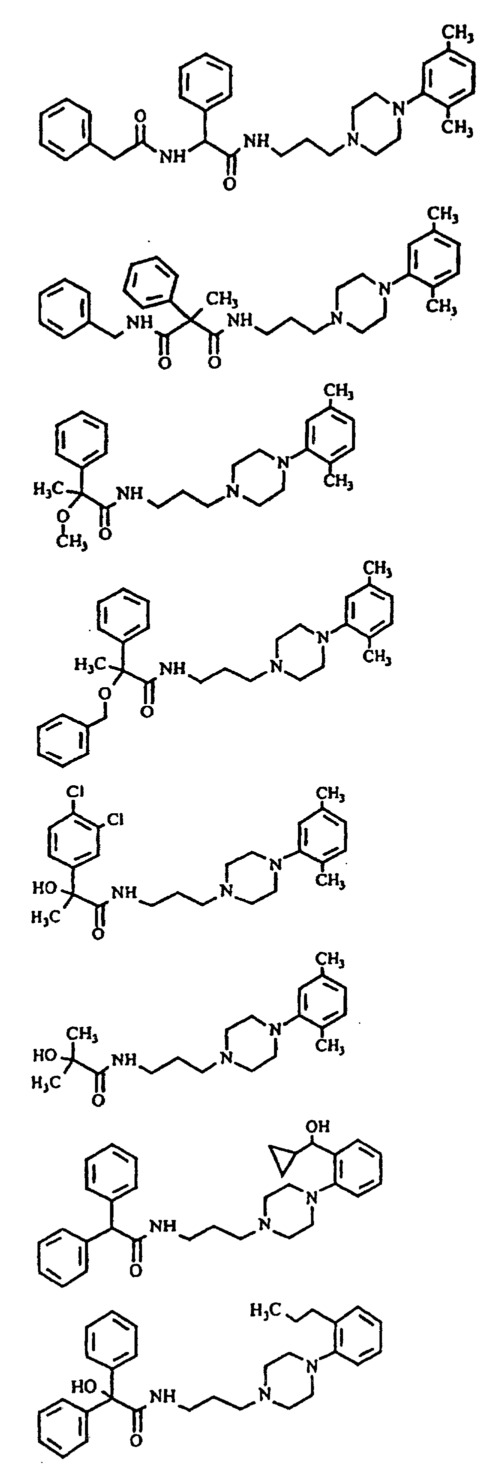 oder eines ihrer pharmazeutisch verträglichen Salze.

11. Verbindung nach Anspruch 1, wobei die Verbindung der folgenden Formel 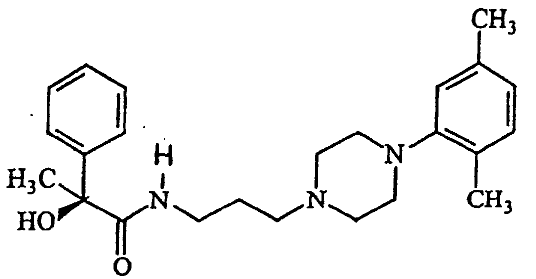 entspricht; oder eines ihrer pharmazeutisch verträglichen Salze.

12. Pharmazeutische Zusammensetzung, die eine Verbindung nach einem der vorhergehenden Ansprüche enthält.

13. Pharmazeutische Zusammensetzung nach Anspruch 12, die eine Verbindung nach Anspruch 4 enthält.

14. Pharmazeutische Zusammensetzung nach Anspruch 13, die eine Verbindung nach den Ansprüchen 5 und 6 enthält.

15. Pharmazeutische Zusammensetzung nach Anspruch 14, die eine Verbindung nach Anspruch 11 enthält.

16. Verwendung einer Verbindung nach einem der Ansprüche 1 bis 11 für die Herstellung eines Arzneimittels für die Behandlung der portalen Hypertension.

17. Verwendung einer Verbindung nach einem der Ansprüche 1 bis 11 für die Herstellung eines Arzneimittels für die Behandlung der Zirrhose.
